(19) Europäisches Patentamt
European Patent Office
Office européen des brevets

(11) EP 1 345 618 B1

(12) EUROPÄISCHE PATENTSCHRIFT

(45) Veröffentlichungstag und Bekanntmachung des Hinweises auf die Patenterteilung:
**23.03.2005 Patentblatt 2005/12**

(21) Anmeldenummer: **01272669.1**

(22) Anmeldetag: **27.12.2001**

(51) Int Cl.[7]: **A61K 38/17**, A61K 31/428, A61P 17/06

(86) Internationale Anmeldenummer:
**PCT/EP2001/015317**

(87) Internationale Veröffentlichungsnummer:
**WO 2002/053171 (11.07.2002 Gazette 2002/28)**

# (54) CHLORZOXAZON ZUR BEHANDLUNG VON PSORIASIS

CHLORZOXAZONE FOR THE TREATMENT OF PSORIASIS

CHLORZOXAZONE POUR LE TRAITEMENT DU PSORIASIS

(84) Benannte Vertragsstaaten:
**AT BE CH CY DE DK ES FI FR GB GR IE IT LI LU MC NL PT SE TR**

(30) Priorität: **28.12.2000 DE 10065475**
**20.03.2001 US 277453 P**

(43) Veröffentlichungstag der Anmeldung:
**24.09.2003 Patentblatt 2003/39**

(73) Patentinhaber:
- **Switch Biotech Aktiengesellschaft 82061 Neuried (DE)**
- **Ludwig Maximilians Universität 80539 München (DE)**
- **Alzheimer, Christian 24105 Kiel (DE)**

(72) Erfinder:
- **GOPPELT, Andreas 80636 München (DE)**
- **ALZHEIMER, Christian 24105 Kiel (DE)**
- **KÖGEL, Heidi 80802 München (DE)**

(74) Vertreter:
**Bösl, Raphael, Dr. rer. nat., Dipl.-Chem. et al**
**Patentanwälte**
**Isenbruck Bösl Hörschler Wichmann Huhn**
**Postfach 860 880**
**81635 München (DE)**

(56) Entgegenhaltungen:

| | |
|---|---|
| **WO-A-00/33834** | **WO-A-00/34228** |
| **WO-A-00/34248** | **WO-A-00/37422** |
| **WO-A-97/18332** | **WO-A-98/11139** |
| **WO-A-99/25347** | **GB-A- 2 273 873** |
| **US-A- 4 569 935** | **US-A- 4 680 296** |

- **KOEGEL HEIDI ET AL: "Expression and biological significance of Ca2+-activated ion channels in human keratinocytes." FASEB JOURNAL, Bd. 15, Nr. 1, Januar 2001 (2001-01), Seiten 145-154, XP002209209 ISSN: 0892-6638**
- **PATENT ABSTRACTS OF JAPAN vol. 1999, no. 13, 30. November 1999 (1999-11-30) & JP 11 209302 A (POLA CHEM IND INC), 3. August 1999 (1999-08-03)**
- **CUTHBERT A W ET AL: "Activation of Ca2+- and cAMP-sensitive K+ channels in murine colonic epithelia by 1-ethyl-2-benzimidazolone." AMERICAN JOURNAL OF PHYSIOLOGY, Bd. 277, Nr. 1 PART 1, Juli 1999 (1999-07), Seiten C111-C120, XP002216462 ISSN: 0002-9513**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 345 618 B1

Printed by Jouve, 75001 PARIS (FR)

## Beschreibung

Die Erfindung betrifft die Verwendung von Chlorzoxazon zur Herstellung eines Diagnostikums oder Arzneimittels zur Diagnose, Prävention und/oder Behandlung von Psoriasis.

Viele Erkrankungen der Haut stehen im Zusammenhang mit einer gestörten Keratinozytenaktivität. Exemplarisch kann die Bedeutung der Keratinozyten für die Aufrechterhaltung der normalen physiologischen Vorgänge in der Haut bei der Wundheilung nachvollzogen werden: der Wundheilungsprozeß ist ein extrem komplexer Prozeß, der durch die Phasen der Blutgerinnung im Bereich der Wunde, die Rekrutierung von Entzündungszellen, Reepithalisierung und Gewebereorganisation gekennzeichnet ist. Bei der Blutgerinnung kommt es zur Bildung einer Fibrinmatrix, die dazu dient den durch die Wunde entstanden Hohlraum auszufüllen und um eine Matrix bereitzustellen, über die Zellen einwandern können. Anschließend werden durch in die Matrix eingelagerte Chemoattraktanten Neutrophile und Monozyten rekrutiert, die eine Entzündungsreaktion initiieren. Danach kommt es zum zentralen Schritt der Reepithalisierung um erneut eine schützende epitheliale Barriere über der Wunde zu bilden. Dies wird durch Keratinozyten bewerkstelligt, die den Großteil der epidermalen Zellen ausmachen und nur in der Haut vorkommen. Um Reepithalisierung zu erreichen, verändern die Keratinozyten Integrin-Zusammensetzung an der Zelloberfläche wodurch sie sich vom ihrem Ausgangsort lösen und über die Wunde migrieren können. Dort heften sie sich wieder an, proliferieren und differenzieren schließlich. Keratinozyten sezemieren Matrix-Metalloproteinasen und Collagenasen, die Proteine im umliegenden Gewebe abbauen und somit die Migration im Wundgewebe ermöglichen. Zudem sezernieren Keratinozyten Wachstumsfaktoren wie z.B. VEGF, KAF, TGF-beta und TGF-alpha. Diese Wachstumsfaktoren wiederum stimulieren die Zellproliferation und die Bildung von Komponenten der extrazellulären Matrix und vermitteln das Wachstum neuer Blutgefäße in das Wundbett. Bei der anschließenden Gewebereorganisation, die bis zu 2 Jahren nach Verwundung andauern kann, kommt es zu einem kontinuierlichen Prozeß der Kollagen-Synthese, des erneuten Abbaus von Kollagen, der schuppenartigen Differenzierung der Keratinozyten und der Bildung einer Narbe.

Der Überblick über den Wundheilungsprozesses verdeutlicht, daß für eine normal verlaufende Wundheilung komplexe räumliche und zeitliche Veränderungen der Keratinozytenaktivität, wie der Migration, Proliferation und Differenzierung erforderlich sind. Störungen der Keratinozytenaktivitäten können daher zu Wundheilungsstörungen und zu anderen Hauterkrankungen führen. Erkrankungen, die mit disfunktionellen Keratinozyten in Zusammenhang gebracht werden sind beispielsweise Psoriasis, Kontaktdermatitis, atopisches Ekzem, Ulzera, Vitiligo, Hyperkeratosen, aktinische Keratosen und Akne.

Auch nach der Schließung einer Wunde kann es zu Störungen der Wundheilung bzw. der Narbe kommen, wie der Bildung hypertropher Narben oder Keloide. Auch bei diesen Krankheiten wurde ein Zusammenhang mit der Keratinozytenaktivität nahegelegt. So wurde in Keratinozyten aus hypertrophen Narben, die nach Verbrennungswunden entstanden waren, ein höheres Maß an Proliferation, Differenzierung und Aktivierung nachgewiesen als in Keratinozyten aus intakter Haut.

Hauterkrankungen, gestörte Wundheilung sowie kosmetische Beeinträchtigungen sind Probleme die bei der Alterung auftreten. So ist die sogenannte Altershaut durch eine Verdünnung der Epidermis und somit der Schutzbarriere gekennzeichnet. Außerdem sinkt die Anzahl an enzymatisch aktiven Melanozyten um 10-20% alle 10 Jahre in Menschen ab 25-30 Jahre, wodurch die Strahlungsexposition steigt. Die Hautpigmentierung alter Haut ist dabei oft heterogen; lokaler Verlust der Pigmentierung sowie Hyperpigmentierung werden beobachtet. Die wurde u.a. auf die Modifizierung der Melanozyten-Keratinozyten-Interaktionen zurückgeführt. Häufig auftretende Pigmentläsionen sind z.B. Lentigo und Epheliden.

Über die molekularen Grundlagen, die den Hauterkrankungen und der Wundheilung und somit auch den Wundheilungsstörungen zugrunde liegen ist nur sehr wenig bekannt. Dies ist durch die enorme Komplexität der Haut und der damit verbundenen Erkrankungen begründet.

So sind bisher nur wenig zufriedenstellende Therapien entwickelt worden, um bei Wundheilungsstörungen eingreifen zu können. Etablierte Therapieformen beschränken sich auf physikalische Unterstützung der Wundheilung (z.B. Verbände, Kompressen, Gele) oder der Transplantation von Hautgeweben, gezüchteten Hautzellen und/oder Matrixproteinen. In den letzten Jahren sind Wachstumsfaktoren zur Verbesserung der Wundheilung erprobt worden, ohne jedoch die konventionelle Therapie entscheidend zu verbessern. Auch die Diagnose von Wundheilungsstörungen beruht auf wenig aussagekräftigen optischen Analysen der Haut, da ein tieferes Verständnis der Genregulation während der Wundheilung bisher fehlt.

Kaliumkanäle sind eine heterogene Gruppe von Proteinen, die eine wichtige Rolle bei der Regulation des Membranpotentials und somit auch des sekundär energetisierten Transmembrantransports von Molekülen spielen. Des weiteren spielen sie häufig eine Rolle bei der Zellproliferation (Nilius und Droogmans; 1994, News in Physiol. Sci, 9: 105-110). Kaliumkanäle wurden in einigen Fällen in Zusammenhang mit genetisch vererbbaren Erkrankungen gebracht (Curran, 1998, Curr. Opin. in Biotech., 9: 565-572). Diese Tatsache, sowie die Vorteile der Kaliumkanäle, daß sich mit ihnen robuste Assaysysteme zur in vitro Charakterisierung und zum "Highthroughput-Screening" entwickeln lassen, begründen die Attraktivität der Kaliumkanäle als Ziel der Medikamentenentwicklung (Curran, supra).

Die Familie der Kaliumkanäle ist eine sehr heterogene Gruppe, die sich in fünf Unterfamilien einteilen läßt: die spannungsaktivierten Kaliumkanäle (Kv), die "long QT related" Kalimkanäle, die "inward rectifying" Kaliumkanäle und die Calcium-aktivierten Kaliumkanäle. Die spannungsabhängigen Kaliumkanäle werden in einer Vielzahl von Veröffentlichungen als geeignete Ziele zur Behandlung von Krankheiten der Haut genannt (WO 99/07411; WO 97/18332; WO 99/25703), jedoch sind bislang keine Medikamente zur Behandlung von Krankheiten, die im Zusammenhang mit gestörter Keratinozytenaktivität stehen, entwickelt worden.

Die Calcium-aktivierten-Kaliumkanäle werden in drei gut charakterisierte Untergruppen eingeteilt: die "small-conductance" (SK); "intermediate conductance" (IK) und "big-conductance" (BK) Kaliumkanäle. Diese Untergruppen unterscheiden sich in ihrer Spannungs- und Calcium-Sensititvität, ihrem Expressionsprofil, der biologischen Funktion und ihren pharmakologischen Eigenschaften.

Homologe des IK-Kanals wurden sowohl im Menschen (hIK1 oder hsk4; Ishii et al. 1997 Proc. Natl. Acad. Sci. USA 94: 11651-11656; Joiner et al., 1997, Proc. Natl. Acad. Sci. USA, 94 : 11013-11018; WO 99/03882; WO 98/11139) als auch in der Maus (mIK1; Vandorpe et al.. 1998, J. Biol. Chem. 273: 21542-21553) identifiziert. Aufgrund der gleichen Eigenschaften wird angenommen, daß der in Erythrozyten rein elektrophysiologisch und pharmakologisch charakterisierte Gardos-Kanal mit hIK1 identisch ist (Ishii et al. 1997 Proc. Natl. Acad. Sci. USA 94: 11651-11656). Der Gardos-Kanal spielt eine wichtige Funktion bei der Stärke der Ausprägung des Sichelzellenanämie-Phänotyps, indem er die Dehydratisierung und das Volumen erkrankter Erythrozyten beeinflusst (Curran, supra). In der Maus wurde die den IK-Kanal kodierende cDNA aus Erythroleukämie-Zellen isoliert (Vandorpe et al., supra). In dieser Studie konnte gezeigt werden, daß die Expression von mIK1 während der Differenzierung von ES-Zellen zu erythroiden Zellen erhöht wird. Zudem konnte die Proliferation und Differenzierung durch Inhibitoren des Ionenkanals inhibiert werden.

Auffällig ist, daß der IK-Kanal im Menschen im Gegensatz zu SK-Kanälen nur in wenigen Zelltypen und Organen exprimiert wird (WO 99/03882; Ishii et al. 1997 Proc. Natl. Acad. Sci. USA 94: 11651-11656; Joiner et al., 1997, Proc. Natl. Acad. Sci. USA, 94: 11013-11018): in allen Veröffentlichungen wird gezeigt, daß die Expression auf nicht elektrisch erregbare Organe, wie Plazenta und Prostata beschränkt ist, während keine Expression im Gehirn oder im Herzen nachgewiesen wurde. Dies spiegelt sich in der Lehrmeinung wieder, daß die Expression von hIK im Menschen auf Zellen des Blutes (z.B. B- und T-Lymphozyten sowie Erythrozyten) und des Blutgefäßsystems (z.B. Endothelzellen der Niere, des Mesenteriums Kapillarendothelzellen im Hirn (Kohler et al., 2000, Circ. Res. 87: 496-503) beschränkt ist (WO 00/34248). Weiterhin wurde die Expression des IK1-Kanals in Zellen der Harnblase sowie des Dickdarms gezeigt (Ohya et al., 2000, Jpn. J. Pharmacol. 84:97-100; Warth et al., 1999, Pflügers Arch. 438: 437-444). In Haut oder Keratinozyten wurde bislang keine Expression von hIK1 oder mIK1 nachgewiesen.

Neben dem Einsatz zur Modulation der Sichelzellenanämie wurde die Verwendung von hIK1 zur Diagnose, Prävention oder Behandlung von Krankheiten, die mit disfunktionellen Leukozyten im Zusammenhang stehen, vorgeschlagen (WO 99/25347). Außerdem finden Inhibitoren des IK-Kanals bei der Entwicklung von Medikamenten zur Behandlung von Diarrhöe und zystischer Fibrose Anwendung. In den letzten Jahren wurden eine Vielzahl von spezifischen Modulatoren der IK-Aktivität entwickelt, die pharmazeutisch verwendet werden können (Syme et al., 2000, Am. J. Physiol. 278: C570-581; WO 99/25347; WO 00/33834; WO 00/34228; WO 00134248; WO 00/37422).

Aufgabe der Erfindung war es Polypeptide und/oder diese kodierende Nukleinsäuren zur Verfügung zu stellen.

Es war weiterhin Aufgabe der Erfindung, Modulatoren der Aktivität der von Polypeptiden bereitzustellen die bei Erkrankungen, die mit gestörter Keratinozytenaktivität im Zusammenhang stehen, beteiligt sind, und deren Verwendung die Diagnose, Prävention oder Behandlung und die Identifizierung und Entwicklung von Pharmazeutika, die bei Erkrankungen, die mit gestörter Keratinozytenaktivität im Zusammenhang stehen, wirksam sind, entscheidend verbessert. Die Verwendung die Diagnose der Modulatoren sollte, Prävention oder Behandlung und die Identifizierung und Entwicklung von Pharmazeutika, die bei Erkrankungen, die mit gestörter Keratinozytenaktivität im Zusammenhang stehen, wirksam sind, entscheidend verbessern.

Bei der Stimulation von Keratinozyten mit ATP, Bradykinin und Histamin, die bei Erkrankungen, die mit gestörter Keratinozytenaktivität im Zusammenhang stehen, in der Haut freigesetzt werden, konnte eine langanhaltende Hyperpolarisierung der Keratinozyten gemessen werden. Überraschenderweise konnte ein "intermediate-conductance" Calcium-aktivierter-Kaliumkanal als Mediator dieser elektrophysiologischen Antwort identifiziert werden, der bisher nicht in der Haut oder in Keratinozyten nachgewiesen und der erstmals mit gestörter Keratinozytenaktivität in Zusammenhang gebracht wurde. Weiterhin konnte zum erstenmal ein Zusammenhang zwischen Expressionslevel und/oder Aktivität eines "intermediate-conductance" Calcium-aktivierten-Kaliumkanals und Kaliumkanal und der Diagnose, Prävention und/oder Behandlung von Erkrankungen, die mit gestörter Keratinozytenaktivität in Zusammenhang stehen, insbesondere von Wundheilungsstörungen und/oder Psoriasis, hergestellt werden. Die Polypeptide, und dessen kodierende Nukleinsäuren gehören nicht zu den bisher bekannten Zielen für die Diagnose, beispielsweise der Indikation- und/oder der Behandlung - wie beispielsweise der Modulation - und/oder Prävention von Erkrankungen, die mit gestörter Keratinozytenaktivität in Zusammenhang stehen oder für die Identifizierung von pharmakologisch aktiven Substanzen, so daß sich aus dieser Erfindung völlig neue Therapieansätze ergeben. Weiterhin wurden Modulatoren der erfindungsgemäßen Polypeptide - wie zum Beispiel Inhibitoren oder Aktivatoren - nicht bei der Diagnose,

Prävention und/oder Behandlung von Erkrankungen, die mit gestörter Keratinozytenaktivität in Zusammenhang, verwendet, so daß sich völlig neue Therapieansätze ergeben.

Beschrieben wird vorliegend daher die Verwendung des intermediate-conductance Calcium-aktivierten Kalium Kanal IK1 Polypeptids aus Mensch (hIK1 oder hSK4; SEQ ID Nr. 3; Ishii et al. 1997 Proc. Natl. Acad. Sci. USA 94: 11651-11656; Joiner et al., 1997, Proc. Natl. Acad. Sci. USA, 94 : 11013-11018; WO 99/03882; WO 98/11139) oder Maus (mIK1; SEQ ID Nr. 4; Vandorpe et al. 1998, J. Biol. Chem. 273: 21542-21553) oder funktionelle Varianten davon und/oder diese kodierende Nukleinsäuren oder Varianten davon, zur Diagnose, Prävention und/oder Behandlung von Erkrankungen, die mit gestörter Keratinozytenaktivität in Zusammenhang stehen oder zur Identifizierung pharmakologisch aktiver Substanzen.

Die Aufgabe wird durch die Verwendung von Chlorzoxazon zur Herstellung eines Diagnostikums zur Diagnose oder eines Arzneimittels zur Prävention und/oder Behandlung von Psoriasis gelöst.

Für keinen der Ionenkanäle oder deren kodierende Nukleinsäuren wurde bisher ein Zusammenhang mit Erkrankungen, die mit gestörter Keratinozytenaktivität in Zusammenhang stehen, beispielsweise bei einer gestörten Wundheilung, beschrieben oder nahegelegt. Es war daher unerwartet, daß diese Verbindungen wie beschrieben verwendet werden können. Die "Accession" Nummern oder Zugangsnummern der erfindungsgemäß verwendeten Polypeptide und ihrer cDNAs sind in Tabelle 1 aufgeführt.

Weiterhin wurde für Chlorzoxazon bisher kein Zusammenhang mit Hauterkrankungen, beispielsweise bei einer gestörten Wundheilung und/oder Psoriasis, beschrieben oder nahegelegt. Es war daher unerwartet, dass diese Verbindung erfindungsgemäß verwendet werden kann.

Darüber hinaus ist nicht bekannt, dass Chlorzoxazon zur Diagnose, Prävention und/oder Behandlung von Erkrankungen, die mit gestörter Keratinozytenaktivität in Zusammenhang stehen, insbesondere von Wundheilungsstörungen und/oder Psoriasis, verwendet werden kann, so dass völlig neue therapeutische Ansätze von dieser Erfindung ausgehen.

Die beschriebenen Polypeptide können weiterhin dadurch gekennzeichnet sein, daß diese synthetisch hergestellt werden. So kann das gesamte Polypeptid oder Teile davon zum Beispiel mit Hilfe der klassischen Synthese (Merrifield-Technik) synthetisiert werden. Teile der vorangehend beschriebenen Polypeptide eignen sich insbesondere zur Gewinnung von Antiseren, mit deren Hilfe geeignete Genexpressionsbanken durchsucht werden können, um so zu weiteren funktionellen Varianten des Polypeptids zu gelangen.

Unter "Keratinozytenaktivität" versteht man, den Zustand einer Keratinozyte, der durch die Parameter Proliferationsrate und dem Differenzierungsgrad der zu untersuchenden Keratinozyte bestimmt wird. Methoden zur Bestimmung der Parameter sind dem Fachmann bekannt (de Fries und Mitsuhashi, 1995, J. Clin. Lab. Anal. 9:89-95; Perros und Weightman, 1991, Cell Prolif. 24:517-23; Savino und Dardenne, 1985, J. Immunol. Methods 85:221-6; Schulz et al., 1994, J. Immunol. Methods 167:1-13; Frahm et al., 1998, J. Immunol. Methods 211:43-50; Rosenthal et al., 1992, J, Invest, Dermatol, 98:343-50). Der Differenzierungsgrad kann beispielsweise durch geeignete Färbungen, die dem Fachmann bekannt sind, bestimmt sind.

Unter "gestörter" Keratinozytenaktivität versteht man eine Keratinozytenaktivität einer Keratinozyte in der Haut, die von der Keratinozytenaktivität von Keratinozyten in normaler, intakter Haut abweicht. Beispielsweise kann sich die Aktivität der "gestörten" Keratinozyten durch eine gesteigerte oder verlangsamte Proliferationsrate oder durch ein verfrühte, beschleunigte, verlangsamte, verspätete oder nicht vorhandene Differenzierung von normalen Keratinozyten unterscheiden. Erkrankungen, die mit gestörter Keratinozytenaktivität in Zusammenhang stehen sind beispielsweise Kontaktdermatitis, atopisches Ekzem, Vitiligo, Hyperkeratosen, aktinische Keratosen, hypertrophe Narben, Keloide, Lentigo, Epheliden, Altershaut. Des weiteren sind insbesondere Wunden, beispielsweise normal heilende Wunden und schlecht heilende Wunden, insbesondere Ulzera bevorzugte Erkrankungen im Sinne der vorliegenden Erfindung die durch gestörte Keratinozytenaktivität gekennzeichnet. Eine besonders bevorzugte Erkrankung ist Psoriasis.

Unter dem Begriff "Regulation" wird beispielsweise die Erhöhung oder Erniedrigung der Menge und/oder Aktivität der Polypeptide oder diese kodierende Nukleinsäure verstanden, wobei diese Änderung beispielsweise auf transkriptioneller, translationeller sowie posttranskriptioneller oder posttranslationeller Ebene stattfinden kann.

Unter dem Begriff "funktionelle Varianten" sind Polypeptide zu verstehen, die beispielsweise wie die wie beschrieben verwendbaren Polypeptiden im Zusammenhang mit gestörter Keratinozytenaktivität stehen und/oder Strukturmerkmale der erfindungsgemäß verwendbaren Polypeptide aufweisen.

Zu funktionellen Varianten zählen auch Fusionsproteine der IK-Kanäle, mit einem Anteil von ca. 1- 300, vorzugsweise ca. 1-200, besonders bevorzugt ca. 1-150, insbesondere ca. 1-100, vor allem ca. 1-50 fremden Aminosäuren, die N-tenninal, C-terminal und als Insertion mit der Polypeptidkette der IK-Kanäle verknüpft sein können. Weiterhin kann ein erfindungsgemäß verwendbarer IK-Kanal mit einem "Green fluorescent Protein" oder Varianten davon verknüpft sein.

Funktionelle Varianten des Polypeptids können auch Teile des Polypeptids mit mindestens 6 Aminosäuren Länge, vorzugsweise mit mindestens 8 Aminosäure Länge, insbesondere mit mindestens 12 Aminosäure Länge, ganz

besonders mit mindestens 20 Aminosäure Länge, vor allem mit mindestens 40 Aminosäure Länge, am besten mit mindestens 80 Aminosäure Länge sein. Eingeschlossen sind auch Deletionen des Polypeptids im Bereich von ca. 1-60, vorzugsweise von ca. 1-30, insbesondere von ca. 1-15, vor allem von ca. 1-5 Aminosäuren. Beispielsweise kann die erste Aminosäure Methionin fehlen, ohne daß die Funktion des Polypeptids wesentlich verändert wird.

"Funktionelle Varianten" im Sinne der vorliegenden Erfindung sind Polypeptide, die eine Sequenzhomologie, insbesondere eine Sequenzidentität, von ca. 50%, bevorzugsweise ca. 60%, insbesondere ca. 70%, besonders bevorzugt ca. 90% , am meisten bevorzugt 95% zu einem der IK-Kanäle mit der Aminosäuresequenz gemäß einer der SEQ ID Nr. 3 oder SEQ ID Nr. 4 aufweisen. Beispiele solcher funktioneller Varianten sind demnach auch die entsprechenden Polypeptide, die aus anderen Organismen als dem Menschen bzw. der Maus, vorzugsweise aus nicht-menschlichen Säugetieren wie z. B. Affen, Schweinen und Ratten stammen. Andere Beispiele sind Polypeptide, die durch unterschiedliche Allele des Gens, in verschiedenen Individuen oder in verschiedenen Organen eines Organismus kodiert werden.

Unter "Sequenzidentität" versteht man die Übereinstimmung (=% Positive) zwischen zwei Sequenzen, die im Falle von Polypeptidsequenzen beispielsweise mittels BlastP 2.0.1 und im Falle von Polynukleotidesequenzen beispielsweise mit BlastN 2.0.14 bestimmt wird, wobei der Filter "off" gesetzt ist (Altschul et al., 1997, Nucleic Acid Res 25: 3389-3402). Zur Bestimmung der Ähnlichkeit zwischen zwei Polypeptiden wird beispielsweise BlastP 2.0.1 verwendet, wobei der Filter "off" gesetzt ist und BLOSUM 62 ist.

Zu funktionellen Varianten zählen beispielsweise auch Polypeptide, die von Nukleinsäuren kodiert werden, die aus nicht-hautspezifischem Gewebe, z. B. Embryonalgewebe, isoliert werden, jedoch nach Expression in einer Keratinozyte die bezeichneten Funktionen besitzen.

Um festzustellen, ob es sich bei einem Polypeptid um eine funktionelle Variante eines verwendbaren Polypeptids handelt, kann mittels funktionaler Tests die Aktivität des zu prüfenden Polypeptids mit der Aktivität eines verwendbaren Polypeptids gemäß SEQ ID Nr. 3 oder gemäß SEQ ID Nr. 4 verglichen werden. Unter der Vorraussetzung, dass das zu prüfende Polypeptid die Anforderungen eine eine funktionelle Variante auf der Ebene der Sequenzidentität erfüllt, handelt es sich bei dem zu prüfenden Polypeptid dann um eine funktionelle Variante, wenn dessen Aktivität im funktionalen Test mit der des verwendbaren Polypeptids ähnlich oder identisch ausfällt.

Diese funktionalen Tests umfassen beispielsweise die Applikation eines Expressionsvektors, der eine das zu prüfendende Polypeptid kodierende Nukleinsäure enthält, oder die Applikation des zu prüfenden Polypeptids selbst oder eines gegen das zu prüfende Polypeptid gerichteten Antikörpers oder eines Antisense-Oligonukleotids in Wunden. Nach Inkubation, beispielsweise eines Expressionsvektors, wird der Fortgang der Wundheilung bei Gabe der unterschiedlichen Expressionsvektoren, enthaltend entweder eine das zu prüfendende Polypeptid kodierende Nukleinsäure oder eine ein erfindungsgemäß verwendbares Polypeptid kodierende Nukleinsäure oder einen Expressionsvektor ohne Insert verglichen. Diese funktionalen Tests können beispielsweise auch auf die Aktivität des zu prüfenden Polypeptids bei Störungen der Wundheilung, zum Beispiel bei schlecht heilenden, Dexamethason behandelten oder diabetischen Wunden von Tieren; angewandt werden. So führte beispielsweise die Applikation von PDGF-A und PDGF-B Polypeptiden auf schlecht heilende Wunden von Kaninchen zu vergleichbarer Verbesserung der Wundheilung (J. Surg. Res., 2000, 93:230-236). Vergleichbare Tests können für Hauterkrankungen, beispielsweise Psoriasis durchgeführt werden. Dabei wird ein Expressionsvektor, der eine das zu prüfende Polypeptid kodierende Nukleinsäure enthält, oder das zu prüfende Polypeptid selbst oder ein gegen das zu prüfende Polypeptid gerichteter Antikörper oder ein Antisense-Oligonukleotid beispielsweise auf eine betroffene humane Hautpartie, die auf SCID-Mäuse transplantiert wurde, aufgetragen und der Verlauf der Hauterkrankung, beispielsweise die Heilung, ermittelt und mit dem Verlauf der Hauterkrankung im Kontrollversuch verglichen. Dies kann im Falle der Psoriasis, beispielsweise auch Antiproliferationsstudien in sogenannte "Mouse Tail Tests" umfassen. Dabei wird die Tatsache ausgenutzt, dass die normale histologische Struktur der Haut eines Mausschwanzes der von humaner von Psoriasis befallener Haut stark ähnelt: So besitzt Schwanzhaut sogenannte "hinge"-Regionen, wo Haare unter den Schuppen hervorkommen; dort ist die Haut orthokeratotisch und besitzt eine granuläre Schicht wie normale Haut. Nach Zugabe eine zu prüfenden Polypeptides werden histologische Schnitte der Schwanzhaut auf die Bildung einer Granulationsschicht, die Induktion orthokeratotischen Gewebes in parakeratotischen Regionen und die Dicke der Epidermis gemessen (Jarret et al., 1970, Br J Dermatol 82: 187-199). Durch die Vermessung sagitaler Schnitte nach Hämatoxylin/Eosin-Färbung wird diese Methode auch quantifizierbar (Bosman et al., 1992, Skin Pharmacol 5: 41-48; Bosman et al., 1994, Skin Pharmakol 7: 324-334). Des weiteren kann die antiinflammatorische Wirkung des zu prüfenden Polypeptides durch topische Applikation auf Mäuse bei denen mit Oxazolon Ödeme oder auf Schweine bei denen mit DNFB allergische Hautreaktionen induziert wurden, eingesetzt werden (Meingassner et al., 1997, Br J Dermatol 137: 568-576). Funktionale Tests beinhalten auch das Messen des Einflusses des zu prüfenden Polypeptides auf das Ausmaß der Hautschuppung. So belegen Messungen des transepidennalen Wasserverlustes (TEWL) bei psoriatrischen Patienten, dass die pathologisch veränderten Epithelschichten ihre Barrierefunktion für Wasser verloren haben (Frodin et al., 1988, Acta Derm Venerol 68: 461-467, siehe Beispiel).

Der Begriff "kodierende Nukleinsäure" bezieht sich auf eine DNA-Sequenz, die für ein isolierbares bioaktives

verwendbares Polypeptid oder einen Vorläufer kodiert. Das Polypeptid kann durch eine Sequenz in voller Länge oder jeden Teil der kodierenden Sequenz kodiert werden, solange die spezifische, beispielsweise Ionenkanal-Aktivität erhalten bleibt.

Es ist bekannt, daß kleine Veränderungen in der Sequenz der verwendbaren Nukleinsäuren vorhanden sein können, zum Beispiel durch die Degenerierung des genetischen Codes, oder daß nicht translatierte Sequenzen am 5' und/oder 3'-Ende der Nukleinsäure angehängt sein können, ohne daß dessen Aktivität wesentlich verändert wird. Diese Erfindung schließt deshalb auch sogenannte "Varianten" der vorangehend beschriebenen Nukleinsäuren ein.

Unter "stringenten Hybridisierungsbedingungen" sind solche Bedingungen zu verstehen, bei denen eine Hybridisierung bei 60°C in 2,5 x SSC-Puffer, gefolgt von mehreren Waschschritten bei 37°C in einer geringeren Pufferkonzentration, wie beispielsweise 0,5 x SSC-Puffer erfolgt und stabil ist.

Mit dem Begriff "Varianten" sind alle DNA-Sequenzen bezeichnet, die komplementär zu einer DNA-Sequenz sind, die unter stringenten Bedingungen mit der Referenzsequenz hybridisieren und für ein Polypeptid kodieren, das eine ähnliche Aktivität aufweist, wie das von der Referenzsequenz kodierte Polypeptid.

Varianten der Nukleinsäuren können auch Teile der verwendeten Nukleinsäuren mit mindestens 8 Nukleotiden Länge, vorzugsweise mit mindestens 18 Nukleotiden Länge, insbesondere mit mindestens 24 Nukleotiden Länge sein.

Bevorzugterweise handelt es sich bei den verwendeten Nukleinsäuren um DNA oder RNA, vorzugsweise eine DNA, insbesondere eine doppelsträngige DNA. Weiterhin kann die Sequenz der Nukleinsäuren dadurch gekennzeichnet sein, daß sie mindestens ein Intron und/oder eine polyA-Sequenz aufweist. Die verwendeten Nukleinsäuren können auch in Form ihrer antisense-Sequenz verwendet werden.

Weiterhin kann eine Nukleinsäure verwendet werden, die synthetisch hergestellt worden ist. So kann die verwendbare Nukleinsäure beispielsweise chemisch anhand der cDNA Sequenzen gemäß SEQ ID Nr. 1 oder SEQ ID Nr. 2 und/oder anhand der Proteinsequenzen gemäß SEQ ID Nr. 3 oder SEQ ID Nr. 4 (siehe auch Tabelle 1) unter Heranziehen des genetischen Codes z. B. nach der Phosphotriester-Methode synthetisiert werden (siehe z. B. Uhlmann, E. & Peyman, A. (1990) Chemical Reviews, 90, 543-584, No. 4).

Eine weitere Verwendung der verwendeten Nukleinsäuresequenzen ist die Konstruktion von anti-sense Oligonukleotiden (Zheng und Kemeny, 1995, Clin. Exp. Immunol. 100:380-2; Nellen und Lichtenstein, 1993, Trends Biochem. Sci. 18:419-23; Stein, 1992, Leukemia 6:967-74) und/oder Ribozymen (Amarzguioui, et al. 1998, Cell. Mol. Life Sci. 54:1175-202; Vaish, et al., 1998, Nucleic Acids Res. 26:5237-42; Persidis, 1997, Nat. Biotechnol. 15:921-2; Couture und Stinchcomb, 1996, Trends Genet. 12:510-5) und/oder von sogenannten kleinen interferierenden RNA Molekülen (siRNAs) (Elbashir et al., Nature 2001;411:494-8). Mit anti-sense Oligonukleotiden kann man die Stabilität der vorangehend beschriebenen Nukleinsäure verringern und/oder die Translation der vorangehend beschriebenen Nukleinsäure inhibieren. Ein ähnlicher Ansatz ist die Verwendung von siRNA-Oligonukleotiden, die ebenfalls zu einer Verringerung der Polypeptid-Menge führen. So kann beispielsweise die Expression der entsprechenden Gene in Hautzellen sowohl in vivo als auch in vitro durch Verwendung von Antisense-Molekülen und/oder siRNA-Molekülen und/oder Ribozymen verringert werden. Diese Oligonukleotide können sich daher als Therapeutikum eignen. Diese Strategie eignet sich für Hautzellen, vorzugsweise für Keratinozyten, insbesondere, wenn die "antisense"-Oligonukleotide mit Liposomen komplexiert werden (Smyth et al., 1997, J. Invest. Dermatol. 108:523-6; White et al., 1999, J. Invest. Dermatol. 112:699-705; White et al., 1999, J. Invest. Dermatol. 112:887-92). Für die Verwendung als Sonde oder als "antisense"-Oligonukleotid ist eine einzelsträngige DNA oder RNA bevorzugt.

Die Sonde kann zur Diagnose von Erkrankungen, die im Zusammenhang mit gestörter Keratinozytenaktivität stehen, gegebenenfalls kombiniert oder zusammen mit geeigneten Zusatz-und/oder Hilfsstoffen, verwendet werden.

Oligonukleotide werden in der Regel schnell durch Endo- oder Exonukleasen, insbesondere durch in der Zelle vorkommende DNasen und RNasen, abgebaut.

Deshalb ist es vorteilhaft, die Nukleinsäure zu modifizieren, um sie gegen den Abbau zu stabilisieren, so daß über einen langen Zeitraum eine hohe Konzentration der Nukleinsäure in der Zelle beibehalten wird (Beigelman et al., 1995, Nucleic Acids Res. 23:3989-94; Dudycz, 1995, WO 95/11910; Macadam et al., 1998, WO 98/37240; Reese et al., 1997, WO 97/29116). Typischerweise kann eine solche Stabilisierung durch die Einführung von einer oder mehrerer Intemukleotid-Phosphargruppen oder durch die Einfiihrung einer oder mehrerer Nicht-Phosphor-Intemukleotide, erhalten werden.

Geeignete modifizierte Intemukleotide sind in Uhlmann und Peymann (1990 Chem. Rev. 90, 544) zusammengefaßt (siehe auch Beigelman et al., 1995 Nucleic Acids Res. 23: 3989-94; Dudycz, 1995, WO 95/11910; Macadam et al., 1998, WO 98/37240; Reese et al., 1997, WO 97/29116). Modifizierte Intemukleotid-Phosphatreste und/oder Nicht-Phosphorbrücken in einer Nukleinsäure, die bei einer der Verwendungen eingesetzt werden können, enthalten zum Beispiel Methylphosphonat, Phosphorthioat, Phosphoramidat, Phosphordithioat, Phosphatester, während Nicht-Phosphor-Intemukleotid-Analoge, beispielsweise Siloxanbrücken, Carbonatbrücken, Carboxymethylester, Acetamidatbrücken und/oder Thioetherbrücken enthalten. Es ist auch beabsichtigt, daß diese Modifizierung die Haltbarkeit einer pharmazeutischen Zusammensetzung, die bei einer der Verwendungen eingesetzt werden kann, verbessert.

Die verwendbaren Nukleinsäuren können zur Herstellung eines Vektors, vorzugsweise in Form eines "shuttle"-Vektors, Phagemids, Cosmids Expressionsvektors oder gentherapeutisch wirksamen Vektors verwendet werden. Weiterhin können unter Verwendung der vorangehend beschriebenen Nukleinsäuren "knock-out"-Genkonstrukte oder Expressionskassetten hergestellt werden.

So kann die verwendbaren Nukleinsäure in einem Vektor, vorzugsweise in einem Expressionsvektor oder gentherapeutisch wirksamen Vektor, enthalten sein. Vorzugsweise enthält der gentherapeutisch wirksame Vektor haut- bzw. Keratinozyten-spezifische regulatorische Sequenzen, die funktionell mit der vorangehend beschriebenen Nukleinsäure verbunden sind.

Für die Expression des betreffenden Gens ist im allgemeinen eine doppelsträngige DNA bevorzugt, wobei der für das Polypeptid kodierende DNA-Bereich besonders bevorzugt ist. Dieser Bereich beginnt beispielsweise mit dem ersten in einer Kozak Sequenz (Kozak, 1987, Nucleic. Acids Res. 15:8125-48) liegenden Start-Codon (ATG) bis zum nächsten Stop-Codon (TAG, TGA bzw. TAA), das im gleichen Leseraster zum ATG liegt. Bei Prokaryonten beginnt dieser Bereich mit einer ein paar Nukleotide oberhalb des Start-Codon liegenden konservierten Sequenz von 6 Nukleotiden, die sogenannte Shine-Dalgarno Sequenz (Shine&Dalgamo, 1975, Eur J Biochem. 57:221-30).

Die Expressionsvektoren können prokaryotische oder eukaryotische Expressionsvektoren sein. Beispiele für prokaryotische Expressionsvektoren sind für die Expression in E. coli z.B. die Vektoren pGEM oder pUC-Derivate und für eukaryotische Expressionsvektoren für die Expression in Saccharomyces cerevisiae z. B. die Vektoren p426Met25 oder p426GAL1 (Mumberg et al. (1994) Nucl. Acids Res., 22, 5767-5768), für die Expression in Insektenzellen z. B. Baculovirus-Vektoren wie in EP-B1-0 127 839 oder EP-B1-0 549 721 offenbart, und für die Expression in Säugerzellen z. B. die Vektoren Rc/CMV und Rc/RSV oder SV40-Vektoren, welche alle allgemein erhältlich sind.

Im allgemeinen enthalten die Expressionsvektoren auch für die jeweilige Wirtszelle geeignete Promotoren, wie z. B. den trp-Promotor für die Expression in E. coli (siehe z. B. EP-B1-0 154 133), den Met 25, GAL 1 oder ADH2-Promotor für die Expression in Hefen (Russel et al. (1983), J. Biol. Chem. 258, 2674-2682; Mumberg, supra) oder den Baculovirus-Polyhedrin-Promotor, für die Expression in Insektenzellen (siehe z. 13. EP-B1-0 127 839). Für die Expression in Säugetierzellen sind beispielsweise Regulationselemente geeignet, die eine konstitutive, regulierbare, gewebsspezifische, zellzyklusspezifische oder metabolischspezifische Expression in eukaryotischen Zellen erlauben. Regulationselemente gemäß der vorliegenden Erfindung enthalten Promotor-, Aktivator-, Enhancer-, Silencer und/oder Repressor-Sequenzen.

Beispiele für geeignete Regulationselemente, die konstitutive Expression in Eukaryonten ermöglichen, sind Promotoren, die von der RNA Polymerase III erkannt werden oder virale Promotoren, CMV-Enhancer, CMV-Promotor, SV40 Promotor oder LTR-Promotoren z. B. von MMTV (mouse mammary tuinour virus; Lee et al. (1981) Nature 214, 228-232) und weitere virale Promotor- und Aktivatorsequenzen, abgeleitet aus beispielsweise HBV, HCV, HSV, HPV, EBV, HTLV oder HIV.

Beispiele für Regulationselemente, die induzierbare Expression in Eukaryonten ermöglichen, sind der Tetracyclinoperator in Kombination mit einem entsprechenden Repressor (Gossen M. et al. (1994) Curr. Opin. Biotechnol. 5, 516-20).

Die Expression der verwendeten Gene kann unter der Kontrolle von gewebespezifischen Promotoren, erfolgen wobei hautspezifische Promotoren wie beispielsweise der humane K10 Promotor (Bailleul et al., 1990. Cell 62: 697-708), der humane K14 Promotor (Vassar et al., 1989, Proc. Natl. Acad. Sci. USA 86: 1563-67) oder der bovine Cytokeratin IV Promotor (Fuchs et al., 1988; The biology of wool and hair (Hrsg.: G.E. Rogers. et al.), S. 287-309. Chapman und Hall, London/New York) besonders vorteilhaft sind.

Weitere Beispiele für Regulationselemente, die gewebsspezifische Expression in Eukaryonten ermöglichen, sind Promotoren oder Aktivatorsequenzen aus Promotoren oder Enhancem von solchen Genen, die für Proteine kodieren, die nur in bestimmten Zelltypen, vorzugsweise Keratinozyten, exprimiert werden.

Beispiele für Regulationselemente, die zellzyklusspezifische Expression in Eukaryonten ermöglichen, sind Promotoren folgender Gene: cdc25, Cyclin A, Cyclin E, cdc2, E2F, B-myb oder DHFR (Zwicker J. und Müller R. (1997) Trends Genet. 13, 3-6).

Beispiele für Regulationselemente, die metabolischspezifische Expression in Eukaryonten ermöglichen, sind Promotoren, die durch Hypoxie, durch Glukosemangel, durch Phosphatkonzentration oder durch Hitzeschock reguliert werden.

Beispiele für Regulationselemente, die gleichzeitig eine räumlich und zeitlich begrenzte Expression erlauben, sind Nukleinsäuren, die für ein Fusionsprotein kodieren, wobei die Nukleinsäure zwischen der Sequenz für die ortsspezifische Rekombinase Cre und einem modifizierten Östrogenrezeptor, der unter der Kontrolle eines gewebespezifischen Promoters steht, enthalten ist. Das resultierende, gewebespezifische zytoplasmatische Fusionsprotein kann durch Gabe des Östrogenanalogs Tamoxifen in den Zellkern translozieren und Rekominationen erzeugen, die zu veränderter Genexpression führt (Feil et al., 1996, Proc Natl Acad Sci 93: 10887-90).Um die Einführung von verwendbaren Nukleinsäuren und damit die Expression des Polypeptids in einer eu- oder prokaryotischen Zelle durch Transfektion, Transformation oder Infektion zu ermöglichen, kann die Nukleinsäure als Plasmid, als Teil eines viralen oder nichtviralen Vektors vorliegen. Als virale Vektoren eignen sich hierbei besonders: Baculoviren, Vakziniaviren, Adenoviren,

adenoassoziierte Viren und Herpesviren. Als nicht-virale Vektoren eignen sich hierbei besonders: Virosomen, Liposomen, kationische Lipide, oder poly-Lysin konjugierte DNA.

Beispiele von gentherapeutisch wirksamen Vektoren sind Virusvektoren, beispielsweise Adenovirusvektoren oder retroviralen Vektoren (Lindemann et al., 1997, Mol. Med. 3: 466-76; Springer et al., 1998, Mol. Cell. 2: 549-58). Eukaryotische Expressionsvektoren eignen sich in isolierter Form für die gentherapeutische Anwendung, da nackte DNA bei topischer Applikation in Hautzellen eindringen kann (Hengge et al., 1996, J. Clin. Invest. 97: 2911-6; Yu et al., 1999, J. Invest. Dermatol. 112: 370-5).

Gentherapeutisch wirksame Vektoren lassen sich auch dadurch erhalten, daß man die verwendeten Nukleinsäure mit Liposomen komplexiert, da damit eine sehr hohe Transfektionseffizienz, insbesondere von Hautzellen, erreicht werden kann (Alexander und Akhurst, 1995, Hum. Mol. Genet. 4: 2279-85). Bei der Lipofektion werden kleine unilamellare Vesikel aus kationischen Lipiden durch Ultraschallbehandlung der Liposomensuspension herstellt. Die DNA wird ionisch auf der Oberfläche der Liposomen gebunden, und zwar in einem solchen Verhältnis, daß eine positive Nettoladung verbleibt und die Plasmid-DNA vollständig von den Liposomen komplexiert wird. Neben den von Felgner et al. (1987, supra) eingesetzten Lipidmischungen DOTMA (1,2-Dioleyloxpropyl-3-trimethylammoniumbromid) und DPOE (Dioleoylphosphatidylethanolamin) wurden inzwischen zahlreiche neue Lipidformulierungen synthetisiert und auf ihre Effizienz der Transfektion verschiedener Zellinien getestet (Behr, J.P. et al. (1989), Proc. Natl. Acad. Sci. USA 86, 6982-6986; Felgner, J.H. et al. (1994) J. Biol. Chem. 269, 2550-2561; Gao, X. & Huang, L. (1991), Biochim. Biophys. Acta 1189, 195-203). Beispiele der neuen Lipidformulierungen sind DOTAP N-[1-(2,3-Dioleoyloxy)propyl]-N,N,N-trimethylammoniumethylsulfat oder DOGS (TRANSFECTAM; Dioctadecylamidoglycylspermin).

Hilfsstoffe, die den Transfer von Nukleinsäuren in die Zelle erhöhen, können beispielsweise Proteine oder Peptide, die an DNA gebunden sind oder synthetische Peptid-DNA-Moleküle, die den Transport der Nukleinsäure in den Kern der Zelle ermöglichen, sein (Schwartz et al. (1999) Gene Therapy 6, 282; Brandén et al. (1999) Nature Biotech. 17, 784). Hilfsstoffe schließen auch Moleküle ein, die die Freisetzung von Nukleinsäuren in das Zytoplasma der Zelle ermöglichen (Planck et al. (1994) J. Biol. Chem. 269, 12918; Kichler et al. (1997) Bioconj. Chem. 8, 213) oder beispielsweise Liposomen (Uhlmann und Peymann (1990) supra). Eine andere besonders geeignete Form von gentherapeutischen Vektoren läßt sich dadurch erhalten, daß man die verwendeten Nukleinsäuren auf Goldpartikeln aufbringt und diese mit Hilfe der sogenannten "Gene Gun" in Gewebe, bevorzugt in die Haut, oder Zellen schießt (Wang et al., 1999, J. Invest. Dermatol., 112:775-81, Tuting et al., 1998, J. Invest. Dermatol. 111:183-8 ). Beim Beschießen der Haut werden bevorzugt epidermale Zellen und Keratinozyten, die den Hauptbestandteil der Epidermis bilden, transfiziert (Udvardi et al., 1999, J. Mol. Med. 77:744-750; Lu und Goldsmith, 1996, Proc. Assoc. Am. Physicians, 108: 165-172).

Eine weitere Form eines gentherapeutisch wirksamen Vektors läßt sich durch das Einbringen von "nackten" Expressionsvektoren in eine biokompatible Matrix, beispielsweise eine Kollagenmatrix, herstellen. Diese Matrix kann in Haut mit gestörter Keratinozytenaktivität, beispielsweise eine Wunde eingebracht werden, um die einwandernden Zellen mit dem Expressionsvektor zu transfizieren und die erfindungsgemäß verwendeten Polypeptide in den Zellen zu exprimieren (Goldstein und Banadio, US 5,962,427).

Für die gentherapeutische Anwendung der vorangehend beschriebenen Nukleinsäure ist es auch von Vorteil, wenn der Teil der Nukleinsäure, der für das Polypeptid kodiert, ein oder mehrere nicht kodierende Sequenzen einschließlich Intronsequenzen, vorzugsweise zwischen Promotor und dem Start-Codon des Polypeptids, und/oder eine polyA-Sequenz, insbesondere die natürlich vorkommende polyA-Sequenz oder eine SV40 Virus polyA-Sequenz, vor allem am 3'-Ende des Gens enthält, da hierdurch eine Stabilisierung der mRNA erreicht werden kann (Jackson, R. J. (1993) Cell 74, 9-14 und Palmiter, R. D. et al. (1991) Proc. Natl. Acad. Sci. USA 88, 478-482).

Die Erfindung beschreibt ferner eine Wirtszelle, vorzugsweise eine Hautzelle, insbesondere eine Keratinozyte, die mindestens einen Vektor und/oder mindestens ein "knock-out"-Genkonstrukt, der(das) eine Nukleinsäure oder Varianten davon, die für ein Polypeptid oder funktionelle Varianten davon gemäß SEQ ID No. ID Nr. 3 oder SEQ ID No. ID Nr. 4 kodiert, einschließt. "Knock-out"-Genkonstrukte sind dem Fachmann zum Beispiel aus den US-Patenten US 5,625,122; US 5,698,765; US 5,583,278 und US 5,750,825 bekannt.

Wirtszellen können sowohl prokaryotische als auch eukaryotische Zellen sein, Beispiele für prokaryotische Wirtszellen sind *E. coli* und für eukaryotische Zellen *S. cerevisiae* oder Insektenzellen.

Die Wirtszellen können zur Diagnose, Prävention, und/oder Behandlung von Erkrankungen, die mit gestörter Keratinozytenaktivität in Zusammenhang stehen, insbesondere von Wundheilungsstörungen und/oder Psoriasis oder zur Identifizierung pharmakologisch aktiver Substanzen verwendet werden.

Eine weitere transformierte Wirtszelle ist eine transgene embryonale nichtmenschliche Stammzelle, die mindestens eine Nukleinsäure oder Variante davon, welche für ein Polypeptid oder funktionelle Variante davon gemäß SEQ ID No. ID Nr. 3 oder SEQ ID No. ID Nr. 4 kodiert, enthält. Vorteilhafte weist ist die vorangehende Nukleinsäure in einem "knock-out"-Genkonstrukt oder einer Expressionskassette oder eine vorangehend beschriebene Stammzelle enthalten. Verfahren zur Transformation, Transfektion oder Infektion von Wirtszellen und/oder Stammzellen sind dem Fachmann gut bekannt und schließen zum Beispiel Elektroporation oder Mikroinjektion ein.

Die Erfindung beschreibt ferner ein transgenes nichtmenschliches Säugetier, das mindestens ein vorangehend beschriebenes "knock-out"-Genkonstrukt oder eine vorangehend beschriebene Expressionskassette, vorzugsweise ins Genom integriert, enthält. Transgene Tiere zeigen im allgemeinen eine gewebespezifisch erhöhte Expression der Nukleinsäuren und/oder Polypeptide und lassen sich zur Analyse und/oder Diagnose von Erkrankungen, die mit gestörter Keratinozytenaktivität in Zusammenhang stehen, insbesondere von Wundheilungsstörungen und/oder Psoriasis, verwenden. So weist beispielsweise eine Activin A transgene Maus eine verbesserte Wundheilung auf (Munz et al., 1999, EMBO J. 18:5205-15) während eine transgene Maus mit dominant negativem KGF-Rezeptor eine verzögerte Wundheilung aufweist (Werner et al., 1994, Science 266:819-22).

Verfahren zur Herstellung von transgenen Tieren, insbesondere der Maus, sind dem Fachmann ebenfalls aus der DE 196 25 049 und den US 4,736,866; US 5,625,122; US 5,698,765; US 5,583,278 und US 5,750,825 bekannt und beinhalten transgene Tiere, die beispielsweise über direkte Injektion von Expressionsvektoren (s.o.) in Embryonen oder Spermatozyten, über die Transfektion von Expressionsvektoren in embryonale Stammzellen (Polites und Pinkert: DNA Microinjection and Transgenic Animal Produktion, Seite 15 bis 68 in Pinkert, 1994: Transgenic animal technology: a laboratory handbook, Academic Press, London, UK; Houdebine, 1997, Harwood Academic Publishers, Amsterdam, The Netherlands; Doetschman: Gene Transfer in Embryonic Stern Cells, Seite 115 bis 146 in Pinkert, 1994, supra; Wood: Retrovirus-Mediated Gene Transfer, Seite 147 bis 176 in Pinkert, 1994, supra; Monastersky: Gene Transfere Technology. Alternative Techniques and Applications, Seite 177 bis 220 in Pinkert, 1994, supra) oder über Isolation von Zellkernen aus differenzierten somatische Zellen, beispielsweise Fibroblasten, und anschliessendem Transfer in entkernte Oozyten (Mc Creath et al., 2000, Nature 405: 1004-5) erzeugt werden können. So weisen bekannte "knock-out"-Mausmodelle, wie beispielsweise die eNOS- (Lee et al., 1999, Am. J. Physiol. 277:H1600-H1608), Nf-1 (Atit et al., 1999, J. Invest. Dermatol. 112:835-42) und Osteopontin (Liaw et al., 1998, J. Clin. Invest. 101:967-71) "knock-out"-Mäuse, eine verschlechterte Wundheilung auf.

Gleichermaßen können vorliegend verwendbare Nukleinsäuren in sogenannte "Targeting"-Vektoren integriert (Pinkert, 1994, supra) können nach Transfektion von embryonalen Stammzellen und homologer Rekombination beispielsweise "knock-out"-Mäuse generiert werden, die im allgemeinen als heterozygote Mäuse verringerte Expression der Nukleinsäure zeigen, während homozygote Mäuse keine Expression der Nukleinsäure mehr aufweisen. Dabei ist eine gewebespezifische Reduktion der Expression verwendeter Gene, beispielsweise in hautspezifischen Zellen, insbesondere in Keratinozyten unter Verwendung des Cre-loxP Systems (stat3 knock-out, Sano et al., EMBO J 1999 18: 4657-68), besonders zu bevorzugen. Die so erzeugten transgenen und "knock-out"-Zellen oder Tiere lassen sich beispielsweise zur Analyse von Erkrankungen, die mit gestörter Keratinozytenaktivität in Zusammenhang stehen, beispielsweise einer Wunde verwenden. Sie lassen sich aber beispielsweise auch zum Screening und zur Identifizierung von pharmakologisch, aktiven Substanzen bzw. gentherapeutisch aktiven Vektoren verwenden.

Die Erfindung beschreibt ferner ein Verfahren zur Herstellung eines Polypeptids zur Diagnose und/oder Prävention und/oder Behandlung von Erkrankungen die mit gestörter Keratinozytenaktivität in Zusammenhang stehen, insbesondere von Wundheilungsstörungen und/oder Psoriasis, oder zur Identifizierung von pharmakologisch aktiven Substanzen in einer geeigneten Wirtszelle, das dadurch gekennzeichnet ist, daß eine vorangehend beschriebene Nukleinsäure verwendet wird.

Das Polypeptid wird beispielsweise durch Expression der vorangehend beschriebenen Nukleinsäure in einem geeigneten Expressionssystem, wie oben bereits dargestellt, nach dem Fachmann allgemein bekannten Methoden hergestellt. Als Wirtszellen eignen sich beispielsweise die *E. coli* Stämme DHS, HB101 oder BL21, der Hefestamm *S. cerevisiae,* die Insektenzellinie Lepidopteran, z. B. von *Spodoptera frugiperda*, oder die tierischen Zellen COS, Vero, 293, HaCaT, und HeLa, die alle allgemein erhältlich sind.

Die Erfindung beschreibt ferner ein Verfahren zur Herstellung eines Fusionsproteins zur Diagnose und/oder Prävention und/oder Behandlung von Erkrankungen, die mit gestörter Keratinozytenaktivität in Zusammenhang stehen, oder zur Identifizierung von pharmakologisch aktiven Substanzen in einer geeigneten Wirtszelle, bei dem eine vorangehend beschriebene Nukleinsäure verwendet wird.

Beschrieben ist ferner eine funktionelle Variante der erfindungsgemäß verwendbaren Polypypetide ein Fusionsprotein, d.h. Fusionsproteine enthaltend mindestens ein Polypeptid gemäß einer SEQ ID Nr. 3 oder SEQ ID Nr. 4 oder eine funktionelle Varianten davon. Diese Fusionsproteine können zur Diagnose, Prävention, und/oder Behandlung von Erkrankungen, die mit gestörter Keratinozytenaktivität in Zusammenhang stehen, insbesondere von Wundheilungsstörungen und/oder Psoriasis, oder zur Identifizierung pharmakologisch aktiver Substanzen verwendet werden.

Hergestellt werden hierbei Fusionsproteine, die die oben beschriebenen Polypeptide enthalten, wobei die Fusionsproteine selbst bereits die Funktion eines vorangehend beschriebenen Polypeptids aufweisen oder erst nach Abspaltung des Fusionsanteils die spezifische Funktion funktionell aktiv sind. Vor allem zählen hierzu Fusionsproteine mit einem Anteil von ca. 1- 300, vorzugsweise ca. 1-200, besonders bevorzugt ca. 1-150, insbesondere ca. 1-100, vor allem ca. 1-50 fremden Aminosäuren. Beispiele solcher Peptidsequenzen sind prokaryotische Peptidsequenzen, die z. B. aus der Galactosidase von *E. coli* abgeleitet sein können. Weiterhin können auch virale Peptidsequenzen, wie zum Beispiel vom Bakteriophagen M13 verwendet werden, um so Fusionsproteine für das dem Fachmann bekannte

"phage display"-Verfahren zu erzeugen.

Weitere Beispiele für Peptidsequenzen für Fusionsproteine sind Peptide, die die Detektion des Fusionsproteins erleichtern, hierzu zählen beispielsweise "Green-fluorescent-protein" oder Varianten davon.

Zur Aufreinigung der vorangehend beschriebenen Proteine kann ein weiteres/weiterer Polypeptid("tag") angefügt sein. Geeignete Protein-Tags erlauben beispielsweise die hochaffine Absorption an eine Matrix, stringentes Waschen mit geeigneten Puffern, ohne den Komplex in nennenswertem Maße zu eluieren und anschließend gezielte Elution des absorbierten Komplexes. Beispiele der dem Fachmann bekannten Protein-Tags sind $(His)_6$-Tag, Myc-tag, FLAG-Tag, Hämagluteinin-Tag, Glutathion-Transferase (GST)-Tag, Intein mit einem Affinitäts-Chintin-binding-Tag oder Maltose-binding protein (MBP)-Tag. Diese Protein-Tags können sich N-, C-terminal und/oder innerhalb des Proteins befinden.

Die Erfindung beschreibt ferner ein Verfahren zur Herstellung eines Antikörpers, vorzugsweise eines polyklonalen oder monoklonalen Antikörpers zur Diagnose, zur Prävention und/oder Behandlung von Erkrankungen, die mit gestörter Keratinozytenaktivität in Zusammenhang stehen, insbesondere von Wundheilungsstörungen und/oder Psoriasis, oder zur Identifizierung von pharmakologisch aktiven Substanzen, bei dem ein Polypeptid oder funktionelle Äquivalente davon oder Teile davon mit mindestens 6 Aminosäuren, vorzugsweise mit mindestens 8 Aminosäuren, insbesondere mit mindestens 12 Aminosäuren verwendet wird.

Das Verfahren erfolgt nach dem Fachmann allgemein bekannten Methoden durch Immunisieren eines Säugetiers, beispielsweise eines Kaninchens, mit dem vorangehend beschriebenen Polypeptid oder den genannten Teilen davon, gegebenenfalls in Anwesenheit von z. B. Freund's Adjuvant und/oder Aluminiumhydroxidgelen (siehe z. B. Diamond, B. A. et al. (1981) The New England Journal of Medicine, 1344-1349). Die im Tier aufgrund einer immunologischen Reaktion entstandenen polyklonalen Antikörper lassen sich anschließend nach allgemein bekannten Methoden leicht aus dem Blut isolieren und z. B. über Säulenchromatographie reinigen. Monoklonale Antikörper können beispielsweise nach der bekannten Methode von Winter & Milstein (Winter, G. & Milstein, C. (1991) Nature, 349, 293-299) hergestellt werden.

Beschrieben ist ferner ein Antikörper zur Analyse, Diagnose, zur Prävention und/oder Behandlung von Erkrankungen, die mit gestörter Keratinozytenaktivität in Zusammenhang stehen, insbesondere von Wundheilungsstörungen und/oder Psoriasis, oder zur Identifizierung von pharmakologisch aktiven Substanzen, die gegen ein vorangehend beschriebenes Polypeptid oder funktionelle Varianten davon, gerichtet sind und mit den vorangehend beschriebenen Polypeptiden spezifisch reagieren, wobei die oben genannten Teile des Polypeptids entweder selbst immunogen sind oder durch Kopplung an geeignete Träger, wie z. B. bovines Serumalbumin, immunogen gemacht bzw. in ihrer Immunogenität gesteigert werden können. Dieser Antikörper ist entweder polyklonal oder monoklonal, bevorzugt ist ein monoklonaler Antikörper. Unter dem Begriff Antikörper versteht man gemäß der vorliegenden Erfindung auch gentechnisch hergestellte und gegebenenfalls modifizierte Antikörper bzw. antigenbindende Teile davon, wie z.B. chimäre Antikörper, humanisierte Antikörper, multifunktionelle Antikörper, bi- oder oligospezifische Antikörper, einzelsträngige Antikörper, F(ab)- oder $F(ab)_2$-Fragmente (siehe z.B. EP-B1-0 368 684, US 4,816,567, US 4,816,397, WO 88/01649, WO 93/06213, WO 98/24884).

Beschrieben ist ferner die Verwendung eines Antikörpers, der gegen ein Polypeptid oder funktionelle Varianten davon gemäß SEQ ID No. ID Nr. 3 oder SEQ ID No. ID Nr. 4 gerichtet ist, zur Diagnose und/oder Prävention und/oder Behandlung von Erkrankungen, die mit gestörter Keratinozytenaktivität in Zusammenhang stehen, insbesondere von Wundheitungsstörungen und/oder Psoriasis, besonders bevorzugt von Psoriasis.

So kann beispielsweise die lokale Injektion von monoklonalen Antikörpern gegen TGF-β1 im Tiermodell die Wundheilung verbessern (Ernst et al., 1996, Gut 39:172-5).

Die Erfindung beschreibt die Verwendung von Modulatoren der Aktivität eines Polypeptids oder funktionelle Varianten davon gemäß SEQ ID Nr. 3 oder SEQ ID Nr. 4, vorzugsweise eines Polypeptids gemäß SEQ ID Nr. 3 oder SEQ ID Nr. 4 zur Diagnose und/oder Prävention und/oder Behandlung von Erkrankungen, die mit gestörter Keratinozytenaktivität in Zusammenhang stehen.

Der Begriff "Modulator" im Sinne der Erfindung schließt chemische Substanzen, Verbindungen, Zusammensetzungen und Gemische ein, die die die Aktivität des erfindungsgemäßen Ionenkanals IK beeinflußen, indem er ihn entweder öffnet (=aktiviert) oder schließt (=inhibiert). Weiterhin kann der Modulator die Kinetik der Kanalaktivität modulieren. Ein Modulator kann ein Inhibitor sein, der die Aktivität des Ionenkanals inhibiert oder ein Aktivator, der die Aktivität des Ionenkanals aktiviert.

Weiterhin schließt der Begriff "Modulator" auch Verbindungen ein, Zusammensetzungen und Gemische, die die Expression der Polypeptide nach der SEQ ID Nr. 3 oder SEQ ID Nr. 4 oder funktionelle Varianten davon modulieren. Die Modulation kann z.B. auf transkriptionaler oder translationaler Ebene stattfinden. Substanzen, die die Menge der erfindungsgemäß verwendbaren Polypeptide erhöhen, sind Aktivatoren, während Substanzen, die die Menge der erfindungsgemäß verwendbaren Polypeptide reduzieren, Inhibitoren sind.

Methoden zur Bestimmung der Kanalaktivität sind dem Fachmann aus der Literatur bekannt. IK-Kanäle wurden beispielsweise mittels in vitro Transkription und Injektion in Oocyten exprimiert und die Ionenkanalaktivität mittels

"Patch-Clamp"-Methode gemessen (WO 98/11139; Gerlach et al., 2000, J. Biol. Chem., 275: 585-598; Khanna et al., 1999, J. Biol. Chem., 274: 14838-14849). Weiterhin können IK-Kanäle in humanen Zellinien exprimiert und mittels der "Patch-Clamp"-Methode analysiert werden (WO 00/37422; WO 99/25347). Auch Zellen mit endogen exprimierten IK-Kanälen können auf diese Weise untersucht werden (Gerlach et al., 2000, J. Biol. Chem., 275: 585-598). In analoger Weise können endogen oder mittels Transfektion in Hautzellen, beispielsweise Keratinozyten, exprimierte IK-Kanäle verwendet werden. Weitere Methoden, die zur Identifizierung von Modulatoren von Kanälen geeignet sind, sind beispielsweise radioaktive $Ru^+$-Fluxtestsysteme und Fluoreszenztestsysteme mit spannungsabhängigen Farbstoffen (Vestergaard-Bogind et al., 1988, J. Membr. Biol. 88: 67-75; Daniel et al., 1991, J. Pharmacol. Meth. 25: 185-193; Holevinsky et al. 1994, J. Membr. Biol. 137: 59-70). Beispielsweise kann ein FLIPR-Testsystem ("Fluorescence Image Plate Reader") verwendet werden (WO 99/25347). Testsysteme zum Test der inhibitorischen oder aktivierenden Wirkung einer Substanz auf den Kalium-Flux durch einen IK-Kanal können durch Zugabe der Substanzen zu einer Lösung die in Kontakt mit IK-Kanal exprimierenden Zellen steht, durchgeführt werden (z. B. Blatz et al., 1986, Nature, 323: 718-720; Park, 1994, J. Physiol., 481: 555-570).

Allgemein kann die Aktivität eines Kanals durch die Messung des elektrischen Stroms oder des Ionenflusses oder der sekundären Effekte des elektrischen Stroms oder des Ionenflusses bestimmt werden. Modulatoren der Ionenhanalaktivität ändem den elektrischen Strom oder den Ionenfluß. Auf diese Weise können zu testende Substanzen auf ihre modulatorischen Eigenschaften hin geprüft werden. Änderungen des Kationenfluxes durch den Kanal können beispielsweise direkt durch Bestimmung der Konzentrationsänderungen der Ionen oder indirekt durch die Messung des Membranpotentials durch radioaktive Markierung der Ionen nachgewiesen werden. Wird der Einfluß der Modulatoren an intakten Zellen oder Organismen nachgewiesen, können verschiedenste sekundäre physiologische Effekte, gemessen werden. Mögliche Effekte sind transkriptionelle Änderungen, Zellvolumenänderungen, Änderungen des Zellmetabolismus, Freisetzung von Hormonen etc (WO 98/11139). Methoden zum Screenen einer chemischen Substanz auf eine modulatorische Wirkung auf einen IK-Kanal sind in WO 99/25347 offenbart.

Für keinen der bekannten Modulatoren der Polypeptide gemäß SEQ ID Nr. 3 oder SEQ ID Nr. 4 ist bisher ein Zusammenhang mit Erkrankungen, die mit gestörter Keratinozytenaktivität in Zusammenhang stehen, insbesondere von Wundheilungsstörungen und/oder Psoriasis, beispielsweise bei einer gestörten Wundheilung, beschrieben oder nahegelegt worden. Es war daher unerwartet, daß diese Verbindungen erfindungsgemäß verwendet werden können.

Im Rahmen der Erfindung handelt es sich bei dem Modulator um einen Inhibitor.

Zur Modulation der IK-Kanäle kann mindestens ein Antikörper, der gegen ein Polypeptid oder funktionelle Varianten davon gemäß SEQ ID No. ID Nr. 3 oder SEQ ID No. ID Nr. 4 gerichtet ist, verwendet werden. Dabei können die Antikörper inhibierend oder aktivierend auf die Aktivität des verwendbaren Polypeptids wirken.

Inhibitoren der IK-Kanal Aktivität sind beispielsweise aus WO 99/25347 bekannt. Beispielsweise kann ein symmetrisches oder asymmetrisches Derivat der 1,4-Dihydropyridin-3,5-dicarbonsäure gemäß der allgemeinen Formel (I):

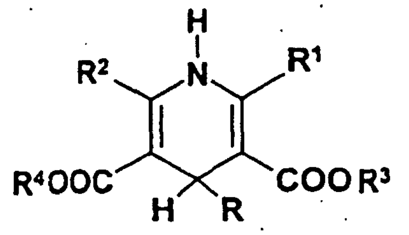

(I)

oder ein pharmazeutisch verträglichesSalz, ein Oxid oder ein Hydrat davon erfindungsgemäß verwendet werden; wobei

R eine Alkylgruppe oder Cycloalkylgruppe bedeutet, die gegebenenfalls mit Halogen, vorzugsweise mit F oder Cl, substituiert ist;

oder R eine mono- oder polycyclische Arylgruppe darstellt, wobei die Arylgruppe durch einen Substituenten ausgewählt aus Halogen-, insbesondere F oder Cl, Trifluormethyl ($-CF_3$), Nitro ($-NO_2$), Cyano (-CN), Azido ($-N_3$), einer Gruppe der Formel $-S(O)_n$-alkyl, $-S(O)_n$-NH-alkyl, oder $-S(O)_n$-N-(alkyl)$_2$, wobei n vorzugsweise 0, 1 oder 2 sein kann, einer Alkylgruppe, einer Cycloalkylgruppe, einer Alkoxygruppe, einer Trifluormethyl-oxygruppe ($-OCF_3$), einer Carboxygruppe (-COOH), einer Gruppe der Formel -COO-Alkyl, einer Carbamoyl Gruppe ($-CONH_2$), und einer Gruppe der Formel -CONH-Alkyl oder CON(Alkyl)$_2$ einfach oder mehrfach substituiert sein kann;

oder R eine mono- oder poly-heterozyklische Gruppe darstellt, wobei die heterozyklische Gruppe einfach oder mehrfach mit einer Alkylgruppe, einer Alkoxygruppe, einer Carboxygruppe (-COOH), einer Gruppe der Formel -COO-Alkyl,

und/oder einer Gruppe der Formel -COO-Phenyl substituiert sein kann, bevorzugte Heteroatome sind N, S und O; und $R^1$, $R^2$, $R^3$ and $R^4$, unabhängig voneinander, Wasserstoff, eine Alkylgruppe, eine Cycloalkylgruppe, eine Alkenylgruppe, eine Alkinylgruppe, eine Alkoxygruppe, eine Phenylgruppe, eine Phenyl-Alkylgruppe, eine Furanylgruppe, eine Furanyl-Alkylgruppe, eine Pyridylgruppe oder eine Pyridyl-Alkylgruppe, die gegebenenfalls mit Halogen, vorzugsweise mit F oder CL substituiert ist, darstellt.

Beschrieben ist die Verwendung eines Inhibitors der allgemeinen Formel (I), bei der R eine $C_{3-7}$ Cycloalkylgruppe, insbesondere eine Cyclohexylgruppe; oder eine Phenylgruppe, vorzugsweise eine monosubstituierte Phenylgruppe, darstellt, wobei die Phenylgruppe einfach oder mehrfach mit einem Substituent ausgewählt aus der Gruppe bestehend aus Halogen, Trifluormethyl ($-CF_3$), Nitro ($-NO_2$), Cyano (-CN);
oder R ist eine Pyridyl- oder eine Dihydropyridylgruppe ist, wobei diese Gruppe mit einer Gruppe der Formel -COO-Alkyl oder einer Gruppe der Formel -COO-Phenyl monosubstituiert sein können.

Es kann ein Inhibitor nach der allgemeinen Formel (I) verwendet werden, wobei R eine 2-Nitrophenylgruppe, eine 3-Nitrophenylgruppe, eine 4-Nitrophenylgruppe, eine 2-Trifluormethylphenylgruppe, eine 3-Trifluormethylphenylgruppe, eine 4-Trifluorethylphenylgruppe, eine 2-Cyanophenylgruppe, eine 3-Cyanophenylgruppe, eine 4-Cyanophenylgruppe darstellt;
oder R eine 2-Pyridyl, 4-Pyridyl, 3-Pyridyl, eine 1,2- oder 1,4- oder 1,6-Dihydro-2-pyridyl-, eine 1,2- oder 1,4- oder 1,6-Dihydro-3-pyridyl-, eine 1,2- oder 1,4- oder 1,6-Dihydro-4-pyridyl-Gruppe darstellt, wobei die Pyridyl- oder Dihydropyridylgruppen mit $C_{1-6}$-Alkyl, einer Gruppe der Formel -COO-$C_{1-6}$-Alkyl oder einer Gruppe der Formel -COO-Phenyl monosubstituiert sein können.

Es kann ein Modulator der allgemeinen Formel (I) verwendet werden, wobei $R^1$, $R^2$, $R^3$, und $R^4$ unabhängig voneinander $C_{1-6}$-Alkyl, vorzugsweise Methyl, Ethyl, Propyl, Isopropyl, Butyl oder Isobutyl bedeuten.

Beschrieben ist die Verwendung eines Inhibitors der allgemeinen Formel (I), wobei der Inhibitor ein asymmetrisches Derivat der 1,4-Dihydropyridin-3,5-dicarbonsäure gemäß der allgemeinen Formel (I) ist. Bevorzugt asymmetrische Derivate sind asymmetrische $C_{1-6}$-Alkyl Derivate der 1,4-Dihydropyridin-3,5-dicarbonsäure (I). Beispielsweise können folgende Substanzen verwendet werden:

1,4-Dihydro-2,6-dimethyl-4-(2-nitrophenyl)-pyridin-3,5-dicarbonsäureethylmethylester;

1,4-Dihydro-2,6-dimethyl-4-(2-nitrophenyl)-pyridin-3,5-dicarbonsäurepropylmethylester;

1,4-Dihydro-2,6-dimethyl-4-(3-nitrophenyl)-pyridin-3,5-dicarbonsäureethylmethylester.

Ferner ist die Verwendung eines symmetrischen Derivats der 1,4-Dihydropyridin-3,5-dicarbonsäure dargestellt durch die allgemeine Formel (I) beschrieben. Mögliche Verbindungen enthalten symmetrische $C_{1-6}$-Alkyl Derivate der 1,4-Dihydropyridin-3,5-dicarbonsäure. Beispielsweise können die folgenden Substanzen verwendet werden:

1,4-Dihydro-2,6-dimethyl-4-(2-nitrophenyl)-pyridin-3,5-dicarbonsäuredimethylester (Nifedipin);

1,4-Dihydro-2,6-dimethyl-4-(2-nitrophenyl)-pyridin-3,5-dicarbonsäurediethylester.

Ferner können Inhibitoren verwendet werden, die Derivate oder Metabolite von Clotrimazol gemäß der allgemeinen Formel (II) (siehe auch WO 99/25347)

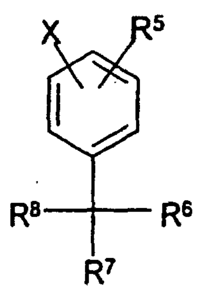

(II)

oder ein pharmazeutisch verträgliches Salz, ein Oxid oder ein Hydrat davon sind;
wobei
X Halogen, insbesondere F oder Cl, eine Trifluormethylgruppe, eine Nitrogruppe- oder eine Cyanogruppe bedeutet;

$R^5$ Wasserstoff, Halogen, insbesondere F oder Cl, Hydroxy, eine Alkylgruppe, eine Cycloalkylgruppe, eine Alkoxygruppe, oder eine Alkyloxygruppe, gegebenenfalls mit Halogen, vorzugsweise F oder Cl, substitutiert, bedeutet;
$R^6$ Wasserstoff, oder eine Phenylgruppe bedeutet, wobei die Phenylgruppe ein- oder mehrfach substituiert sein kann mit Substituenten ausgewählt aus Halogen, vorzugsweise F oder Cl, und Hydroxy;
$R^7$ Wasserstoff, Halogen, vorzugsweise F oder Cl, Hydroxy, Alkyl oder Alkoxy, gegebenenfalls mit Halogen, vorzugsweise F oder Cl, substitutiert, bedeutet;
$R^8$ stellt eine Gruppe der Formel $-Y-CH_2-R^9$ dar, wobei Y Sauerstoff (-O-) oder Schwefel (-S-) darstellt; eine Gruppe der Formel $=NO-CH_2-R^9$; eine Gruppe der Formel $-O-Phenyl-CH=CH_2$; eine Gruppe der Formel $-CH_2-CH(CH_3)-S-Phenyl$, wobei die Phenylgruppe ein- oder mehrfach substituiert sein kann mit Substituenten ausgewählt aus Halogen, vorzugsweise F oder Cl, und Hydroxy; oder eine Phenylgruppe, wobei die Phenylgruppe ein- oder mehrfach substituiert sein kann mit Substituenten ausgewählt aus Halogen, vorzugsweise F oder Cl, und Hydroxy; und wobei $R^9$ eine Ethenylgruppe ($CH_2=CH-$); eine Phenylgruppe, wobei die Phenylgruppe ein- oder mehrfach mit Substituenten ausgewählt aus Halogen, vorzugsweise F oder Cl, und Hydroxygruppe substituiert sein kann; eine Phenyl-S-Phenylgruppe; eine Gruppe der Formel $CH_2-O-Phenyl$, wobei die Phenylgruppe ein- oder mehrfach substituiert sein kann mit Substituenten ausgewählt aus Halogen, vorzugsweise F oder Cl, und Hydroxygruppe; oder eine Gruppe der allgemeinen Formel (VII),

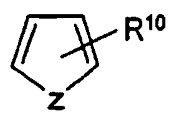

(VII)

wobei Z S, O, oder N bedeutet;
und $R^{10}$ Wasserstoff, Halogen, vorzugsweise F oder Cl, oder Hydroxygruppe bedeutet.

Beschrieben ist die Verwendung folgender Derivate und Metabolite:

2-Chlorphenyl-4-hydroxyphenyl-phenyl-methan; 2-Chlorphenyl-bis-phenylmethan; 2-Chlorphenyl-bis-phenyl-methanol; 3-(1-[2,4-dichlorphenyl]-ethoxymethyl)-2-chlorthiophen; O-(2,4-dichlorbenzyl)-2,4-dichloracetophenonoxim; 1-(2,4-dichlor)-1-(4-(phenylthio)-benzyloxy)-ethan; 1-(2,4-dichlorphenyl)-1-1-(allyloxy)-ethan; 1-(2,4-dichlorphenyl)-1-(4-chlorbenzylthio)-ethan; 1-(2,4-dichlorphenyl)-1-(2,4-dichlorbenzyloxy)-ethan; 1-(2,4-dichlorphenyl)-etyl-2,6-dichlorbenzylether; 1-(2-[4-chlorphenoxy]-ethyloxy)-1-(2,4-dichlorphenyl)-propen; 1-(2,4-dichlorphenyl)-ethyl-(4-chlorphenyl)-methylether; 3-Chlorbenzyl-2-vinylphenylether und 1-(4-chlorphenyl)-3-(2,6-dichlorphenytthio)-butan.

Das Dokument WO 99/25347 offenbart weitere Substanzen, die als Inhibitoren von hIK1 wirken und verwendet werden können. Beispiele sind die Imidazol-Derivate Miconazol, Econazol, Butoconazol, Oxiconazol, Sulconazol, Thioconazol, die Triazol-Derivate Fluconazol, Terconazol, Itraconazol, die Nitroimidazol Derivate Metronidazol, Tinidazol, Nimorazol, Omidazol und Benznidazol.

Weitere geeignete verwendbare Modulatoren sind Oxim-Derivate gemäß der allgemeinen Formel (III) (siehe WO 00/34228):

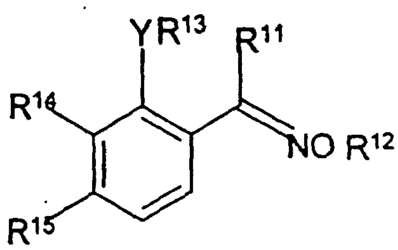

(III)

oder ein pharmazeutisch verträgliches Salz, ein Oxid oder ein Hydrat davon;
wobei
Y Sauerstoff, Schwefel oder eine Aminogruppe, vorzugsweise eine Aminogruppe der Formel $NHR^{13}$ darstellt;
$R^{11}$ Wasserstoff; eine Alkylgruppe; eine Cycloalkylgruppe; eine Hydroxygruppe; eine Alkoxygruppe; eine Acylgruppe; eine Phenylgruppe oder eine Benzylgruppe, wobei die Gruppe, insbesondere die Phenyl- oder Benzylgruppe einfach

oder mehrfach mit Substituenten substituiert sein kann, ausgewählt aus Halogen, insbesondere F oder Cl, $-CF_3$, $-NO_2$, -CN, Alkyl-, Cycloalkyl-, Hydroxy- und Alkoxygruppe; eine Gruppe der Formel $CH_2CN$; eine Gruppe der Formel $CH_2CO_2R'$, wobei R' Wasserstoff oder eine Alkylgruppe darstellt; eine Gruppe der Formel $CH_2CONR^{IV}R^{V}$, wobei $R^{IV}$ und $R^{V}$ unabhängig voneinander Wasserstoff oder eine Alkylgruppe darstellen, gegebenenfalls mit Halogen substituiert; oder eine Gruppe der Formel $-CH_2C(=NOH)NH_2$ bedeutet;
$R^{12}$ Wasserstoff; eine Alkylgruppe; eine Cycloalkylgruppe; eine Phenyl- oder eine Benzylgruppe bedeutet, wobei die Gruppe, insbesondere die Phenyl- oder Benzylgruppe, einfach oder mehrfach mit Substituenten ausgewählt aus Halogen, insbesondere F oder Cl, $-CF_3$, $-NO_2$, -CN, Alkyl-, Cycloalkyl-, Hydroxy- und Alkoxygruppe substituiert sein kann;
$R^{13}$ Wasserstoff; eine Alkylgruppe; eine Cycloalkylgruppe; eine Phenyl- oder eine Benzylgruppe bedeutet, wobei die Gruppe, insbesondere die Phenyl- oder Benzylgruppe, einfach oder mehrfach mit Substituenten ausgewählt aus Halogen, insbesondere F oder Cl, $-CF_3$, $-NO_2$, -CN, Alkyl-, Cycloalkyl-, Hydroxy- und Alkoxygruppe substituiert sein kann; und
$R^{14}$ und $R^{15}$ unabhängig voneinander Wasserstoff; Halogen, insbesondere F oder Cl; $-CF_3$; $-NO_2$; CN; eine Alkylgruppe, eine Alkoxygruppe; eine Phenyl- oder eine Benzylgruppe, wobei die Gruppe, insbesondere die Phenyl- oder Benzylgruppe, einfach oder mehrfach mit Substituenten ausgewählt aus Halogen, insbesondere F oder Cl, $-CF_3$,$-NO_2$, -CN, Alkyl, Cycloalkyl, Hydroxy und Alkoxy substituiert sein kann; oder eine Gruppe der Formel $-SO_2NR''R'''$ bedeuten, wobei R'' und R''' unabhängig voneinander Wasserstoff oder eine Alkylgruppe bedeuten;
oder $R^{14}$ und $R^{15}$ bilden zusammen einen zusätzlichen 4- bis 7-gliedrigen kondensierten Ring, wobei der kondensierte Ring aromatisch oder partiell gesättigt sein kann, und ein- oder mehrfach mit Substituenten ausgewählt aus Halogen, insbesondere F oder Cl, $-CF_3$, $-NO_2$, -CN und einer Gruppe der Formel - $SO_2NR''R'''$ substituiert sein kann, wobei R'' und R''' unabhängig voneinander Wasserstoff oder eine Alkylgruppe bedeuten.

Beim verwendbaren Modulators kann $R^{11}$ Wasserstoff oder eine Alkylgruppe sein. Ferner kann $R^{12}$ Wasserstoff, eine Alkylgruppe oder eine Benzyl- oder Phenylgruppe sein, wobei die wobei die Phenyl- oder Benzylgruppe einfach oder mehrfach mit Substituenten ausgewählt aus Halogen, $-CF_3$, $-NO_2$, - CN, Alkyl, Cycloalkyl, Hydroxy und Alkoxy substituiert sein kann.

Y kann Sauerstoff oder eine Aminogruppe der Formel $NHR^{13}$ darstellen, wobei $R^{13}$ hier Wasserstoff, Alkyl, Benzyl oder Acetyl bedeutet.

$R^{14}$ und $R^{15}$ können unabhängig voneinander Wasserstoff, Halogen, Alkyl oder Alkoxy bedeuten.

$R^{14}$ und $R^{15}$ können zusammen einen zusätzlichen 6-gliedrigen kondensierten Ring bilden, wobei der kondensierte Ring aromatisch oder teilweise gesättigt sein kann, wobei der kondensierte Ring einfach oder mehrfach substituiert sein kann mit Substituenten ausgewählt aus Halogen, insbesondere F oder Cl, $-CF_3$, $-NO_2$, -CN und einer Gruppe der Formel- $SO_2NR''R'''$, wobei R'' und R''' unabhängig voneinander Wasserstoff oder eine Alkylgruppe bedeuten.

Es können folgende Oxim-Derivate als Modulatoren verwendet werden:

2-Aldoximo-1-naphtol; 2-Aldoximo-5,6-dimethylphenol; 2-Aldoximo-5,6-dichlorphenol; O-Benzyl-2-Formyl-5,6-dichlorphenol-oxim; O-Methyl-2-Formyl-5,6-dichlorphenol-oxim; 2-Aldoximo-5,6,7,8-tetrahydro-1-naphtol; 1-Hydroxy-2-acetonaphton-oxim; 1-Methoxy-2-acetonaphton-oxim; O-Ethyl 1-Methoxy-2-acetonaphton-oxim; 1-Ethoxy-2-acetonaphton-oxim; 1-Benzyloxy-2-acetonaphton-oxim; 2-Hydroxy-3,4-dimethylacetophenon-oxim; 2-Methoxy-3,4-dimethylacetophenon-oxim; 2-Hydroxy-3,4-dimethoxyacetophenon-oxim; 2,3,4-Trimethoxyacetophenon-oxim; 1-Hydroxy-5,6,7,8-tetrahydro-2-acetonaphtonoxim; 1-Methoxy-5,6,7,8-tetrahydro-2-acetonaphton-oxim; 1-(2-Propyloxy)-2-acetonaphton-oxim; N-(2-acetylphenyl)-acetamid-oxim.

Ferner können chemische Verbindungen gemäß der allgemeinen Formel (IV) (siehe WO 00/34248)

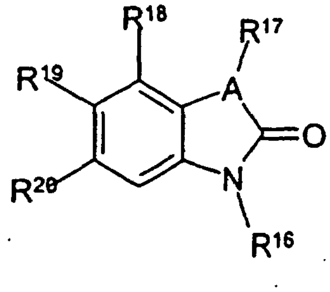

(IV)

oder ein pharmazeutisch verträgliches Salz, ein Oxid oder ein Hydrat davon als Modulatoren verwendet werden; wobei

A Sauerstoff, Schwefel, oder Stickstoff bedeutet;

$R^{16}$ Wasserstoff; eine Alkylgruppe; eine Cycloalkylgruppe; eine Hydroxygruppe; eine Alkoxygruppe; eine Acylgruppe, eine Phenylgruppe oder eine Benzylgruppe dar, wobei die Gruppe, insbesondere die Phenyl- oder Benzylgruppe, einfach oder mehrfach mit Substituenten substituiert sein kann ausgewählt aus Halogen, insbesondere F oder Cl, $-CF_3$, $-NO_2$, -CN, Alkyl, Cycloalkyl, Hydroxy und Alkoxy; eine Gruppe der Formel $CH_2CN$; eine Gruppe der Formel $CH_2CO_2R'$, wobei R' Wasserstoff oder eine Alkylgruppe darstellt; eine Gruppe der Formel $CH_2CONR^{IV}R^{V}$, wobei $R^{IV}$ und $R^{V}$ unabhängig voneinander Wasserstoff oder eine Alkylgruppe darstellen; oder eine Gruppe der Formel $-CH_2C(=NOH)NH_2$ bedeutet;

$R^{17}$ Wasserstoff; eine Alkylgruppe; eine Cycloalkylgruppe; eine Phenyl- oder eine Benzylgruppe bedeutet, wobei die Gruppe, insbesondere die Phenyl- oder Benzylgruppe, einfach oder mehrfach mit Substituenten ausgewählt aus Halogen, insbesondere F oder Cl, $-CF_3$, $-NO_2$, -CN, Alkyl, Cycloalkyl, Hydroxy und Alkoxy substituiert sein kann;

$R^{18}$, $R^{19}$ und $R^{20}$ unabhängig voneinander Wasserstoff; Halogen, insbesondere F oder Cl; $-CF_3$; $-NO_2$; -CN; eine Alkylgruppe; eine Alkoxygruppe; eine Phenyl- oder eine Benzylgruppe, wobei die Gruppe, insbesondere die Phenyl- oder Benzylgruppe, einfach oder mehrfach mit Substituenten ausgewählt aus Halogen, insbesondere F oder Cl, $-CF_3$, $-NO_2$, -CN, Alkyl, Cycloalkyl, Hydroxy und Alkoxy substituiert sein kann; oder eine Gruppe der Formel $-SO_2NR''R'''$ bedeuten, wobei R'' und R''' unabhängig voneinander Wasserstoff oder eine Alkylgruppe, gegebenenfalls substituiert, vorzugsweise mit F oder Cl, bedeuten; oder $R^{20}$ wie oben definiert ist und $R^{18}$ und $R^{19}$ zusammen einen zusätzlichen 4-bis 7-gliedrigen kondensierten Ring bilden, wobei der kondensierte Ring heterozyklisch sein kann, wobei die Heteroatome vorzugsweise N, S oder 0 sind, aromatisch, gesättigt oder partiell gesättigt sein kann, und gegebenenfalls mit Substituenten ausgewählt aus Halogen, insbesondere F oder Cl, $-CF_3$, $-NO_2$, -CN und einer Gruppe der Formel $-SO_2NR''R'''$, wobei R'' und R''' unabhängig voneinander Wasserstoff oder eine Alkylgruppe bedeuten.

Ferner kann $R^{16}$ Wasserstoff oder eine Alkylgruppe darstellen. Ferner kann $R^{17}$ Wasserstoff oder eine Alkylgruppe darstellen. Ferner können $R^{19}$, $R^{20}$ und $R^{18}$ unabhängig voneinander Wasserstoff oder eine Alkylgruppe darstellen.

Möglich ist die Verwendung von 1-Ethyl-4,5-dichlorbenzimidazolon, 1,3-Diethyl-4,5-dichlorbenzimidazolon, 3-Ethyl-5-chlorbenzoxazolon, und der Aktivatoren 1-Ethyl-2-benzimidazolinon (1-EBIO), 1-Ethyl-4,5-dichlor-2-benzirnidazolinon (Boehringer et al., 2000 J. Med. Chem.,43:2664), 5,6-Dichloro-1-ethyl-1,3-dihydro-2H-benzimidazol-2-on und (DCEBIO; Singh et al.,2001, J Pharmacol Exp Ther; 296:600-11). Erfindungsgemäß wird Chlorzoxazon (Syme et al., 2000, Am. J. Physiol. 278: C570-C581) verwendet.

Ferner kann ein Modulator gemäß Formel (IV), verwendet werden, bei dem $R^{18}$ und $R^{19}$ zusammen einen 5- oder 6-gliedrigen kondensierten Ring bilden, wobei der Ring heterozyklisch sein kann; weiterhin kann der Ring aromatisch, gesättigt oder partiell gesättigt sein und der Ring mit Substituenten ausgewählt aus der Gruppe bestehend aus Halogen, insbesondere F oder Cl, $-CF_3$, $-NO_2$, -CN und einer Gruppe der Formel - $SO_2NR''R'''$ kann, gegebenenfalls einfach oder mehrfach substituiert sein, wobei R'' und R''' unabhängig voneinander Wasserstoff oder eine Alkylgruppe, gegebenenfalls substituiert, insbesondere mit F oder Cl, bedeuten.

Möglich ist die Verwendung von 1,3-Diethyl-5,6,7,8-tetrahydronaphto-[1,2-d]-imidazolinon, 1-Ethyl-5,6,7,8-tetrahydronaphto-[1,2-d]-imidazolinon; 3-Ethyl-naphto-[1,2-d]-oxazolinon; 3-Ethyl-naphto-[1,2-d]-imidazolinon; 3-Ethytchinolino-[5,6-d]-imidazolinon, 3-Benzyl-(2, 1, 3-thiadiazolo)-[4,5-g]-benzimidazolon.

Weiterhin kann eine chemische Verbindung gemäß der allgemeinen Formel (V) (siehe WO 00/37422):

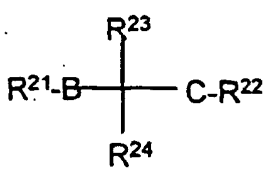

(V)

oder ein pharmazeutisch verträgliches Salz, ein Oxid oder ein Hydrat davon als Modulator verwendet werden; wobei

B und C, unabhängig voneinander eine Gruppe der Formel $-(CH_2)_n-$, der Formel - $(CH_2)_n-Y'-$ (in eine der beiden Richtungen) oder der Formel $-(CH_2)_n-Y'-(CH_2)_m-$ ist, wobei in dieser Formel n und m unabhängig voneinander 0 ,1 ,2 ,3 oder 4 darstellen, Y' O, S oder NR''' darstellt, wobei R''' Wasserstoff oder eine Alkylgruppe ist;

$R^{21}$ und $R^{22}$ unabhängig voneinander Alkyl, Alkenyl, Alkinyl, Cycloalkyl, Amino, Trihalogemnethyl, insbesondere Trifluormethyl oder Triochlormethyl, Nitro, Cyano, Phenyl oder eine Gruppe der Formel OR', -SR', -R'OR'', -R'SR'', - C(O)R', -C(S)R', -C(O)OR', -C(S)OR', -C(O)SR', -C(S)SR', -C(O)NR'(OR''), - C(S)NR'(OR''), -C(O)NR'(SR''), -C(S)NR'

(SR"), -CH(CN)$_2$, -C(S)NR'R", - C(O)NR'R", -CH[C(O)R']$_2$, -CH[C(S)R']$_2$, -CH[C(O)OR']$_2$, -CH[C(S)OR']$_2$, - CH[C(O)SR']$_2$, -CH[C(S)SR']$_2$, CH$_2$OR', CH$_2$SR', -NR'C(O)R", oder OC(O)R' bedeuten;
oder eine ungesättigte, teilweise gesättigte oder gesättigte mono- oder polyzyklische Gruppe, eine Aralkylgruppe oder hetero-Alkylgruppe ist, wobei die mono- oder polyzyklische Gruppen, Aralkylgruppen oder hetero-Alkylgruppen mit einer Gruppe bestehend aus Halogen, insbesondere F oder Cl, Trihalogenmethyl, insbesondere Trifluor- oder Trichlormethyl, Alkyl, Alkenyl, Alkinyl, Amino, Nitro, Cyano oder Amido, oder einer Gruppe der Formel -R', - OR', SR',-R'OR", -R'SR", -C(O)R', -C(S)R', -C(O)OR', -C(S)OR', -C(O)SR', -C(S)SR', oder einer Phenyl- oder Phenoxygruppe, ein- oder mehrfach substituiert sein können, wobei die Gruppe, insbesondere die Phenyl- oder Phenoxygruppe mit einer Gruppe bestehend aus Halogen, insbesondere F oder Cl, Trihalogenmethyl, insbesondere Trifluor- oder Trichlormethyl, Alkyl, Alkenyl, Alkinyl, Amino, Nitro, Cyano oder Amido, oder einer Gruppe der Formel -R', - OR', SR', -R'OR", -R'SR", -C(O)R', -C(S)R', -C(O)OR', -C(S)OR', -C(O)SR', -C(S)SR', -NR'C(O)R", oder OC(O)R' ein- oder mehrfach substituiert sein kann;
wobei
R' und R" unabhängig voneinander Wasserstoff, Alkyl, Alkenyl, Alkinyl, Cycloalkyl, Alkoxy oder Phenyl, gegebenenfalls substituiert, insbesondere mit F oder Cl, darstellen oder eine Gruppe der Formel NR'"R"", wobei R'" und R"" unabhängig voneinander Wasserstoff oder Alkyl bedeuten;
$R^{23}$ und $R^{24}$ unabhängig voneinander, Alkyl, Alkenyl, Alkinyl, Cycloalkyl, Amino, Trihalogenmethyl, insbesondere Trifluor- oder Trichlonnethyl, Nitro, Cyano, Phenyl oder eine Gruppe der Formel OR', -SR', -R'OR", -R'SR", - C(O)R', -C(S)R', -C(O)OR', -C(S)OR', -C(O)SR', -C(S)SR', -C(O)NR'(OR"), - C(S)NR'(OR"), -C(O)NR'(SR"), -C(S)NR'(SR"), -CH(CN)$_2$, -C(S)NR'R", - C(O)NR'R", -CH[C(O)R']$_2$, -CH[C(S)R']$_2$, -CH[C(O)OR']$_2$, -CH[C(S)OR']$_2$, - CH[C(O)SR']$_2$, -CH[C(S)SR']$_2$, CH$_2$OR', CH$_2$SR', -NR'C(O)R", oder OC(O)R' bedeuten;
wobei
R' und R" unabhängig voneinander Wasserstoff, Alkyl, Alkenyl, Alkinyl, Cycloalkyl, Alkoxy, Phenyl oder eine Gruppe der Formel NR'"R"" bedeuten, wobei R'" und R"" unabhängig voneinander Wasserstoff oder Alkyl, gegebenenfalls substituiert, insbesondere mit F oder Cl, bedeuten;
oder $R^{23}$ und $R^{24}$ zusammen eine ungesättigte, partiell gesättigte oder vollständig gesättigte mono- oder polyzyklische Gruppe, oder eine mono- oder poly-heterozyklische Gruppe bilden, wobei das Heteroatom vorzugsweise N, S oder O ist, wobei die mono- oder polyzyklischen Gruppen ein- oder mehrfach substituiert sein können mit einer Gruppe bestehend aus Halogen, insbesondere F oder Cl, Trihalogenmethyl, insbesondere Trifluor- oder Trichlormethyl, Alkyl, Alkenyl, Alkinyl, Amino, Nitro, Cyano oder Amido, oder einer Gruppe der Formel -R', - OR', SR', -R'OR", -R'SR", -C(O)R', -C(S)R', -C(O)OR', -C(S)OR', -C(O)SR', -C(S)SR', oder einer Phenyl- oder Phenoxygruppe, wobei die Gruppe, insbesondere die Phenyl- oder Phenoxygruppe mit einer Gruppe bestehend aus Halogen, insbesondere F oder Cl, Trihalogenmethyl, insbesondere Trifluor- oder Trichlormethyl, Alkyl, Alkenyl, Alkinyl, Amino, Nitro, Cyano oder Amido, oder einer Gruppe der Formel -R', -OR', SR', -R'OR", -R'SR", -C(O)R', -C(S)R', - C(O)OR', -C(S)OR', -C(O)SR', -C(S)SR', -NR'C(O)R", oder OC(O)R' ein- oder mehrfach substituiert sein kann;
wobei
R' und R" unabhängig voneinander Wasserstoff, Alkyl, Alkenyl, Alkinyl, Cycloalkyl, Alkoxy, Phenyl oder eine Gruppe der Formel NR'"R"" bedeuten, wobei R'" und R"" unabhängig voneinander Wasserstoff oder Alkyl, gegebenenfalls substituiert, insbesondere mit F oder Cl, bedeuten.

Ferner kann der verwendbare Modulator ein Malonsäureester-Derivat gemäß der allgemeinen Formel (VIII)

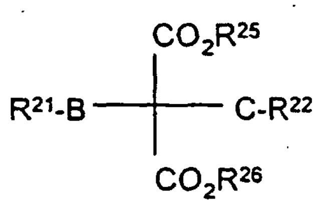

(VIII)

oder ein pharmazeutisch verträgliches Salz, ein Oxid oder ein Hydrat davon sein,
wobei
A, B, $R^{21}$ und $R^{22}$ wie oben definiert sind und $R^{25}$ und $R^{26}$, unabhängig voneinander Wasserstoff, Alkyl, Cycloalkyl, gegebenenfalls substituiert, insbesondere mit F oder Cl, oder eine Gruppe der Formel NR'"R"" bedeuten, wobei R'" und R"" unabhängig voneinander Wasserstoff oder Alkyl, gegebenenfalls substituiert, insbesondere mit F oder Cl, darstellen.

Ferner kann ein Malonsäureester-Derivat wie oben beschrieben verwendet werden, wobei $R^{25}$ und $R^{26}$ zusammen einen heterozyklischen 6-9 gliedrigen Ring bilden und ein Diester Derivat ergeben.

Besonders vorteilhaft ist die Verwendung folgender Malonsäurederivate als Modulatoren der IK-Aktivität:

Diethyl-2-(4-fluorphenyl)-2-(3-picolyl)-malonat; Diethyl-2-(4-nitrophenyl)-2-(2-picolyl)-malonat; Diethyl-2-(4-nitrophenyl)-2-(4-picolyl)-malonat; Diethyl-2-phenyl-2-(3-picolyl)-malonat; Diethyl-2-(5-chlor-2-nitro-4-(trifluormethyl)-phenyl)-2-(3-picolyl)-malonat; Diethyl-2-benzyl-2-(3-picolyl)-malonat; Diethyl-2-(4-nitrophenyl)-2-[(benzotriazol-1-yl)-methyl]-malonat; Diethyl-2-(2-thienyl)-2-(2-picolyl)-malonat; Diethyl-2-(4-(acetylamino)-phenyl)-2-(2-picolyl)-malonat; Diethyl-2-(4-(benzoylamino)-phenyl)-2-(2-picolyl)-malonat oder 2-(4-Nitrophenyl)-2-(2-picolyl)-malononitril.

Weitere Substanzen gemäß der allgemeinen Formel V, deren Verwendung möglich ist, sind 2-(3-Phenoxyphenyl)-butyronitril; 2-(2-Chlorphenyl)-butyronitril; Dicyclopropan-(4-chlorphenyl)-carbinol; Ethyl-1-(4-chlorphenyl)-cyclopentan-1-carboxylat und 1-(4-chlorphenyl)-1-(3-methyl-5-oxadiazolyl)-cyclopentan.

Die verwendeten Modulatoren können als Aktivator der IK-Aktivität wirken.

Isatin-Derivate gemäß folgender allgemeiner Formel (VI) (siehe auch WO 00/33834)

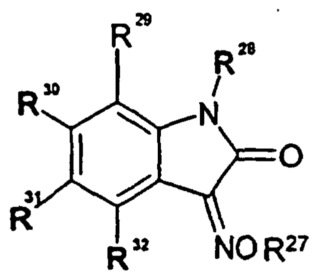

(VI)

oder ein pharmazeutisch verträgliches Salz, ein Oxid oder ein Hydrat davon können als Aktivator verwendet werden; wobei

$R^{27}$ Wasserstoff; eine Alkylgruppe; eine Cycloalkylgruppe; eine Acylgruppe; eine Phenyl- oder Benzylgruppe, wobei die Gruppe, insbesondere die Phenyl- oder Benzylgruppe mit einer Gruppe ausgewählt aus Halogen, insbesondere F oder Cl, $-NO_2$, -CN, $-CF_3$, Alkyl, Cycloalkyl, Hydroxy und Alkoxy ein- oder mehrfach substituiert sein kann; eine Gruppe der Formel $-CH_2CN$; eine Gruppe der Formel $-CH_2CO_2R'$, wobei R' Wasserstoff oder eine Alkylgruppe, gegebenenfalls substituiert, insbesondere mit F oder Cl, bedeutet; eine Gruppe der Formel - $CH_2CONR^{IV}R^{V}$, wobei $R^{IV}$ und $R^{V}$ unabhängig voneinander Wasserstoff, Alkyl, gegebenenfalls substituiert, insbesondere mit F oder Cl, Phenyl- oder Benzylgruppe darstellen, wobei die Phenyl oder Benzylgruppe ein- oder mehrfach mit Halogen und/oder Alkyl substituiert sein kann, oder $R^{IV}$ und $R^{V}$ zusammen mit den N-Atom an dem sie gebunden sind, einen 4- bis 7-gliedrigen monozyklischen Ring bilden, wobei die heterozyklische Gruppe mit einer Gruppe ausgewählt aus Halogen, insbesondere F oder Cl, Alkyl, Cycloalkyl, Alkyloxy, Cycloalkyloxy, Phenyl oder Benzyl ein- oder mehrfach substituiert sein kann; oder eine Gruppe der Formel $-CH_2C(=NOH)NH_2$ bedeutet.

$R^{28}$ Wasserstoff; eine Alkylgruppe; eine Cycloalkylgruppe; eine Gruppe der Formel $-CH_2CO_2R'$, wobei R' Wasserstoff oder eine Alkylgruppe bedeutet; oder eine Phenyl- oder Benzylgruppe bedeutet, wobei die Phenyl- oder Benzylgruppe, gegebenenfalls mit Substituenten ausgewählt aus Halogen, insbesondere mit F oder Cl, $-NO_2$, -CN, $CF_3$, Alkyl, Cycloalkyl, Hydroxy und Alkoxy ein- oder mehrfach substituiert sein kann; und

$R^{29}$, $R^{30}$, $R^{31}$ und $R^{32}$ unabhängig voneinander Wasserstoff; Halogen, insbesondere mit F oder Cl; $-NO_2$; -CN; $CF_3$; Alkyl; eine Alkoxygruppe; eine Phenyl- oder Benzylgruppe, wobei die Phenyl- oder Benzylgruppen ein- oder mehrfach mit Substituenten substituiert sein können ausgewählt aus Halogen, insbesondere mit F oder Cl, $-NO_2$, -CN, $CF_3$, Alkyl, Cycloalkyl, Hydroxy und Alkoxy; oder eine Gruppe der Formel $-SO_2NR''R'''$ bedeuten, wobei R'' und R''' unabhängig voneinander Wasserstoff oder eine Alkylgruppe, gegebenenfalls substituiert, insbesondere mit F oder Cl, darstellen; oder $R^{31}$ und $R^{32}$ wie oben definiert sind, und $R^{29}$ und $R^{30}$ zusammen einen zusätzlichen 4- bis 7-gliedrigen kondensierten Ring bilden, wobei der kondensierte Ring aromatisch, partiell gesättigt oder gesättigt sein kann, und der kondensierte Ring mit Substituenten ausgewählt aus Halogen, insbesondere mit F oder Cl, $-NO_2$, -CN, $CF_3$, und einer Gruppe der Formel $-SO_2NR''R'''$ ein- oder mehrfach substituiert sein kann, wobei R'' und R''' unabhängig voneinander Wasserstoff oder eine Alkylgruppe, gegebenenfalls substituiert, insbesondere mit F oder Cl, darstellen.

$R^{27}$ der Formel (VI) kann Wasserstoff; eine $C_{1-6}$ Alkylgruppe; eine Phenylgruppe; eine Benzylgruppe; eine Gruppe der Formel $-CH_2CO_2R'$, wobei R' Wasserstoff oder eine $C_{1-6}$ Alkylgruppe bedeuten, gegebenenfalls substituiert, insbesondere mit F oder Cl, darstellt; eine Gruppe der Formel $-CH_2NH-Z$, wobei Z eine Phenyl oder Benzylgruppe bedeutet, wobei diese Phenyl- oder Benzylgruppen ein- oder mehrfach mit Halogen, insbesondere mit F oder Cl, substituiert sein kann; oder eine Gruppe der Formel $CH_2CO-Y''$, wobei Y'' eine heterozyklische 6-gliedrige monozyklische Gruppe enthaltend mindestens ein Stickstoff Atom als Heteroatom ist, und die ein- oder mehrfach mit einer $C_{1-6}$ Alkylgruppe oder einer Phenylgruppe substituiert sein kann. Y" ist vorzugsweise eine Piperidinyl- oder eine Piperazinylgruppe.

$R^{28}$ der Formel (VI) kann Wasserstoff, $C_{1-6}$ Alkylgruppe, Phenyl, Benzyl oder eine Gruppe der Formel -$CH_2COOH$ bedeuten.

$R^{29}$, $R^{30}$, $R^{31}$, und $R^{32}$ können unabhängig voneinander Wasserstoff, F, Br, Cl, $NO_2$, CN, $CF_3$ oder $C_{1-6}$ Alkyl sein, gegebenenfalls substituiert, insbesondere mit F oder Cl.

Modulatoren, die verwendet werden können sind: 5,7-Dinitro-1-methyl-1H-indol-2,3-dion-3-(O-methyloxim); 5-Brom-7-nitro-1H-indol-2,3-dion-3-oxim; 5-Bromisatin-3-oxim; 5,6-Dichlor-1-methylisatin-3-oxim; 4,5-Dichlorisatin-3-oxim; 4,5-Dichlor-1-methylisatin-3-oxim; O-Methyl-4,5-dichlor-1-methylisatin-3-oxim; Benzoisatin-3-oxim; 6,7-Dichlorisatin 3-oxim; O-Methyl 6,7-dichlorisatin-3-oxim; O-Methyl 6,7-dichlor-1-methylisatin-3-oxim; 6,7-Dichlor-1-methylisatin-3-oxim; O-t-Butyl-6,7-dichlorisatin-3-oxim; O-((4-Phenylpiperazin-1-yl)-carbonylmethyl)-6,7-dichlor-isatin-3-oxim; 6-Fluor-7-methoxyisatin-3-oxim; O-(4-Chlorbenzylamino)methyl-6,7-dichlorisatin-3-oxim; 6,7-Difluorisatin-3-oxim; 6,7-Dimethylisatin-3-oxim; 5,6-Dichlorisatin-3-oxim; O-Carboxymethyl-5-Bromisatin-3-oxim; O-(Ethoxycarbonylmethyl)-5-bromisatin-3-oxim; O-(Carboxymethyl)-6,7-dichlorisatin-3-oxim; O-(Carboxymethyl)-6,7-dichlorisatin 3-oxim; 6-Chlor-7-methylisatin-3-oxim; 6-Fluor-7-methylisatin-3-oxim.

Ein weiterer Modulator, der verwendet werden kann, ist Zoxazolamin, der als Aktivator von hIK1 wirkt (Syme et al., 2000, Am. J. Physiol. 278: C570-C581).

Halogen bedeutet ein Fluor-, Chlor, Brom- oder Iod-Atom. Besonders vorteilhalb Halogene sind F oder Cl.

Eine Alkylgruppe bedeutet vorliegend eine einwertige, gesättigte, unverzweigte oder verzweigte Kohlenwasserstoffkette. Die Kohlenwasserstoffkette enthält vorzugsweise 1 bis 12 Kohlenstoffatome ($C_{1-12}$-Alkyl), insbesondere 1 bis 6 Kohlenstoffatome ($C_{1-6}$-Alkyl; Niederalkyl), enthaltend Pentyl, Isopentyl, Neopentyl, tertiäres Pentyl, Hexyl und Isohexyl. Ferner kann Alkyl eine $C_{1-4}$-Alkylgruppe darstellen, enthaltend Butyl, Isobutyl, sekundäres Butyl und tertiäres Butyl. Ferner kann Alkyl eine $C_{1-3}$-Alkylgruppe bedeuten, die insbesondere Methyl, Ethyl, Propyl oder Isopropyl sein kann.

Eine Cycloalkylgruppe bedeutet eine zyklische Alkylgruppe, die bevorzugt drei bis sieben Kohlenstoffatome enthält ($C_{3-7}$-Cycloalkyl), einschließlich Cyclopropyl, Cyclobutyl, Cyclopentyl und Cyclohexyl.

Eine Alkoxygruppe bedeutet eine "Alkyl-O-" Gruppe, wobei Alkyl wie oben definiert ist.

Eine Aminogruppe kann eine primäre (-$NH_2$), sekundäre (-NH-R) oder tertiäre Aminogruppe (-N-R'R") sein, wobei R' und R" unabhängig voneinander Alkyl, wie oben definiert, bedeutet.

Eine Acylgruppe bedeutet eine Carboxygruppe oder eine Alkylcarbonylgruppe, wobei Alkyl wie oben definiert ist. Beispiele bevorzugter Acylgruppen sind Carboxy, Acetyl und Propionyl.

Eine Alkenylgruppe bedeutet eine Kohlenstoffkette enthaltend eine oder mehrere Doppelbindungen, einschließlich Di-enen, Tri-enen und Poly-enen. Vorteilhafterweise enthält die Alkenylgruppe zwischen zwei und sechs Kohlenstoffatome ($C_{2-6}$-Alkenyl). Besonders vorteilhafte Alkenylgruppen sind Ethenyl, 1,2 oder 2,3-Propenyl; oder 1,2-, 2,3-, oder 3,4-Butenyl.

Eine Alkinylgruppe benennt eine Kohlenstoffkette enthaltend eine oder mehrere Dreifachbindungen, einschließlich Di-inen, Th-inen und Poly-inen. Vorteilhafterweise enthält die Alkinylgruppe zwischen zwei und sechs Kohlenstoffatome ($C_{2-6}$-alkinyl). Besonders vorteilhafte Alkinylgruppen sind Ethinyl, 1,2 oder 2,3-Propinyl; oder 1,2-, 2,3-, oder 3,4-Butinyl.

Eine Amidogruppe bedeutet einen Substituenten der Formel R'-CO-NH- oder R'-CO-N(alkyl)-, wobei R' Wasserstoff oder eine Alkylgruppe wie oben definiert ist. Beispiele für Amidogruppen sind Formamido, Acetamido und Propionamido.

Eine mono- oder polyzyklische Arylgruppe bedeutet eine monozyklische oder polyzyklische aromatische Kohlenwasserstoffgruppe. Beispiele für Arylgruppen sind Phenyl, Naphtyl und Anthracenyl.

Eine ungesättigte mono- oder polyzyklische Gruppe bedeutet eine mono- oder polyzyklische Arylgruppe, d. h. eine monozyklische oder polyzyklische aromatische Kohlenwasserstoffkette. Ein Beispiel einer teilweise gesättigten monozyklischen Gruppe ist Cyclopenta-2,4-dien-1-yliden.

Eine Aralkylgruppe bedeutet eine Arylgruppe, wie oben definiert, wobei die Arylgruppe mit einer Alkylgruppe, wie oben definiert verbunden ist. Ein Beispiel ist Benzyl.

Eine mono- oder heterozyklische Gruppe bedeutet eine mono- oder polyzyklische Verbindung, die ein oder mehrere Heteroatome in der Ringstruktur enthält. Vorteilhafte Heteroatome enthalten Stickstoff (N), Sauerstoff (O) und Schwefel (S). Eine oder mehrere Ringstrukturen können aromatisch (d.h. Heteroaryl), gesättigt oder partiell gesättigt sein. Vorteilhafte monozyklische Gruppen enthalten 5- und 6-gliedrige heterozyklische Gruppen. Beispiele monozyklischer heterozyklischer Gruppen enthalten Furan-2-yl; Furan-3-yl; 2-, 4- oder 5-Imidazolyl, 3-, 4-, oder 5-Isoxazolyl, 1-, 2-, 3-Pyridinyl und 1- oder 2-Thienyl. Beispiele gesättigter oder partiell gesättigter monozyklischer heterozyklischer Gruppen enthalten 1,3,5,6,2-Dioxadiazinyl, Piperazinyl, Piperidinyl, 1,2-, 1,3- oder 1,4- Pyranyl und Pyrrolidinyl. Beispiele aromatischer heterozyklischer Gruppen enthalten Acridinyl, Carbazolyl, Indazolyl, Chinolinyl, Benzofuranyl.

Eine Heteroalkylgruppe benennt eine mono- oder poly-heterozyklische Gruppe wie oben definiert, wobei die heterozyklische Gruppe mit einer Alkylgruppe wie oben definiert, verbunden ist.

Besonders vorteilhafte verwendbare, Modulatoren, sind hIK4- Aktivatoren 1-EBIO (1-Ethyl-2-Benzimidazolinon), DCEBIO (5,6-Dichloro-1-ethyl-1,3-dihydro-2H-benzimidazol-2-on) sowie die Benzoxazolone Chlorzoxazon und Zoxazolamin, die als Aktivatoren von hIK1 wirken (Syme et al., 2000, Am. J. Physiol. 278: C570-C581). Weiterhin sind Isatin-Derivate aus WO 00/33834 als Aktivatoren von hIK1 bekannt. Beispiele sind 5-Bromoisatin 3-Oxim und 5,6-Dichloro-1-Methylisatin 3-Oxim.

Besonders vorteilhafte Inhibitoren von hIK4 sind Charybdotoxin und Clotrimazol (Khanna et al., 1999, J. Biol. Chem. 274: 14838-14849). WO 99/25347 offenbart weitere Substanzen die als Inhibitoren von hIK1 wirken. Beispiele sind die Imidazol-Derivate Miconazol, Econazol, Butoconazol, Oxiconazol, Sulconazol, Thioconazol, die Triazol-Derivate Fluconazol, Terconazol, Itraconazol, die Nitroimidazol Derivate Metronidazol, Tinidazol, Nimorazol, Ornidazol, Benznidazol. Außerdem offenbart das Dokument Derivate der 1,4-Dihydropyridin-3,5-dicarbonsäure, beispielsweise 1,4-dihydro-2,6-dimethyl-4-(2-nitrophenyl)pyridin-3,5-dicarbonsäureethylmethylester, sowie Clotrimazol-Derivate, beispielsweise 2-Chlorphenyl-4-hydroxyphenyl-phenyl-methan.

Die verwendeten Modulatoren können zur Diagnose und/oder Prävention und/oder Behandlung von Erkrankungen, die mit gestörter Keratinozytenaktivität in Zusammenhang stehen, insbesondere von Wundheilungsstörungen; besonders bevorzugt Psoriasis, verwendet werden. Erkrankungen, die mit gestörter Keratinozytenaktivität in Zusammenhang stehen, sind Kontaktdermatitis, atopisches Ekzem, Vitiligo, Hyperkeratosen, aktinische Keratosen, hypertrophe Narben, Keloide, Lentigo, Epheliden und Altershaut. Weitere Erkrankungen sind Wunden, beispielsweise normal heilende Wunden und schlecht heilende Wunden, insbesondere Ulzera, sowie Psoriasis.

Die vorliegende Erfindung beschreibt auch ein Verfahren zur Herstellung eines Arzneimittels zur Behandlung von Erkrankungen, die mit gestörter Keratinozytenaktivität in Zusammenhang stehen, insbesondere von Wundheilungsstörungen, besonders bevorzugt von Psoriasis, bei dem mindestens eine erfindungsgemäß verwendbare Nukleinsäure, mindestens ein erfindungsgemäß verwendbares Polypeptid, mindestens ein erfindungsgemäß verwendbarer Antikörper und/oder mindestens ein erfindungsgemäß verwendbarer Modulator zusammen mit geeigneten Zusatz- und Hilfsstoffen kombiniert wird.

Im Falle von Psoriasis ist die Verwendung eines Modulators der Aktivität des Ionenkanals, beispielsweise eines Aktivators der Aktivität des Ionenkanals oder eines Inhibitors der Aktivität des Ionenkanals vorteilhaft. Beispiele für Modulatoren sind DCEBIO, Zoxazolamin, Chlorzoxazon, 1-EBIO sowie ein spezifischer Antikörper. Weiterhin ist im Falle von Psoriasis eine Nukleinsäure vorteilhaft, das zu einer Runterregulation der Menge an IK-Kanal führt, beispielsweise ein antisense-Oligonukleotid, eine siRNA oder ein Ribozym.

Im Falle der Wundheilung, insbesondere der Ulzera, ist die Erhöhung der Menge an IK Kanal beispielsweise durch gentherapeutische Behandlung mit einer Nukleinsäure kodierend für ein verwendbares Polypeptid vorteilhaft.

Die vorliegende Erfindung beschreibt weiterhin ein nach diesem Verfahren hergestelltes Arzneimittel zur Behandlung von Erkrankungen, die mit gestörter Keratinozytenaktivität in Zusammenhang stehen, insbesondere von Wundheilungsstörungen und/oder Psoriasis, besonders vorteilhaft Psoriasis, das mindestens ein Polypeptid, gemäß SEQ ID Nr. 3 oder SEQ ID Nr. 4, oder ein eine diese kodierende Nukleinsäure oder oder mindestens ein erfindungsgemäß verwendbaren Antikörper oder mindestens einen verwendbaren Modulator, gegebenenfalls zusammen mit geeigneten Zusatz- und Hilfsstoffen, enthält.

Die Erfindung beschreibt weiterhin die Verwendung dieses Arzneimittels zur Behandlung von Erkrankungen, die mit gestörter Keratinozytenaktivität in Zusammenhang stehen.

Die Therapie der Erkrankungen, die mit gestörter Keratinozytenaktivität in Zusammenhang stehen, kann auf herkömmliche Weise, z.B. durch Verbände, Pflaster, Kompressen oder Gele erfolgen, die die erfindungsgemäßen Arzneimittel enthalten. So ist es möglich, die geeignete Zusatz- oder Hilfsstoffe, wie z. B. physiologische Kochsalzlösung, entmineralisiertes Wasser, Stabilisatoren, Proteinaseinhibitoren, Gelformulierungen, wie z.B. weiße Vaseline, dünnflüssiges Paraffin und/oder gelbes Wachs, etc., enthaltenden Arzneimittel topisch und lokal zu verabreichen, um die Erkrankung sofort und unmittelbar zu beeinflussen. Die Verabreichung der erfindungsgemäßen Arzneimittel kann weiterhin, gegebenenfalls in Form von Liposomenkomplexen bzw. Goldpartikelkomplexen, ebenfalls topisch und lokal im Bereich der erkrankten Hautfläche erfolgen. Weiterhin kann die Behandlung mittels eines transdermalen therapeutischen Systems (TTS) erfolgen, das eine zeitlich gesteuerte Abgabe der erfindungsgemäßen Arzneimittel ermöglicht. TTS sind zum Beispiel aus den EP 0 944 398 A1, EP 0 916 336 A1, EP 0 889 723 A1 oder EP 0 852 493 A1 bekannt. Die Behandlung mittels der erfindungsgemäßen Arzneimittel kann aber auch über orale Dosierungsformen, wie z.B. Tabletten oder Kapseln, über die Schleimhäute, zum Beispiel der Nase oder der Mundhöhle, oder in Form von unter die Haut implantierten Dispositorien erfolgen.

Eine möglich Form eines Arzneimittels dient zur Prävention und/oder Behandlung von Psoriasis, wobei die Keratinozytenaktivität verringert werden soll. Dabei ist die Inhibition der (elektrophysiologischen) Aktivität des erfindungsgemäßen hIK1-Kanals durch geeignete Modulatoren und/oder Antikörper besonders vorteilhaft.

Ein besonders bevorzugte Ausführungsform besteht in der Verabreichung eines Medikaments enthaltend

einen erfindungsgemäß verwendbaren Modulator kann in Form der aktiven Form des Modulators erfolgen. Bevorzugt ist eine Zusammensetzung enthaltend die aktive Form des Modulators, optional in Form eines pharmazeutisch annehmbaren Salzes, zusammen mit einem oder mehreren geeigneten Zusatz- und Hilfsstoffen, wie beispielsweise Adjuvantien, Trägermaterialien und Pufferlösungen und Verdünnungsmitteln, wie z. B. physiologische Kochsalzlösung, entmineralisiertes Wasser, Stabilisatoren, Proteinaseinhibitoren, Gelformulierungen, wie z.B. weiße Vaseline, dünnflüssiges Paraffin und/oder gelbes Wachs, etc., zu verabreichen, die insbesondere für die topische Applikation geeignet sind um die Wundheilung sofort und unmittelbar zu beeinflussen.

Vorteilhaft ist die Verwendung eines gegen das hIK1-Kanal Polypeptid oder eine funktionelle Variante davon gerichteten Antikörpers oder eines Antikörperfragments als Arzneimittel gemäß vorliegender Erfindung.

Besonders vorteilhaft ist die Verringerung der Expression des erfingungsgemäßen hIK1 Polypeptid kodierende Nukleinsäuren.

Besonders vorteilhaft sind anti-sense Nukleotide (siehe oben) und Ribozyme (WO 99/15703) sowie sogenannte "small interfering RNAs" (siRNAs). Dies sind doppelsträngige RNA Moleküle, die sequenzspezifisch und posttranskriptionell die Genexpression herunterregulieren (Elbashir et al., 2001, Nature 411: 494-498).

Vorteilhaft ist die Prävention und/oder Behandlung von Wundheilungsstörungen durch die besiepielsweise die Gabe eines Polypeptides oder das Polypetid kodierende Nukleinsäure(n).

Für die gentherapeutische Anwendung beim Menschen ist vor allem ein Arzneimittel geeignet, das die beschriebenen Nukleinsäure in nackter Form oder in Form eines der oben beschriebenen gentherapeutisch wirksamen Vektoren oder in mit Liposomen bzw. Goldpartikeln komplexierter Form enthält. Der pharmazeutische Träger ist beispielsweise eine physiologische Pufferlösung, vorzugsweise mit einem pH von ca. 6,0-8,0, vorzugsweise von ca. 6,8-7,8. Insbesondere von ca. 7,4 und/oder einer Osmolarität von ca. 200-400 milliosmol/Liter, vorzugsweise von ca. 290-310 milliosmol/Liter. Zusätzlich kann der pharmazeutische Träger geeignete Stabilisatoren, wie z.B. Nukleaseinhibitoren, vorzugsweise Komplexbildner wie EDTA und/oder andere dem Fachmann bekannte Hilfsstoffe enthalten.

Die Verabreichung der beschriebenen Nukleinsäure gegebenenfalls in Form der oben näher beschriebenen Virusvektoren oder als Liposomenkomplexe bzw. Goldpartikelkomplex erfolgt üblicherweise topisch und lokal im. Bereich der Wunde.

Vorteilhaft ist das Polypeptid selbst mit geeigneten Zusatz- oder Hilfsstoffen, wie z. B. physiologische Kochsalzlösung, entmineralisiertes Wasser, Stabilisatoren, Proteinaseinhibitoren, Gelformulierungen, wie z.B. weiße Vaseline, dünnflüssiges Paraffin und/oder gelbes Wachs, etc., zu verabreichen, um die Wundheilung sofort und unmittelbar zu beeinflussen.

Vorteilhaft ist ferner die Transplantation autologer oder allogener Zellen, die das erfindungsgemäße Polypetid exprimiert mit Hilfe eines geeigneten Trägermaterials, z.B. sogenannte Microcarrier, die aus biokompatiblen Materialien, wie beispielsweise einer Dextranmatrix, bestehen (siehe US598888).

Die vorliegende Erfindung beschreibt weiterhin ein Verfahren zur Herstellung eines Diagnostikums zur Diagnose von Erkrankungen, die mit gestörter Keratinozytenaktivität in Zusammenhang stehen, insbesondere von Wundheilungsstörungen und/oder Psoriasis, das dadurch gekennzeichnet ist, daß mindestens eine Nukleinsäure, gemäß SEQ ID Nr. 1 und 2, mindestens ein Polypeptid, gemäß SEQ Id Nr. 3 und 4, oder mindestens ein Modulator, gegebenenfalls zusammen mit geeigneten Zusatz- und Hilfsstoffen, verwendet wird.

Die vorliegende Erfindung beschreibt ein Diagnostikum zur Diagnose von Erkrankungen, die mit gestörter Keratinozytenaktivität in Zusammenhang stehen, insbesondere von Wundheilungsstörungen und/oder Psoriasis, das mindestens eine Nukleinsäure, gemäß SEQ ID Nr. 1 und 2, mindestens ein Polypeptid, gemäß SEQ ID Nr. 3 und 4, oder mindestens einen Modulator gegebenenfalls zusammen mit geeigneten Zusatz- und Hilfsstoffen, enthält.

Beispielsweise kann anhand einer der vorangehend beschriebenen Nukleinsäuren ein Diagnostikum auf der Basis der Polymerasekettenreaktion (Beispiele 3, 4, 5; PCR-Diagnostik, z. B. gemäß EP 0 200 362) oder eines "RNase-Protection-Assays", wie in Beispiel 2 näher dargestellt, hergestellt werden. Diese Tests beruhen auf der spezifischen Hybridisierung der vorangehend beschriebenen Nukleinsäuren mit dem komplementären Gegenstrang, üblicherweise der entsprechenden mRNA. Die vorangehend beschriebene Nukleinsäure kann hierbei auch modifiziert sein, wie z. B. in EP 0 063 879 beschrieben. Beispielsweise wird ein vorangehend beschriebenes DNA-Fragment mittels geeigneter Reagenzien, z. B. radioaktiv mit $\alpha$-$P^{32}$-dCTP oder nicht-radioaktiv mit Biotin oder Digoxigenin, nach allgemein bekannten Methoden markiert und mit isolierter RNA, die vorzugsweise vorher an geeignete Membranen aus z. B. Cellulose oder Nylon gebunden wurde, inkubiert. Bei gleicher Menge an untersuchter RNA aus jeder Gewebeprobe kann somit die Menge an mRNA bestimmt werden, die spezifisch durch die Sonde markiert wurde und mit der Menge an mRNA aus gesundem Gewebe verglichen werden. Alternativ kann die Bestimmung an mRNA auch in Gewebeschnitten mit Hilfe der *in situ* Hybridisierung (siehe z.B. Werner et al., 1992, Proc. Natl. Acad. Sci. U S A 89:6896-900) erfolgen.

Mit Hilfe des Diagnostikums kann somit auch eine Gewebeprobe *in vitro* auf die Expressionsstärke des korrespondierenden Gens spezifisch gemessen werden, um eine mögliche Erkrankung, die mit gestörter Keratinozytenaktivität in Zusammenhang steht, sicher diagnostizieren zu können (Beispiele 2, 3, 4, 5). Insbesondere eignet sich ein solches Verfahren zur frühzeitigen Prognose von Störungen. Dies ermöglicht einen präventiven Therapieeinsatz und

die Analyse von Prädispositionen. So ist die Expression des Gens *mIK1* schon im nicht verwundeten Zustand in der intakten Haut alter Mäuse, die nach Verwundung Wundheilungsstörungen aufwies, im Vergleich zu intakter Haut von Kontrollmäusen und insbesondere zu intakter Haut von jungen Mäusen erhöht. Die Expressionsstärke des Gens *mIK1* ermöglicht daher eine Voraussage der Wundheilungsstörung noch im intakten Gewebe (Beispiel 3, Tabelle 2).

Ein weiteres Diagnostikum enthält das beschriebene Polypeptid bzw. die oben näher beschriebenen immunogenen Teile davon. Das Polypeptid bzw. die Teile davon, die vorzugsweise an eine Festphase, z. B. aus Nitrocellulose oder Nylon, gebunden sind, können beispielsweise mit der zu untersuchenden Körperflüssigkeit, z. B. Wundsekret, in vitro in Berührung gebracht werden, um so beispielsweise mit Autoimmunantikörper reagieren zu können. Der Antikörper-Peptid-Komplex kann anschließend beispielsweise anhand markierter Antihuman-IgG- oder Antihujnan-IgM-Antikörper nachgewiesen werden. Bei der Markierung handelt es sich beispielsweise um ein Enzym, wie Peroxidase, das eine Farbreaktion katalysiert. Die Anwesenheit und die Menge an anwesenden Autoimmunantikörper kann somit über die Farbreaktion leicht und schnell nachgewiesen werden.

Ein anderes Diagnostikum enthält die Antikörper selbst. Mit Hilfe dieser Antikörper kann beispielsweise eine Gewebeprobe leicht und schnell dahingehend untersucht werden, ob das betreffende Polypeptid in einer erhöhten Menge vorhanden ist, um dadurch einen Hinweis auf eine mögliche Erkrankung, die mit gestörter Keratinozytenaktivität im Zusammenhang steht, zu erhalten. In diesem Fall sind die Antikörper beispielsweise mit einem Enzym, wie oben bereits beschrieben, markiert. Der spezifische Antikörper-Peptid-Komplex kann dadurch leicht und ebenso schnell über eine enzymatische Farbreaktion nachgewiesen werden.

Ein weiteres Diagnostikum enthält eine Sonde, vorzugsweise eine DNA-Sonde, und/oder Primer. Dies eröffnet eine weitere Möglichkeit, die verwendbaren Nukleinsäuren, zum Beispiel durch die Isolierung aus einer geeigneten Genbank, beispielsweise aus einer wundspezifischen Genbank, anhand einer geeigneten Sonde zu erhalten (siehe z. B. J. Sambrook et al., 1989, Molecular Cloning. A Laboratory Manual 2. Auflage., Cold Spring Harbor Laboratory, Cold Spring Harbor, NY Kapitel 8 Seite 8.1 bis 8.81, Kapitel 9 Seite 9.47 bis 9.58 und Kapitel 10 Seite 10.1 bis 10.67).

Als Sonde eignen sich beispielsweise DNA- oder RNA-Fragmente mit einer Länge von ca. 100-1000 Nukleotiden, vorzugsweise mit einer Länge von ca. 200-500 Nukleotiden, insbesondere mit einer Länge von ca. 100-400 Nukleotiden deren Sequenz aus den Polypeptidsequenzen oder funktionelle Varianten davon gemäß SEQ ID Nr. 3 oder SEQ ID Nr. 4 des Sequenzprotokolls und/oder anhand der cDNA Sequenzen oder Varianten davon gemäß SEQ ID Nr. 1 oder SEQ ID Nr. 2 angegebenen Datenbankeinträge abgeleitet werden kann (siehe auch Beispiel 2).

Alternativ können anhand der abgeleiteten Nukleinsäuresequenzen Oligonukleotide synthetisiert werden, die sich als Primer für eine Polymerase Kettenreaktion eignen. Mit diesen kann die verwendbare Nukleinsäure oder Teile dieser aus cDNA, beispielsweise hautspezifischer cDNA, amplifiziert und isoliert werden (Beispiele 2, 3, 4, 5). Als Primer eignen sich beispielsweise DNA-Fragmente mit einer Länge von ca. 10-100 Nukleotiden, vorzugsweise mit einer Länge von ca. 10 bis 50 Nukleotiden, insbesondere mit einer Länge von 10-30 Nukleotiden deren Sequenz aus den Polypeptiden gemäß den SEQ ID Nr. 3 bis SEQ ID Nr. 4 und/oder anhand der cDNA Sequenzen gemäß SEQ ID Nr. 1 oder SEQ ID Nr. 2 abgeleitet werden kann (Beispiele 3, 4, 5).

Die Erfindung beschreibt ferner ein Verfahren zur Herstellung eines Tests zur Auffindung pharmakologisch aktiver Substanzen, die im Zusammenhang mit Erkrankungen mit gestörter Keratinozytenaktivität stehen, insbesondere von Wundheilungsstörungen und/oder Psoriasis, dadurch gekennzeichnet, daß mindestens eine Nukleinsäure, gemäß SEQ ID Nr. 1 und 2, mindestens eines Polypeptids, gemäß SEQ ID Nr. 3 und 4, mindestens ein Antikörper oder mindestens ein Modulator gegebenenfalls zusammen mit geeigneten Zusatz- und Hilfsstoffen, zur Herstellung des Tests verwendet wird.

Unter dem Begriff "pharmakologisch aktive Substanzen" im Sinne der vorliegenden Erfindung sind alle diejenigen Moleküle, Verbindungen und/oder Zusammensetzungen und Stoffgemische zu verstehen, die mit den vorangehend beschriebenen Nukleinsäuren, Polypeptiden oder Antikörpern, gegebenenfalls zusammen mit geeigneten Zusatz- und Hilfsstoffen, unter geeigneten Bedingungen in Wechselwirkung treten können. Mögliche pharmakologisch aktive Substanzen sind einfache chemische organische oder anorganische Moleküle oder Verbindungen, können aber auch Peptide, Proteine oder Komplexe davon enthalten. Die pharmakologisch aktiven Substanzen können aufgrund ihrer Wechselwirkung die Funktion(en) der Nukleinsäuren, Polypeptide oder Antikörper *in vivo* oder *in vitro* beeinflussen oder auch nur an die vorangehend beschriebenen Nukleinsäuren, Polypeptide oder Antikörper binden oder mit ihnen andere Wechselwirkungen kovalenter oder nicht-kovalenter Weise eingehen. Insbesondere sind pharmakologisch aktive Sustanzen Substanzen, die die Kanalaktivität wie oben beschrieben modulieren können.

Die Erfindung beschreibt weiterhin einen Test zur Identifizierung pharmakologisch aktiver Substanzen in Zusammenhang mit Erkrankungen, die mit gestörter Keratinozytenaktivität in Zusammenhang stehen, der mindestens eine Nukleinsäure, mindestens ein Polypeptid oder mindestens einen Modulator, gegebenenfalls zusammen mit geeigneten Zusatz- und Hilfsstoffen, einschließt.

Ein geeignetes System läßt sich beispielsweise durch die stabile Transformation von epidermalen bzw. dermalen Zellen, insbesondere von Keratinozyten mit Expressionsvektoren, die selektierbare Markergene und die vorangehend beschriebenen Nukleinsäuren enthalten, herstellen. Bei diesem Verfahren wird die Expression der vorangehend beschriebenen Nukleinsäuren in den Zellen so verändert, daß sie der pathologisch gestörten Expression *in vivo* entspricht. Auch anti-sense Oligonukleotide, die die verwendbaren Nukleinsäuresequenzen enthalten, können zu diesem Zweck eingesetzt werden. Von besonderem Vorteil für diese Systeme ist es daher, das Expressionsverhalten der Gene bei gestörten physiologischen Prozessen, wie in dieser Anmeldung beschrieben, zu kennen. Oft kann so das pathologische Verhalten der Zellen *in vitro* nachgeahmt werden und es können Substanzen gesucht werden, die das normale Verhalten der Zellen wieder herstellen und die ein therapeutisches Potential besitzen.

Für diese Testsysteme eignen sich z.B. HaCaT-Zellen, die allgemein erhältlich sind, und der Expressionsvektor pCMV4 (Anderson et al., 1989, J. Biol. Chem. 264:8222-9). Die verwendbare Nukleinsäure kann dabei sowohl in sense als auch in anti-sense Orientierung in die Expressionsvektoren integriert werden, so daß die funktionelle Konzentration an mRNA der entsprechenden Gene in den Zellen entweder erhöht, oder durch Hybridisierung mit der antisense-RNA erniedrigt wird. Nach der Transformation und Selektion stabiler Transformanden zeigen die Zellen in Kultur im allgemeinen ein verändertes Proliferations-, Migrations, und/oder Differenzierungsverhalten im Vergleich zu Kontrollzellen. Dieses Verhalten *in vitro* ist häufig mit der Funktion der entsprechenden Gene bei regenerativen Prozessen im Organismus korreliert (Yu et al., 1997, Arch. Dermatol. Res. 289:352-9; Mils er al., 1997, Oncogene 14: 15555-61; Charvat et al., 1998, Exp Dermatol 7: 184-90; Mythily et al., 1999, J. Gen. Virol. 80:1707-13; Werner, 1998, Cytokine Growth Factor Rev. 9:153-65) und läßt sich mit einfachen und schnell durchzuführenden Tests nachweisen, so daß man darauf basierend Testsysteme zur Identifizierung pharmakologisch aktiver Substanzen aufbauen kann. So läßt sich das Proliferationsverhalten von Zellen sehr schnell durch z.B. den Einbau von markierten Nukleotiden in die DNA der Zellen (siehe z.B. de Fries und Mitsuhashi, 1995, J. Clin. Lab. Anal. 9:89-95; Perros und Weightman, 1991, Cell Prolif. 24:517-23; Savino und Dardenne, 1985, J. Immunol. Methods 85:221-6), durch Anfärbung der Zellen mit spezifischen Farbstoffen (Schulz et al., 1994, J. Immunol. Methods 167:1-13) oder über immunologische Verfahren (Frahm et al., 1998, J. Immunol. Methods 211:43-50) nachweisen. Die Migration läßt sich einfach durch den "Migration Index" Test (Charvat et al., supra) und vergleichbare Testsysteme (Benestad et al., 1987, Cell Tissue Kinet. 20:109-19, Junger et al., 1993, J. Immunol. Methods 160:73-9) nachweisen. Als Differenzierungsmarker eignen sich z.B. Keratin 6, 10 und 14 sowie Loricrin und Involucrin (Rosenthal et al., 1992, J, Invest, Dermatol, 98:343-50), deren Expression z.B. über allgemein erhältliche Antikörper leicht nachzuweisen ist.

Ein anderes geeignetes Testsystem basiert auf der Identifikation von Interaktionen mit dem sogenannten "Two-Hybrid System" (Fields und Stemglanz, 1994, Trends in Genetics, 10, 286-292; Colas und Brent, 1998 TIBTECH, 16, 355-363). Bei diesem Test werden Zellen mit Expressionsvektoren transformiert, die Fusionsproteine aus dem verwendbaren Polypeptid und einer DNA-Bindungsdomäne eines Transkriptionsfaktors wie beispielsweise Gal4 oder LexA exprimieren. Die transformierten Zellen enthalten außerdem ein Reportergen, dessen Promotor Bindungsstellen für die entsprechende DNA Bindungsdomäne enthalten. Durch Transformation eines weiteren Expressionsvektors, der ein zweites Fusionsprotein aus einem bekannten bzw. unbekannten Polypeptid mit einer Aktivierungsdomäne, beispielsweise von Gal4 oder Herpes Virus VP16, exprimiert, kann die Expression des Reportergens stark gesteigert werden, wenn das zweite Fusionsprotein mit dem erfindungsgemäß verwendbaren Polypeptid interagiert. Diese Expressionssteigerung kann man ausnutzen, um neue pharmakologisch aktive Substanzen zu identifizieren, beispielsweise indem man zur Konstruktion des zweiten Fusionsproteins eine cDNA-Bibliothek aus sich regenerierenden Gewebe herstellt. Zudem läßt sich dieses Testsystem zum Screening von Substanzen ausnutzen, die eine Interaktion zwischen dem verwendbaren Polypeptid und einer bereits bekannten pharmakologisch aktive Substanzen inhibieren. Solche Substanzen verringern die Expression des Reportergens in Zellen, die Fusionsproteine des erfindungsgemäß verwendbaren Polypeptids und der pharmakologisch aktiven Substanzen exprimieren (Vidal und Endoh, 1999, Trends in Biotechnology, 17:374-81). So lassen sich schnell neue Wirkstoffe identifizieren, die zur Therapie von Erkrankungen, die mit gestörter Keratinozytenaktivität in Zusammenhang stehen, eingesetzt werden können.

Pharmakologisch aktive Substanzen der verwendbaren Polypeptide können auch Nukleinsäuren sein, die über Selektionsverfahren, wie beispielsweise SELEX (siehe Jayasena, 1999, Clin. Chem. 45:1628-50; Klug und Famulok, 1994, M. Mol. Biol. Rep. 20:97-107; Toole et al., 1996, US 5582981) isoliert werden. Im SELEX-Verfahren werden typischerweise aus einem großen Pool unterschiedlicher, einzelsträngigе RNA Moleküle durch wiederholte Amplifikation und Selektion diejenigen Moleküle isoliert, die an ein Polypeptide mit hoher Affinität binden (Aptamere). Aptamere können auch in ihrer spiegelbildlichen Form, beispielsweise als L-Ribonukleotid, synthetisiert und selektioniert werden (Nolte et al., 1996, Nat. Biotechnol. 14:1116-9; Klussmann et al., 1996, Nat. Biotechnol. 14:1112-5). So isolierte Formen haben den Vorteil, das sie nicht von natürlich vorkommenden Ribonukleasen abgebaut werden und daher größere Stabilität besitzen.

Die Erfindung beschreibt ferner ein Verfahren zur Herstellung eines auf einem Trägermaterial fixierten Arrays zur Analyse in Zusammenhang mit Erkrankungen, die mit gestörter Keratinozytenaktivität in Zusammenhang stehen, bei dem mindestens eine Nukleinsäure, mindestens ein Polypeptid oder mindestens ein Antikörper zur Herstellung verwendet wird.

Verfahren zur Herstellung von solchen Arrays sind zum Beispiel aus der WO 89/109077, WO 90/15070, WO 95/35505 und US 5,744,305 mittels Spottings, Druckens oder Festphasenchemie in Verbindung mit photolabilen

Schutzgruppen bekannt.

Die Erfindung beschreibt ferner ein auf einem Trägermaterial fixiertes Array zur Analyse in Zusammenhang mit Erkrankungen, die mit gestörter Keratinozytenaktivität in Zusammenhang stehen, insbesondere von Wundheilungsstörungen und/oder Psoriasis, das dadurch gekennzeichnet ist, daß es mindestens eine Nukleinsäure und/oder mindestens ein Polypeptid und/oder oder mindestens einen Antikörper enthält. Arrays können zur Analyse in Zusammenhang mit Erkrankungen, die mit gestörter Keratinozytenaktivität in Zusammenhang stehen, insbesondere von Wundheilungsstörungen und/oder Psoriasis verwendet werden.

Die Erfindung beschreibt ferner ein Verfahren zur Herstellung eines DNA-Chips und/oder Protein-Chips zur Analyse in Zusammenhang mit Erkrankungen, die mit gestörter Keratinozytenaktivität in Zusammenhang stehen, insbesondere von Wundheilungsstörungen und/oder Psoriasis, das dadurch gekennzeichnet ist, daß mindestens eine Nukleinsäure, gemäß SEQ ID Nr. 1 und 2, mindestens ein Polypeptid, gemäß SEQ ID Nr. 3 und 4, oder mindestens ein Antikörper, wie vorangehend beschrieben, zur Herstellung verwendet wird.

Verfahren zur Herstellung solcher DNA-Chips und/oder Protein-Chips sind zum Beispiel aus der WO 89/109077, WO 90/15070, WO 95/35505 und US 5,744,305 mittels Spottings, Druckens oder Festphasenchemie in Verbindung mit photolabilen Schutzgruppen bekannt.

Die Erfindung beschreibt ferner einen DNA-Chip und/oder Protein-Chip zur Analyse in Zusammenhang mit Erkrankungen, die mit gestörter Keratinozytenaktivität in Zusammenhang stehen, insbesondere von Wundheilungsstörungen und/oder Psoriasis, der mindestens eine Nukleinsäure, gemäß SEQ ID Nr. 1 und 2, und/oder mindestens ein Polypeptid, gemäß SEQ ID Nr. 3und 4, und/oder oder mindestens einen Antikörper enthält. DNA-Chips sind zum Beispiel aus der US 5,837,832 bekannt.

Die Erfindung beschreibt ferner ein Arzneimittel zur Indikation und Therapie, das eine verwendbare Nukleinsäure, ein verwendbares Polypeptid oder einen verwendbaren Modulator und gegebenenfalls geeignete Zusatz- oder Hilfsstoffe enthält und ein Verfahren zur Herstellung eines solchen Arzneimittels zur Behandlung von Erkrankungen, die mit gestörter Keratinozytenaktivität in Zusammenhang stehen, insbesondere von Wundheilungsstörungen und/oder Psoriasis, bei dem eine verwendbare Nukleinsäure, gemäß SEQ ID Nr. 1 und 2, ein verwendbares Polypeptid, gemäß SEQ ID Nr. 3 und 4, oder einen verwendbarer Modulator mit einem pharmazeutisch annehmbaren Träger formuliert wird.

Der Wortlaut sämtlicher Ansprüche wird hiermit durch Bezugnahme zum Inhalt der Beschreibung.

Die folgenden Tabellen und Figuren und die folgenden Beispiele sollen die Erfindung lediglich näher beschreiben ohne sie zu beschränken.

**Tabellen und Figuren**

**Tabelle 1** zeigt die Auflistung der erfindungsgemäß verwendbaren hIK1 Polypeptide und Nukleinsäuren, sowie deren Datenbankzugangsnummern (accession numbers).

**Tabelle 2** zeigt die mittels "TaqMan"-Analyse bestimmte differentielle Regulation der mIK1-Expression bei verschiedenen Wundheilungszuständen der Maus als Beispiel für Erkrankungen, die mit gestörter Keratinozytenaktivität in Zusammenhang stehen.

**Tabelle 3** zeigt die mittels "TaqMan"-Analyse bestimmte Kinetik der differentiellen Regulation der mIK1-Expression bei der Wundheilung als Beispiel für eine Erkrankung, die mit gestörter Keratinozytenaktivität in Zusammenhang steht.

**Tabelle 4** zeigt die mittels "TaqMan"-Analyse bestimmte differentielle Regulation der mIK1-Expression in zwei verschiedenen Wundheilungszuständen des Menschen (normale Wundheilung und Ulkus) als Beispiel für Erkrankungen, die mit gestörter Keratinozytenaktivität in Zusammenhang stehen.

**Tabelle 5** zeigt das mittels "TaqMan"-Analyse bestimmte Expressionsprofil von hIK1 in verschiedenen Organen von Menschen.

**Figur 1** zeigt die mittels "Patch-Clamp"-Analyse bestimmte Änderung des Membranpotentials (Vm) in mV als elektrophysiologische Antwort von HaCaT-Keratinozyten auf (A) verschiedene Konzentrationen von ATP , (B) 10 μM Bradykinin oder 100 μM Histamin (C) "caged $IP_3$" vor und nach UV-induzierter Blitzphotolyse.

**Figur 2** zeigt den mittels "Patch-Clamp"-Analyse bestimmten Einfluß verschiedener Modulatoren von hIK1 auf

die ATP-induzierte, elektrophysiologische Antwort als Änderung des Membranpotentials (Vm) in mV. (A) Zugabe von Charybdotoxin in verschiedenen Konzentrationen vor der Zugabe von ATP (B) Zugabe von Clotrimazol (1 μM) vor der Zugabe von ATP (C) Zugabe von Charybdotoxin (100 nM) nach der Zugabe von ATP (D) Zugabe von 1-EBIO (1 mM) vor der Zugabe von ATP.

**Figur 3** zeigt die Immunfärbung von gerade konfluenten (konfluent), 2 Tage postkonfluenten (2d postkonfluent), 4 Tage postkonfluenten (4d postkonfluent) und 6 Tage postkonfluenten (6 Tage postkonfluent) HaCaT-Zellen mit einem Anti-K10-Antikörper.

**Figur 4** zeigt mittels "RNAse Protection Assay" bestimmte Menge an hIK1 in proliferierenden (Spur "1"), gerade konfluenten (Spur "2") und 4 Tage postkonfluenten (Spur "3") HaCaT-Keratinozyten als Autoradiographie. In der Spur "probe" sind 1000 cpm der Hybridisierungssonde aufgetragen. Die Spur "tRNA" enthält die Negativkontrolle.

**Figur 5** zeigt den Einfluß verschiedener Modulatoren von hIK1 auf die Proliferation von HaCaT-Keratinozyten (n=3). Als Kontrolle wurden Zellen verwendet, bei denen nur das jeweilige Lösungsmittel zum Medium zugegeben wurde. Clotrimazol (10 μM) und 1-EBIO (1 mM) inhibierten die Proliferation vollständig, während die Zugabe von Charybdotoxin (200 nM) die Proliferation verlangsamte.

**Figur 6** zeigt mittels Immunoblot detektierte Mengen an Proteinmarkern für die Keratinozytendifferenzierung Involucrin, Keratin 1 (K1) und Keratin 10 (K10) unter dem Einfluss von 1000 μM 1-EBIO (Spuren 1 und 2), 100 μM 1-EBIO (Spuren 3 und 4), 1 μM 1-EBIO (Spuren 5 und 6), 1000 μM Zoxazolamin (Spuren 7 und 8), 100 μM Zoxazolamin (Spuren 9 und 10), 1 μM Zoxazolamin (Spuren 11 und 12) und in der Kontrolle (Spur "C"; 1%DMSO/DMEM) in HaCaT Keratinozyten.

**Figur 7** zeigt den Einfluss verschiedender Konzentrationen des Aktivators von hIK1, 1-EBIO, auf die Proliferation von HaCaT Keratinozyten ohne Zugabe von Serum. Als Maß für die Proliferation wurde der Einbau von BrDU durch Messung der Absorption bei 450 nm (A[450 nm]) bestimmt. Eine stärkere Proliferation spiegelt sich in höherer Absorption wieder. Als Proliferationskontrolle wurden Zellen verwendet, die unter dem Einfluss von Serum gewachsen sind (Spur "KGM-10% FCS"). Als Vergleich für den Effekt von 1-EBIO wurde ein weiterer hIK1 Aktivator, Zoxazolamin (Spur "1000 μM Zox") verwendet. Die Negativlcontrollen ohne Aktivator bzw. nur mit dem Lösungsmittel DMSO sind mit "KGM" bzw. "KGM-DMSO" gekennzeichnet.

**Figur 8** zeigt den Einfluss verschiedener Konzentrationen des hIK1 Aktivators, 1-EBIO, auf die Serum-induzierte Proliferation von HaCaT Keratinocyten (Spur "DMEM-10%FCS"). Als Maß für die Proliferation wurde der Einbau von BrDU durch Messung der Absorption bei 450 nm (A[450 nm]) bestimmt. Eine stärkere Proliferation spiegelt sich in höherer Absorption wieder. Als negative Kontrolle für das Lösungsmittel, wurde DMSO in DMEM/10%FCS ohne Aktivator verwendet (Spur "DMSO"). Als Positivkontrolle wurde der hIK1-Aktivator Zoxazolamin in DMEM/10%FCS verwendet (Spur "1000 μM Zox").

Die Polypeptidsequenzen von mIK1 und hIK1, sowie deren kodierende Nukleinsäuren sind gemäß SEQ ID Nr. 1 bis SEQ ID Nr. 4 aufgeführt.

Die für die Beispiele verwendeten Oligonukleotide und Polynukleotide sind gemäß SEQ ID Nr. 5 bis SEQ ID Nr. 13 aufgeführt.

**Beispiele**

Beispiel 1: Der "intermediate-conductance" Calcium-abhängige Kaliumkanal hIK1 vermittelt eine lang-anhaltende, durch 1-EBIO modulierte Hyperpolarisierung von Keratinozyten nach Stimulation mit ATP, Bradykinin oder Histamin

Um einen Ionenkanal zu identifizieren, der wesentlich bei der Signaltransduktion wundheilungsspezifischer Signale beteiligt ist, wurden HaCaT-Zellen elektrophysiologisch untersucht. Die Anzucht der HaCaT-Keratinozyten erfolgte in DEM mit 10% FRS (Gibco BRL), den Antibiotika (Penicillin/Streptomycin 100 U/ml) zugesetzt worden war. Die $Ca^{2+}$-Endkonzentration betrug 1,8 mM. Die Zellen wurden inkubiert bis eine konfluente Zellkultur entstanden war; anschließend wurde das Kulturmedium durch "Standard Bath Solution" (SBS; 130 mM NaCl, 3 mM KCl; 2 mM $CaCl_2$; 2 mM $MgCl_2$; 25 mM HEPES/NaHEPES; 10 mM D-Glukose; pH 7,4) ersetzt und die Schalen an einem inversen Mikroskop (Axiovert, Zeiss) untersucht. Die "Patch"-Pipetten wurden aus Borosilikatröhrchen an einer horizontalen Ausziehvorrichtung (DMZ, Zeitz) nach dem zweistufigen Ausziehverfahren hergestellt. Die Pipetten wurden anschließend mit einer Lösung, die 135 mM Kaliumglukonat, 10 mM KCl, 1,6 mM $Na_2HPO_4$, 0,73 mM $CaCl_2$, 1,03 mM $MgCl_2$, 1 mM EGTA, 14 mM HEPES/NaHEPES und 100 mg/ml Nystatin; pH 7,2 enthielt, gefüllt ($Ca^{2+}$-Aktivität in der Lösung= $10^{-7}$ M). Der Widerstand dieser "Patch"-Pipetten betrug 5-8 GΩ im Bad. Nachdem eine Versiegelung im GΩ-Bereich hergestellt wurde (typischerweise 1,5-2 GΩ) wurde der Verstärker auf Strom-Klemm Modus umgestellt. Innerhalb von 3-5 min erreichten die Membranpotentiale stabile Werte. Die elektrophysiologischen Signale wurden mittels eines Axopatch 200 Verstärkers in Verbindung mit einem TL-1 Labmaster Interface und AXOTAPE Software (Axon Instruments) aufgenommen, verstärkt und digitalisiert. Die statistische Auswertung (ein-Weg ANOVA mit Bonferri post-test Vergleich) wurde mit Graphpad prism 2.0 durchgeführt.

Bei Experimenten, bei denen die intrazelluläre $Ca^{2+}$-Konzentration durch Blitzphotolyse von "caged $IP_3$" (Calbiochem) erhöht wurde, wurde das "Patch-Clamp Recording" in der Ganzzell-Konfiguration durchgeführt, wobei eine Pipettenlösung enthaltend 120 mM Kaliumglukonat, 20 mM KCl, 1 mM $MgCl_2$, 10 mM HEPES/NaHEPES, 2 mM $Na_2$ATP, 0,3 mM NaGTP (pH 7,2) und 10-100 μM "caged $IP_3$" verwendet wurde. Nach 5-7 min Ganzzell-Aufzeichnung wurde IP3 durch einen UV-Lichtblitz freigesetzt, der durch eine Xenon-Bogen-Lampe (75 W; Anregungsfilter: 330 ± 40 nm), die mit dem Epifluoreszenzport des Mikroskops verbunden war, generiert wurde. Der UV-Lichtstrahl wurde durch das 40-fach Phasenkontrastobjektiv auf die Keratinozyten fokussiert. Die Dauer des Blitzes (1 s) wurde durch einen Uniblitz "Shutter" (Vincent Assoc.) eingestellt.

Bei Experimenten, bei denen die gesamte Lösung, in der die Zellen inkubiert wurden ausgewechselt wurde, wurde ein konischer Plexiglaseinsatz mit mehrfachen Einflußleitungen und einer Ausflußleitung in die Kulturschale eingesetzt, um das Volumen der Ableitkammer auf 1 ml zu reduzieren. Der Zufluß von Flüssigkeit war Schwerkraftabhängig und wurde durch magnetische Ventile kontrolliert. Der Ausfluß wurde durch das Anlegen eines negativen Drucks mittels einer Vakuumpumpe bewirkt. Die Zugabe von Substanzen erfolgte unter visueller Beobachtung direkt auf die zu untersuchenden Keratinozyten mittels eines ferngesteuerten, magnetgesteuerten Y-Rohrsystem (Durchmesser des Ausflußröhrchens: 100 μm).

Die elektrophysiologischen Ableitungen mittels perforierten "Patches" wurden an HaCaT-Zellen durchgeführt, die zu einem konfluenten Monolayer gewachsen waren. Im Strom-Klemm Modus war das durchschnittliche Membranpotential der Keratinozyten -42 ± 1 mV (n=76) unter Kontrollbedingungen. Die Perfusion der HaCaT-Zellen mit ATP (1-1000 μM) induzierte eine biphasische Änderung des Membranpotentials, die aus einer kurzen, transienten Depolarisierung gefolgt von einer deutlichen und langanhaltenden Hyperpolarisierung bestand (Figur 1 A). Die starke Hyperpolarisierung dauerte bei ATP-Konzentrationen von ≥ 10 μM mindestens 5 min nach ATP-Zugabe (5 min) an, wobei einige Keratinozyten sogar während der ganzen Beobachtungszeit (bis zu 20 min) hyperpolarisiert blieben. Diese Beobachtung zeigt, daß Änderungen der ATP-Konzentration langanhaltende Änderungen des Membranpotentials und somit der Keratinozytenaktivität bewirken.

Die Hyperpolarisierung nach Zugabe von 1 μM ATP stellte einen durch Auswaschen der Lösung rasch reversiblen Prozeß dar (n=9). Bei Zugabe von 10 μM ATP wurde eine Hyperpolarisierung um 30 ± 2 mV (n=28) beobachtet. Eine quantitativ und kinetisch praktisch identische Antwort wurde gemessen, wenn den Keratinozyten Bradykinin (10 μM, n=3) oder Histamin (100 μM, n=4) zugegeben wurde, was darauf hinweist, daß die Substanzen ATP, Histamin und Bradykinin dasselbe Effektorsystem regulieren (Figur 1 B).

Da bekannt ist, daß alle drei extrazellulären Mediatoren den Phosphoinositid-Metabolismus in Keratinozyten stimulieren können (Talwar et al., 1989, J. Invest. Dermatol. 93:241-245; Pillai et al., 1992, J. Clin. Invest. 90: 42-51 ; Rosenbach et al., 1993, Arch. Dermatol. Res. 285: 393-396), wurde untersucht, ob die Bildung von $IP_3$ und der nachfolgende Anstieg der cytosolischen $Ca^{2+}$-Konzentration Teil des Signaltransduktionsweges sind. Dazu wurden Ganzzell-Ableitungen mit Keratinozyten durchgeführt, die mit "caged-$IP_3$" (10-100 μM) beladen wurden. In allen untersuchten Zellen (n=7) wurde beobachtet, daß die plötzliche Umwandlung von "caged-$IP_3$" in $IP_3$ durch Blitz-Photolyse zu einer schnellen, transienten Depolarisierung, gefolgt von einer Hyperpolarisierung führte (Figur 1 C). Die $IP_3$-induzierte biphasische Spannungsänderung stimmte sehr genau mit der überein, die nach Zugabe von 1 μM ATP beobachtet wurde. Die auffallende Ähnlichkeit zwischen den elektrophysiologischen Antworten, die durch die drei Agonisten ATP, Histamin, Bradykinin sowie $IP_3$ bewirkt wurden deutet darauf hin, daß die biphasische Änderung der Membranspannung durch die Mobilisierung von $Ca^{2+}$ aus $IP_3$-sensitiven Speichern vermittelt wurde.

Da die drei extrazellulären Mediatoren neben dem $IP_3$-vermittelten Signalweg auch weitere Signalkaskaden aktivieren könnten, wurde untersucht, ob die Stimulation der Adenylylzyklase (AC) oder der Proteinkinase C (PKC) die ATP-vermittelte elek-trophysiologische Antwort nachahmen oder beeinflussen.

Die Vorbehandlung der HaCaT-Zellen mit dem AC-Aktivator Forskolin (10 μM) für 3-10 min beeinflußte weder das Ruhepotential noch den Spannungsverlauf, der nach ATP-Zugabe beobachtet wird (n=5). Die Stimulation von PKC mit dem Phorbolester PMA (60 ng/μl) verursachte eine langsame Depolarisierung auf -32 ± 2 mV, beeinflußte jedoch nicht den Effekt von ATP (n=4). Die Inhibition der PKC mit Calphostin C (50 nM) wiederum führte zu einer schwachen Hyperpolarisierung auf -58 ± 3 mV, beeinflußte jedoch ebenfalls nicht die ATP-vermittelte Spannungsänderung.

Mögliche Kandidaten, die für diese erstmals nachgewiesene starke und langanhaltende Veränderung des

Membranpotentials nach Stimulation mit den Signalmolekülen ATP, Histamin und Bradykinin verantwortlich sein können, sind Calcium-abhängige Kaliumkanäle. Um den für die Hyperpolarisierung verantwortlichen Ionenkanal zu identifizieren, wurde der Einfluß verschiedener $K^+$-Kanalblocker auf den ATP-vermittelten elektrophysiologischen Effekt untersucht. Charybdotoxin (ChTx; Alomone Labs) verursachte eine dosisabhängige Inhibition der ATP-vermittelten Hyperpolarisierung. Wie in Figur 2 A gezeigt, verlangsamte die Zugabe von ChTx (10-100 nM, n=15) die Repolarisierung der schnellen Depolarisierung und verhinderte eine Hyperpolarisierung. Wurde ChTx in einer Konzentration von 100 nM (n=4) eingesetzt, verhinderte ChTx die Repolarisierung vollständig und das Membranpotential blieb auf einem depolarisierten Plateau solange ATP zugegeben wurde. Derselbe Effekt wurde mit 1 μM Clotrimazol (n=3; Figur 2 B) beobachtet. Wurde ChTx (100 nM) zugegeben, nachdem die ATP-vermittelte Hyperpolarisierung gemessen wurde, wurde eine vollständige Umkehrung der Hyperpolarisierung beobachtet, obwohl ATP weiterhin vorhanden war (n=2; Figur 2 C). Keiner der anderen getesteten $K^+$-Kanalblocker (TEA, $Ba^{2+}$, und Verapamil) führten zu einer vergleichbaren Depolarisierung auf ein Plateau während der Behandlung mit ATP. Verapamil (10-100μM), das $K^+$-Ströme in terminal differenzierenden Keratinozyten inhibiert (Mauro et al., 1997, J. Invest. Derm., 108: 864-870), beeinflußte den hyperpolarisierenden Effekt von ATP nicht (Hyperpolarisierung auf -73 ± 1 mV, n=3). $K^+$-Kanäle, die durch Clotrimazol und Charybdotoxin inhibiert werden, sind spannungsunabhängige Calcium-aktivierte Kaliumkanäle. Um die Identität weiter aufzuklären, wurde untersucht, ob 1-EBIO, ein spezifischer Aktivator des "intermediate-conductance" Calcium-aktivierte Kaliumkanals hIK1 (Syme et al., 2000, Am. J. Physiol., 278: C570-581), die ATP-vermittelte Hyperpolarisierung nachahmen kann. Wie in Figur 2 D gezeigt, induzierte 1-EBIO (1 mM; Tocris) eine schnelle und starke Hyperpolarisierung auf -83 ± 3 mV (n=3). Wurde ATP (10 μM) in der Gegenwart von 1-EBIO zugegeben, wurde die typische transiente Depolarisierung beobachtet, die jedoch viel stärker war als unter Kontrollbedingungen (vgl. Figur 1 A), was sich durch den erhöhten Fluß von Chloridionen und Kationen durch die erhöhte Spannung über die Membran erklären läßt. Weiterhin wurde beobachtet, dass ein Derivat von 1-EBIO, DCEBIO (5,6-dichloro-1-ethyl-1,3-dihydro-2H-benzimidazol-2-on) eine 300-1000fach stärkere Depolarisation als 1-EBIO der HaCaT Zellen hervorrufen kann. Dieses Resultat zeigt, dass DCEBIO in viel geringeren Konzentrationen eingesetzt werden kann, um den gleichen elektrophysiologischen Effekt wie mit 1-EBIO zu erreichen (ca. 300-1000 fach weniger konzentriert) und ist somit ein besonders bevorzugter Modulator des hIK1-Kanals.

Somit konnte zum ersten Mal hIK1 in Keratinozyten nachgewiesen und dessen Rolle bei der Keratinozytenaktivität aufgezeigt werden. Dies zeigt, daß das Polypeptid hIK1 und dieses kodierende Nukleinsäuren sowie Modulatoren des Kanals zur Verwendung bei Krankheiten, die mit gestörter Keratinozytenaktivität in Zusammenhang stehen, insbesondere bei Wundheilungsstörungen und/oder Psoriasis, geeignet sind.

Beispiel 2: Nachweis der Expression von hIK1 in HaCaT-Zellen und differentielle Regulation von hsk4 (=IK1) in differenzierenden Keratinozyten

Um zu untersuchen, ob nicht nur die Aktivität, sondern auch die Expression von hIK1 in Keratinozyten reguliert ist, wurde überprüft, ob die Aktivität der Keratinozyten mit der Expression von hIK1 korreliert.

Dazu wurden proliferierende subkonfluente Zellen, Zellen, die gerade Konfluenz erreicht hatten, und ruhende Zellen (4 Tage nach Konfluenz) untersucht. Die Aktivität dieser Keratinozyten wurde anhand der differenzierungsspezifischen Keratin-10-Expression bestimmt. Dazu wurden die Zellen in eiskaltem PBS gewaschen und mit Aceton/Methanol (1:1) 20 min bei -20°C fixiert. Endogene Peroxidasen wurden mit 1 % $H_2O_2$ bei Raumtemperatur blockiert. Nachdem die unspezifischen Bindungsstellen mit 3% BSA in PBS abgesättigt worden waren, wurden die Zellen über Nacht in einer 1:500 Verdünnung des Maus-anti-K10 Antikörpers (DAKO) in 3% BSA in PBS bei 4°C inkubiert und anschließend drei Mal mit PBS und einmal mit 3% BSA in PBS gewaschen. Nach 2 h Inkubation mit einem Peroxidase-gekoppelten anti-Maus Antikörper bei Raumtemperatur wurden die Zellen dreimal mit PBS und einmal mit dd$H_2O$ gewaschen. Anschließend wurden die Zellen mit dem Peroxidase Substrat-Kit und 3-Amino-9-ethylcarbazol als chromogenes Substrat (Vector Laboratories) gefärbt. Während die proliferierenden Zellen und die Zellen, die gerade Konfluenz erreicht hatten, keine Färbung aufwiesen, zeigte es sich, daß ein Großteil der postkonfluenten Zellen gefärbt war (Figur 3). Die Expression des Differenzierungs-spezifischen Gens Keratin 10 zeigte, daß die ruhenden Zellen zu einem großen Teil aus partiell differenzierten Zellen bestanden (Fuchs, 1988, Trends Genet. 4:277-281; Ryle et al., 1989, Differentiation, 40:42-54).

Aus parallel angezogenen, proliferierenden, konfluenten als auch postkonfluenten Zellen wurde RNA nach Standardmethoden isoliert (Chomczynski und Sacchi, 1987, Anal. Biochem. 62:156-159) und ein "RNAse Protection Assay" zum Nachweis von hIK1 durchgeführt (Werner et al., 1992, Proc. Natl. Acad. Sci. USA 89: 6896-6900).

In Kürze: Es wurde ein mittels PCR amplifiziertes Genfragment in den Transkriptionsvektor pBluescript KSII (+) (Stratagene) kloniert und das Plasmid linearisiert. Ein "antisense"-Transkript wurde anschließend *in vitro* mittels T3 oder T7 Polymerase und $^{32}$P-UTP (800 Ci/mol) hergestellt. Je 20 μg RNA wurden bei 42°C Übernacht mit 100,000 cpm markiertem "antisense"-Transkript hybridisiert. Als Negativkontrolle wurden 20 μg tRNA verwendet. Die Hybride wurden anschließend 1 h bei 30°C mit RNAse und RNAse T1 verdaut. Die geschützten Fragmente wurden auf 5%

Acrylamid/8 M Harnstoff Gelen aufgetrennt und mittels Autoradiographie analysiert. Für den Nachweis von hIK1 wurde eine Riboprobe (SEQ ID Nr. 5) verwendet, die komplemetär zu den Nukleotiden 740-1004 des humanen hIK1-Gens ist. Das Ergebnis des Versuchs ist in Figur 4 dargestellt. Es wurde beobachtet, daß hIK1 stark in proliferierenden (Spur "1") und gerade konfluenten Zellen (Spur "2") exprimiert wird, während die Menge in postkonfluenten, partiell differenzierten Zellen signifikant reduziert war (Spur "3"). Bei der Negativkontrolle (Spur "tRNA") wurde kein Signal beobachtet. In der Spur "Probe" wurden 1000 cpm der Hybridisierungssonde als Kontrolle der radioaktiven Markierung und als Größenstandard aufgetragen. Die Ergebnisse, die drei Mal mit RNAs von unabhängigen Versuchen wiederholt wurden, zeigen daher, daß der Beginn der Differenzierung von HaCaT-Keratinozyten mit einer verringerten hIK1-mRNA einhergeht und somit ein Zusammenhang zwischen Keratinozytenaktivität und hIK1-Expression besteht.

Beispiel 3: Differentielle Expression von mIK1 in Wunden der Maus

Die Haut ist ein hoch-komplexes System, dessen Zellzusammensetzung z.B. während einer Erkrankung räumlichen und zeitlichen Änderungen unterworfen ist. Desweiteren ändern sich die Aktivitäten der Zellen beispielweise durch Wechselwirkung mit anderen Zellen. Daher können Prozesse in der Haut nur unzureichend durch einfache Zellsysteme nachgeahmt werden. Um zu bestätigen, daß IK1 nicht nur in Zellkultur, sondern auch bei physiologischen Prozessen in der Haut eine wichtige Rolle bei der Regulation von Zellaktivitäten spielt, wurde am Beispiel der Wundheilung und verschiedener Wundheilungszustände die Expression von murinen mIK 1 (SEQ ID Nr. 2) in Biopsien von Stanzwunden verfolgt. Die Untersuchung erfolgte mittels "TaqMan Analyse" im GeneAmp5700 von Applied Biosystems. Normal heilende Tag 1-Wunden und intakte Haut wurde aus mit isotonischer Kochsalzlösung behandelten 10 Wochen alten BALB/c Mäusen durch Scherenschnitt gewonnen. Um Gewebe von Mäusen mit schlecht heilenden Wunden zu gewinnen, wurden BALB/c Mäuse vor der Verwundung mit Dexamethason (0,5 mg Dexamethason in isotonischer Salzlösung pro kg Körpergewicht i.p. zwei mal pro Tag für 5 Tage) behandelt. Als Kontrolle wurde intakte Haut von Mäusen verwendet, die mit isotonischer Kochsalzlösung, wie oben beschrieben, behandelt wurden. Um Gewebe von jungen und alten Mäusen zu gewinnen, wurden unbehandelte Tag 1-Wunden und intakte Hautproben von 4 Wochen alten und 12 Monate alten BALB/c Mäusen verwendet. Wundgewebe und intakte Haut von Mäusen mit Diabetes (*db*/*db* Maus) wurde erhalten, indem unbehandelte Tag 1-Wunden und intakte Haut von 10 Wochen alten C57B/Ks-*db*/*db*/Ola Mäusen mittels Scherenschnitt isoliert wurden. C57B/Ks wildtyp Mäuse wurden dabei als Kontrolltiere eingesetzt. Von letzteren Tieren wurde ebenfalls intakte Haut sowie unbehandelte Tag 1-Wunden gewonnen.

Die Isolierung der RNA erfolgte durch Homogenisieren der Biopsien in RNAclean Puffer (AGS, Heidelberg), dem 1/100 Volumenanteil 2-Mercaptoethanol zugesetzt worden war unter Verwendung eines Dispersers. Anschließend wurde die RNA durch zweimaliges Phenolisieren mittels mit Wasser gesättigtem saurem Phenol und in Gegenwart von 1-Brom-3-Chlor-Propan extrahiert. Anschließend wurden eine Isopropanol- und eine Ethanol-Fällung durchgeführt und die RNA mit 75% Ethanol gewaschen. Danach wurde ein DNase I-Verdau der RNA durchgeführt. Hierzu wurden 20 µg RNA (ad 50 µl mit DEPC-behandeltem Wasser) mit 5,7 µl Transkriptions-Puffer (Roche), 1 µl RNase-Inhibitor (Roche; 40 U/µl) und 1 µl DNase I (Roche; 10 U/µl) 20 min bei 37°C inkubiert. Dann wurde wiederum 1 µl DNase I zugegeben und weitere 20 min bei 37°C inkubiert. Anschließend wurde die RNA phenolisiert, Ethanol-gefällt und gewaschen. Alle oben aufgeführten Schritte wurden mit DEPC (Diethylpyrocarbonat)-behandelten Lösungen bzw. Flüssigkeiten durchgeführt soweit diese keine reaktiven Aminogruppen enthielten. Anschließend erfolgte die Herstellung von cDNA aus der extrahierten RNA. Dies erfolgte in Gegenwart von 1 x "TaqMan" RT-Puffer (PE Applied Biosystems), 5,5 mM $MgCl_2$ (PE Applied Biosystems), je 500 µM dNTPs (PE Applied Biosystems), 2,5 µM Random Hexamere (PE Applied Biosystems), 1,25 U/µl MultiScribe Reverse Transcriptase (50 U/µl, PE Applied Biosystems), 0,4 U/µl RNase-Inhibitor (20 U/µl, PE Applied Biosystems), 20 µl RNA (50 ng/µl) und DEPC-behandeltem Wasser (ad 100 µl Volumen). Nach Zugabe der RNA und guter Durchmischung wurde die Lösung auf 2 0,2 ml Gefäße verteilt (je 50 µl) und die reverse Transkription in einem Temperatur-Cycler durchgeführt (10 min bei 25°C; 30 min bei 48°C und 5 min bei 95°C).

Die nachfolgende Quantifizierung der cDNA erfolgte mittels quantitativer PCR unter Verwendung des "SYBR-Green PCR Master Mixes" (PE Applied Biosystems), wobei für die Bestimmung der mIK1-cDNA Menge eine Dreifachbestimmung (jeweils mit mIK1-Primern und GAPDH-Primem) durchgeführt wurde. Die Stammlösung für jedes Triplett enthielt bei 57 µl Gesamtvolumen 37,5 µl 2 x "SYBR Master Mix", 0,75 µl AmpErase UNG (1 U/µl) und 18,75 µl DEPC-behandeltes Wasser. Pro Dreifachbestimmung wurden zu 57 µl Stammlösung je 1,5 µl Vorwärts- und Rüchwärts-Primer (mIK1 Primer 1 (SEQ ID Nr. 6) und mIK1 Primer 2 (SEQ ID Nr. 7)) in einem zuvor optimierten Konzentrationsverhältnis hinzugefügt. Je 60 µl der Stammlösung/Primer-Mischung wurde mit 15 µl cDNA-Lösung (2 ng/µl) gemischt und auf 3 "wells" verteilt. Parallel dazu wurde eine Stammlösung mit Primern zur Bestimmung von GAPDH (SEQ ID Nr. 12 und SEQ ID Nr. 13) als Referenz hergestellt, mit weiteren 15 µl der gleichen cDNA-Lösung gemischt und auf 3 "Wells" verteilt. Außerdem wurden verschiedene cDNA-Lösungen als Verdünnungsreihe hergestellt um eine Standardkurve für die GAPDH-PCR zu erstellen (4 ng/µl; 2 ng/µl; 1 ng/µl; 0,5 ng/µl und 0,25 ng/µl). Je 15 µl dieser cDNA-Lösungen wurden mit 60 µl Stammlösung/Primer-Mischung zur Bestimmung von GAPDH gemischt und auf 3 "wells" verteilt. Ebenso wurde eine Standardkurve für die mIK1-PCR erstellt; dabei wurden dieselben Verdünnungen, die auch für die GAPDH-Standardkurve zum Einsatz kamen, verwendet. Als Kontrolle diente ein PCR-Ansatz ohne cDNA. Zu jeweils 60 µl Stammlösung/Primer-Mischung von jeweils mIK1 und GAPDH wurden je 15 µl DEPC-Wasser gegeben, gemischt und jeweils auf 3 "Wells" verteilt. Die Amplifikation der Ansätze wurde im GeneAmp 5700 durchgeführt (2 min bei 50°C; 10 min bei 95°C, gefolgt von 3 Zyklen mit 15 s bei 96°C und 2 min bei 60°C; danach 37 Zyklen mit 15 s bei 95°C und 1 min bei 60°C).

Die Auswertung erfolgte durch die Bestimmung der relativen Abundanz von mIK1 im Bezug auf die GAPDH-Referenz. Dazu wurde zuerst eine Standardkurve erstellt, indem die $C_T$-Werte der Verdünnungsreihe gegen den Logarithmus der cDNA-Menge im PCR-Ansatz (in ng umgeschriebener RNA) aufgetragen wurde und die Steigung (s) der Geraden ermittelt wurde. Die Effizienz (E) der PCR ergibt sich dann wie folgt: $E = 10^{-1/s} - 1$. Die relative Abundanz (X) der untersuchten cDNA Spezies mIK1 (Y) im Bezug auf GAPDH ist dann: $X=(1+E_{GAPDH})^{C_T(GAPDH)}/(1+E_Y)^{C_T(Y)}$. Anschließend wurden die Zahlenwerte normiert, indem die Menge an cDNA aus intakte Haut von 10 Wochen alten BALB/c Kontrolltieren bzw. die intakte Haut der C57B/Ks Kontrolltiere gleich 1 gesetzt wurden. Die relativen Änderungen der mIK1-Expression in verschiedenen Wundheilungszuständen sind in Tabelle 2 zusammengestellt. Es zeigt sich, daß in allen Wundheilungszuständen der Maus eine erhöhte mIK1-Expression im Vergleich zur intakten Haut zu beobachten war. Auffällig war, daß in der Wunde der Kontrolltiere und der gut heilenden Wunde junger Mäuse eine starke Hochregulation um den Faktor 3 bzw. 4 gemessen wurde, während in Wunden alter Mäuse nur eine schwache, nicht-signifikante Erhöhung der Expression beobachtet wurde. Auch in schlecht-heilenden, mit Dexamethason-behandelten Wunden wurde eine im Vergleich zu Kontrolltieren weniger starker Anstieg der Expression beobachtet (Faktor 2,2). Dies unterstreicht, daß in murinen Wunden die Wundheilung mit einer starken Erhöhung der mIK1-Expression einhergeht und daß eine gestörte Keratinozytenaktivität, wie sie in Wunden älterer oder mit Dexamethason-behandelten Tieren verstärkter auftreten kann, durch eine verminderte mIK1-Expression gekennzeichnet sein kann. Generell kann daher eine Modulation, bevorzugterweise eine Erhöhung der Expression und/oder Aktivität von IK1 vorteilhaft zur Behandlung oder Prävention von Krankheiten, die mit gestörter Keratinozytenaktivität im Zusammenhang stehen, eingesetzt werden.

Um geeignete Zeitpunkte für eine Diagnose, die Prävention und/oder Behandlung einer mit gestörter Keratinozytenaktivität im Zusammenhang stehenden Krankheit zu bestimmen, wurde die Expression von mIK1 in murinen Biopsien von verschiedenen Zeitpunkten der Wundheilung bestimmt. Dabei wurden zum Zeitpunkt 1 h, 7 h, 15 h, 24 h, 3 Tage, 5 Tage, 7 Tage und 14 Tage nach Wundsetzung Wundbiopsien sowie eine Biopsie von intakter Haut von 10 Wochen alten Kontrollmäusen wie oben beschrieben durch Scherenschnitt erhalten. Die RNA wurde aus dem Gewebe isoliert und die relative Menge an mIK1 in den Biopsien bestimmt. Dabei zeigte sich daß bereits 7 h nach Wundsetzung eine deutliche Hochregulation der mIK1-Expression zu beobachten ist (Tabelle 3). Bis zum Ende des Beobachtungszeitraums (Tag 14 nach Wundsetzung) blieb die mIK1-Expression im Bezug auf intakte Haut erhöht, was darauf hinweist, daß die Regulation der mIK1-Expression über den gesamten Verlauf der Wundheilung für einen optimalen Verlauf der Wundheilung essentiell ist. Dies unterstreicht die besondere Eignung von IK1-Kanälen zur Diagnose, Prävention und/oder Behandlung von Krankheiten, die mit gestörter Keratinozytenaktivität im Zusammenhang stehen, da zu jedem Zeitpunkt eine Diagnose durchgeführt, bzw. die Prävention und/oder Behandlung begonnen werden kann.

Beispiel 4: Differentielle Expression von hIK1 in humanen Wunden

Mäuse haben sich als geeignetes Modellsystem für die Untersuchung der Wundheilung im Menschen erwiesen. Trotzdem sollte überprüft werden, ob eine in der Maus am Beispiel der Wundheilung beobachtete differentielle Expression der erfindungsgemäß verwendbaren Nukleinsäuren auch im Menschen nachgewiesen werden kann. Dazu wurden von gesunden Probanden aus unbehandelter intakter Haut, von Tag 1- Wunden oder von Tag 5-Wunden mittels 4 mm bzw. 6 mm Stanzen Hautproben entnommen. Von jeder Gruppe (intakte Haut, Tag 1-Wunde, Tag 5-Wunde) wurden die Biopsien von jeweils 14 Probanden gepoolt. Außerdem wurde von Patienten mit chronischen venösen Ulzera (Ulcera cruris venosum) Stanzbiopsien gleichzeitig sowohl von intakter Haut als auch vom Wundgrund und Wundrand entnommen. Von jeder Gruppe (intakte Haut, Wundrand, Wundgrund) wurden die Biopsien von jeweils 6 Probanden gepoolt. Die Biopsien wurden mittels einer Kugelmühle zerkleinert und daraus, wie im Beispiel 3 beschrieben, RNA isoliert wie in Beispiel 3 beschrieben, danach DNase I verdaut und anschließend in cDNA umgeschrieben. Die Quantifizierung wundheilungsrelevanter cDNAs erfolgte ebenfalls wie in Beispiel 3 beschrieben. Die Ergebnisse der Experimente sind in Tabelle 4 zusammengestellt. Für die Analyse von hIK1 wurden die Primer für die Amplifikation (hGAPDH-Primer 1: CATGGGTGTGAACCATGAGAAG (SEQ ID Nr. 10); hGAPDH-Primer 2: CTAAGCAGTTGGTGGTGCAGG (SEQ ID Nr. 11), hIK1 Primer 1: GCGCTCTCAATCAAGTCCG (SEQ ID Nr. 8), hIK1 Primer 2: GTGTTCATGTAAAGCTTGGCCA (SEQ ID Nr. 9)) anhand der bekannten Sequenzen von humanem GAPDH (GenBank: M17851) und humanem hIK1 (SEQ ID Nr. 1) ausgewählt. Für die Quantifizierung wurden je Ansatz cDNA aus 10 ng revers transkribierter Gesamt-RNA in einem Gesamtvolumen von 25 µl amplifiziert. Die PCR wurde entsprechend den Angaben des Herstellers (PE Applied Biosystems, SYBR Green PCR and RT-PCR Reagents Protocol, 1998) durchgeführt. Die $C_T$-Werte wurden analysiert und daraus wurde die Häufigkeit von hIK1-mRNA relativ zu GAPDH-mRNA berechnet. Die Ergebnisse des Versuchs sind in Tabelle 4 dargestellt. Es zeigte sich, daß die Menge an hIK1-mRNA in humanen Tag 1-Wunden im Vergleich zu intakter Haut nur leicht erhöht war, während am Tag 5 nach Wundsetzung eine verminderte Expression von hIK1 gemessen wurde, was beispielsweise durch die verstärkte Differenzierung der Keratinozyten (siehe Beispiel 2) begründet sein kann. Im Wundgrund von Ulkus-Patienten mit stark gestörter Keratinozytenaktivität dagegen ist keine verminderte Expression im Vergleich zu intakter Haut beobachtbar. Dies zeigt, daß eine Regulation der Keratinozytenaktivität essentiell ist um Erkrankungen, die mit gestörter Keratinozytenaktivität im Zusammenhang stehen, zu vermeiden und daß daher hIK1 ein geeignetes therapeutisches Ziel darstellt. Generell kann daher eine Modulation, bevorzugterweise eine Erhöhung der Expression und/oder Aktiviät von IK1 vorteilhaft zur Behandlung oder Prävention von Krankheiten, die mit gestörter Keratinozytenahrtivität im Zusammenhang stehen, eingesetzt werden. Bevorzugterweise ist die Expression von IK1 in Wunden, insbesondere Ulzera der Haut zu erhöhen.

Beispiel 5: Lokalisierung von hIK1 in verschiedenen Organen

Die Expression von hIK1 wurde bereits in verschiedenen Geweben im Menschen nachgewiesen (Ishii et al. 1997; Proc. Natl. Acad. Sci. USA 94: 11651-11656; Joiner et al. 1997; Proc. Natl. Acad. Sci. USA 94: 11013-11018; WO 99/03882). Auffällig ist, daß die Expression auf nicht elektrisch erregbare Organe, wie Plazenta und Prostata beschränkt ist, jedoch keine Expression im Gehirn oder im Herzen nachgewiesen wurde. Dies spiegelt sich in der Lehrmeinung wider, daß die Expression von hIK1 im Menschen auf Zellen des Blutes und des Blutgefäßsystems beschränkt ist (WO00/34248). Aufgrund der selektiven Expression in nicht-erregbaren Organen gelten Modulatoren dieses Kanals als geeignete Medikamente für eine Vielzahl von Krankheiten in verschiedenen Verabreichungensformen, da die Gefahr kardiovaskulärer Komplikationen als gering angenommen wird. Die Expression in Haut wurde trotz der Vielzahl der Veröffentlichungen, die sich mit der Lokalisierung des hIK1 in Geweben oder Zellen beschäftigen, bislang weder im Menschen noch in einem anderen Säugetier nachgewiesen.

Um zu untersuchen ob und in welcher Menge hIK1 in der Haut relativ zu anderen Organen exprimiert wird, wurden Organ-spezifische Gesamt-RNA Pools von erwachsenen menschlichen Spendern verwendet (BioChain Institute, Inc.): von 5 Spendern wurde Nieren-Gesamt-RNA isoliert, von 5 Spendern Lungen-Gesamt-RNA, von 4 Spendern Fettgewebe-Gesamt-RNA, von 5 Spendern Leber-Gesamt-RNA, von 5 Spendern Gehirn-Gesamt-RNA, von 5 Spendern Herz-Gesamt-RNA, von 2 Spendern Milz-Gesamt-RNA, von 5 Spendern Skelettmuskel-Gesamt-RNA und von 5 Spendern Dünndarm-Gesamt-RNA isoliert, mit DNAse I verdaut, und die Gesamt-RNAs getrennnt nach Organen gepoolt. Anschließend wurde eine "TaqMan Analyse" wie in Beispiel 3 beschrieben mit den Gesamt-RNAs dieser Organe und Gesamt-RNA aus Haut, die wie in Beispiel 4 erhalten wurde, durchgeführt und die Häufigkeit von hIK1-mRNA relativ zu GAPDH-mRNA bestimmt. Die relativen Mengen an hIK1-mRNA in den Organen sind in Tabelle 5 angegeben. Überraschenderweise konnte zum ersten Mal nachgewiesen werden, daß hIK1 in der Haut exprimiert wird. Zudem konnte beobachtet werden, daß hIK1 in Haut in vergleichbarem Maße exprimiert wird wie beispielsweise in Lunge und Milz. In letzteren beiden Organen war eine vergleichsweise starke Expression von hIK1 nachgewiesen worden (WO 99/03882). Weiterhin konnte überraschenderweise die Expression von hIK1 in Gehirn, Herz und Skelettmuskel nachgewiesen werden. Das zum Stand der Technik widersprüchliche Ergebnis ist wahrscheinlich durch die höhere Empfindlichkeit der TaqMan Analyse gegenüber der im Stand der Technik verwendete "Northern Blot"-Analyse begründet. Dieses Experiment zeigt daher zwei Aspekte der vorliegenden Erfindung auf. Einerseits ist die Expression von hIK1 eine Voraussetzung dafür, daß das Gen hIK1, das Protein und Modulatoren seiner Aktivität zur Diagnose, Prävention und/oder Behandlung von Krankheiten, die mit gestörter Keratinozytenaktivität im Zusammenhang stehen, verwendet werden können. Andererseits zeigt der überraschende Nachweis der Expression von hIK1 in erregbaren Organen, daß entgegen der Lehrmeinung kardiovaskuläre Komplikationen beispielsweise bei oraler Gabe wahrscheinlich sind. Die Prävention und/oder Behandlung von Krankheiten, die mit gestörter Keratinozytenaktivität im Zusammenhang stehen, insbesondere Wundheilungsstörungen und/oder Psoriasis, sind dagegen vorzugsweise topisch zu behandeln, da diese Form der Applikation den direkten und spezifischen Kontakt mit den Keratinozyten gewährleistet. Daher ist die Verwendung von hIK1, dessen kodierenden Nukleinsäuren und/oder Modulatoren seiner Aktivität besonders vorteilhaft bei der Prävention und/oder Behandlung dieser Krankheiten.

Beispiel 6: Sowohl die Aktivierung als auch die Inhibition der hIK1- Aktivität beeinflussen die Keratinozytenaktivität

Es sollte nun gezeigt werden, daß Modulatoren des IK-Kanals die Keratinozytenaktivität regulieren können.

Am Beispiel des Aktivators von hIK1, 1-EBIO (Syme et al., 2000, Am. J. Physiol., 278: C570-C581), wurde der Einfluß von Modulatoren auf die Differenzierung untersucht. Dazu wurden HaCaT-Zellen 2 Tage in Medium angezogen. Zu den proliferierenden, subkonfluenten Zellen wurde dann 1 mM 1-EBIO (Tocris) zum Medium zugegeben und 3 Tage inkubiert. Anschließend wurde die Differenzierung der Keratinozyten mittels Immunfärbung mit einem K10-Antikörper wie in Beispiel 2 gezeigt untersucht. Dabei zeigte sich, daß ein Großteil der Kontrollzellen, denen kein

1-EBIO zugegeben wurde, partiell differenziert waren, was durch Immunfärbung unter Verwendung des Anti-K10-Antikörper wie in Beispiel 2 gezeigt wurde. Die in Gegenwart von 1-EBIO inkubierten Zellen wiesen einen signifikant reduzierten Anteil an gefärbten Zellen auf.

Diese Beobachtungen wurden durch Western Blot Analysen bestätigt. Dazu wurden in einer 6-"Well"- Platte $5x10^5$ HaCaT Zellen pro "Well" in Medium angezogen. Am nächsten Tag wurde das Medium gewechselt auf Medium, das verschiedene Konzentrationen an 1-EBIO bzw. 2-Amino-5-Chlorbenzoxazol (Zoxazolamin (Aldrich)) enthielt. Als Lösungsmittelkontrolle wurde 1% DMSO (Spur "C") in Medium benutzt. Die Zellen wurden 4 Tage lang inkubiert, wobei das Medium täglich ersetzt wurde. Nach 4 Tagen wurden die Proteinkonzentration in den Zelllysaten bestimmt (BCA Assay, Pierce). Gleiche Proteinmengen aus den verschiedenen Lysaten wurden anschliessend mittels Immunoblot gegen die Keratinozytendifferenzierungsmarker Involucrin (1:500, Sigma) sowie Keratin 1 und 10 (1:250, Progen) verglichen (Figur 6). Es ist eindeutig erkennbar, dass die beiden Aktivatoren von hIK1, 1-EBIO und Zoxazolamin, die Differenzierung und somit die Keratinozytenaktivität von HaCaT Zellen dosisabhängig inhibieren.

Der Einfluß von Aktivatoren des IK1 auf die Proliferation wurde anhand von 1-EBIO und Zoxazolamin (supra) untersucht. Dabei wurde zunächst der Einfluss verschiedener Konzentrationen von 1-EBIO auf die Wachstumsrate von HaCaT Zellen in An- und Abwesenheit von Serum gemessen. Zu diesem Zweck wurden $1x10^4$ HaCaT Zellen pro "Well" in einer 96-"Well" Platte 5 Tage lang in Keratinozytenwachstumsmedium (KGM) kultiviert, um den Zellzyklus zu synchronisieren, gefolgt von einer zweitägigen Inkubation verschiedener Konzentrationen an 1-EBIO in KGM (siehe Figuren 7 und 8) in Abwesenheit (Figur 7) und in Anwesenheit (Figur 8) von Serum. Als Positivkontrolle bezüglich der Proliferation wurde KGM/10% FCS verwendet, während der IK1-Aktivator Zoxazolamin in einer Konzentration von 1 mM als Vergleich für den 1-EBIO-Effekt genutzt wurde. Als Lösungmittelkontrolle diente 1% DMSO in KGM. Die Zellproliferation wurde mit Hilfe des Einbaus von Bromo-Dideoxyuridine (BrDU) während der Zellteilung gemäß den Angaben des Herstellers gemessen ("The Cell Proliferation ELISA, BrDU, Roche). Der Einbau von BrDU wurde dabei durch die Bestimmung der Absorption bei 450 nm quantifiziert. In Figur 7 und 8 ist jeweils die Absorption (A[450nm]) als Maß für die Proliferationsrate auf der Y-Achse aufgetragen. Eine höhere Absorption spiegelt eine stärkere Proliferation wieder. Der Vergleich der beiden IK1 Aktivatoren 1-EBIO und Zoxazolamin zwischen den Serum-behandelten und den unbehandelten Zellen zeigt eindeutig, dass die beiden Aktivatoren zum Einen alleine nicht in der Lage sind, die Zellproliferation zu stimulieren (Figur 7). Darüber hinaus sind beide Aktivatoren in der Lage die seruminduzierte Proliferation zu inhibieren und können somit die Keratinozytenaktivität modulieren (Figur 8). Dabei ist zu erwähnen, dass die Morphologie der Zellen unverändert bleibt, das heißt, dass die Zellen in einem ruhenden Zustand "verharren" und es keine Anzeichen von Apoptose gibt. Figur 8 zeigt ausserdem, dass die Inhibition durch den hIK1 Aktivator 1-EBIO dosisabhängig ist und den stärksten Effekt bei einer Konzentration von 1 mM zeigt.

Um den Effekt der Inhibitoren des hIK1 Kanals, Clotrimazol und Charybdotoxin, zu untersuchen, wurde zu subkonfluent gewachsenen Zellen entweder 10 μM Clotrimazol, 200 nM Charybdotoxin (ChTx) oder 1 mM 1-EBIO zugegeben und jeden Tag die Anzahl Zellen pro well bestimmt.

Wie aus Figur 5 (n=3) ersichtlich ist, inhibierte die Zugabe sowohl eines der beiden Inhibitoren als auch die Zugabe des Aktivators von hIK1 die Proliferation, während das Wachstum der Kontrollzellen, denen keine Modulatoren zugesetzt worden waren, unbeeinträchtigt blieb. Die Zugabe von Clotrimazol und 1-EBIO in der angegebenen Konzentration führte zur vollständigen Inhibition; dagegen wurde bei Zugabe von 200 nM Charybdotoxin ein nur verlangsamtes Wachstum festgestellt.

Die Ergebnisse des Versuchs zeigen, daß Modulatoren des hIK1 Kanals beide Parameter der Keratinozytenaktivität, die Proliferation und die Differenzierung, gleichzeitig beeinflussen. Dies gilt interessanterweise in gleicher Weise für Aktivatoren als auch Inhibitoren: die Zugabe führt zur Inhibition von Proliferation und Differenzierung. Dies kann durch die bekannte Rolle des Ionenkanals im Zellzyklus erklärt werden: vermutlich sind sowohl Phasen der verstärkten Öffnung als auch des Schliessens für den Durchlauf eines Zellzyklus erforderlich. Somit würde sowohl die dauerhafte Aktivierung als auch dauerhafte Inhibierung der Ionenkanalaktivität zu einer Inhibition der Proliferation führen, was den experimentellen Beobachtungen entspricht. Entsprechend kann eine vergleichbare Wirkung auf die Differenzierung abgeleitet werden. Durch das Auffinden von Modulatoren, die sowohl die Differenzierung als auch die Proliferation der Keratinozyten inhibieren, ergibt sich erstmals die Möglichkeit zur Entwicklung völlig neuartige Wirkstoffe. Somit können sich durch die Modulation verschiedener Aktivitäten von hIK1 für die Behandlung und/oder Prävention von Krankheiten, die mit gestörter Keratinozytenaktivität im Zusammenhang stehen spezielle und völlig neue Therapiemöglichkeiten ergeben. So ist beispielsweise die Modulation der Aktivität von hIK1 gerade für die Behandlung von Psoriasis besonders vorteilhaft, wo die Ursache der Erkrankung in einer Kombination aus Zellhyperproliferation und reduzierter Apoptose differenzierer Zellen begründet ist. Insbesondere bevorzugt sind Derivate der Modulatoren, die in geringerer Konzentration eingesetzt werden können, beispielsweise DCEBIO (5,6-Dichloro-1-ethyl-1,3-dihydro-2H-benzimidazol-2-on) als dichloro-Derivat von 1-EBIO. Entsprechend ist zur Behandlung von Wundheilungsstörungen, bei denen eine Stimulation der Keratinozytenproliferation anzustreben ist, eine Erhöhung der Menge des IK Kanals bevorzugt. Dies kann beispielsweise gentherapeutisch unter Verwendung eines gentherapeutischen Vektors enthaltend eine Nukleinsäure kodierend für einen erfindungsgemäß verwendbaren IK Kanal erfolgen.

Die Prioritätsanmeldung DE 100 65 475.4 wurde am 28. Dezember 2000, und die Prioritätsanmeldung US 60/277,453 wurde am 20. März 2000 angemeldet.

Tabelle 1

| Nr. | NAME | Organismus | PROTEIN | SEQ ID Nr. | cDNA | SEQ ID Nr. |
|---|---|---|---|---|---|---|
| 1. | hIK1 | Homo Sapiens | trEMBL: O15554 | 3 | EMBL: AF000972 | 1 |
| 2. | mIK1 | Mus musculus | trEMBL: O89109 | 4 | EMBL: AF042487 | 2 |

Tabelle 2

| Wundheilungszustand | Rel. mIK1 cDNA Menge |
|---|---|
| Intakte Haut<br>Kontrolltiere | 1,00 |
| Wunde<br>Kontrolltiere | 3,00 |
| Intakte Haut<br>Dexamethason-behandelte Tiere | 1,20 |
| Wunde<br>Dexamethason-behandelte Tiere | 2,68 |
| Intakte Haut<br>Junge Tiere | 0,52 |
| Wunde<br>Junge Tiere | 2,06 |
| Intakte Haut<br>Alte Tiere | 1,45 |
| Wunde<br>Alte Tiere | 1,55 |
| Intakte Haut<br>Diabetes Kontrolltiere (C57B/Ks) | 1,00 |
| Wunde<br>Diabetes Kontrolltiere (C57B/Ks) | 2,45 |
| Intakte Haut<br>Diabetes Tiere (C57B/Ks-*db*/*db*/Ola) | 0,41 |
| Wunde<br>Diabetes Tiere (C57B/Ks-*db*/*db*/Ola) | 2,04 |

Tabelle 3

| Zeitpunkt nach Wundsetzung | Rel. mIK1 cDNA Menge |
|---|---|
| Intakte Haut | 1,00 |
| 1 h | 1,03 |
| 7 h | 1,71 |
| 15 h | 2,24 |
| 24 h | 1,86 |
| 3 d | 2,71 |
| 5 d | 2,59 |

Tabelle 3 (fortgesetzt)

| Zeitpunkt nach Wundsetzung | Rel. mIK1 cDNA Menge |
|---|---|
| 7 d | 2,43 |
| 14 d | 2,24 |

Tabelle 4

| Biopsie | Rel. hIK1 cDNA Menge |
|---|---|
| Patienten-Pool 1: Intakte Haut Gesunde Patienten | **1,00** |
| Patienten-Pool 2: Tag 1-Wunde Gesunde Patienten | **1,32** |
| Patienten-Pool 3: Tag 5-Wunde Gesunde Patienten | **0,64** |
| Patienten-Pool 4: Intakte Haut Ulkus-Patienten | **0,83** |
| Patienten-Pool 4: Wundrand Ulkus Patienten | **0,42** |
| Patienten-Pool 4: Wundgrund Ulkus Patienten | **0,81** |

Tabelle 5

| Organ | Rel. hIK cDNA Menge |
|---|---|
| Haut | 0,54 |
| Niere | 0,41 |
| Leber | 0,23 |
| Gehirn | 0,39 |
| Herz | 0,36 |
| Milz | 0,66 |
| Skelettmuskel | 0,28 |
| Dünndarm | 0,56 |
| Lunge | 0,58 |
| Fettgewebe | 0,58 |

## Patentansprüche

**1.** Verwendung von Chlorzoxazon zur Herstellung eines Diagnostikums zur Diagnose oder eines Arzneimittels zur Prävention und/oder Behandlung von Psoriasis.

## Claims

**1.** Use of chlorzoxazone for producing a diagnostic agent for diagnosing, or a pharmaceutical for preventing and/or treating, psoriasis.

## Revendications

1. Utilisation de chlorzoxazone pour la fabrication d'un moyen de diagnostic destiné à déceler le Psoriasis ou à un médicament pour la prévention et/ou le traitement du Psoriasis.

Figur 1

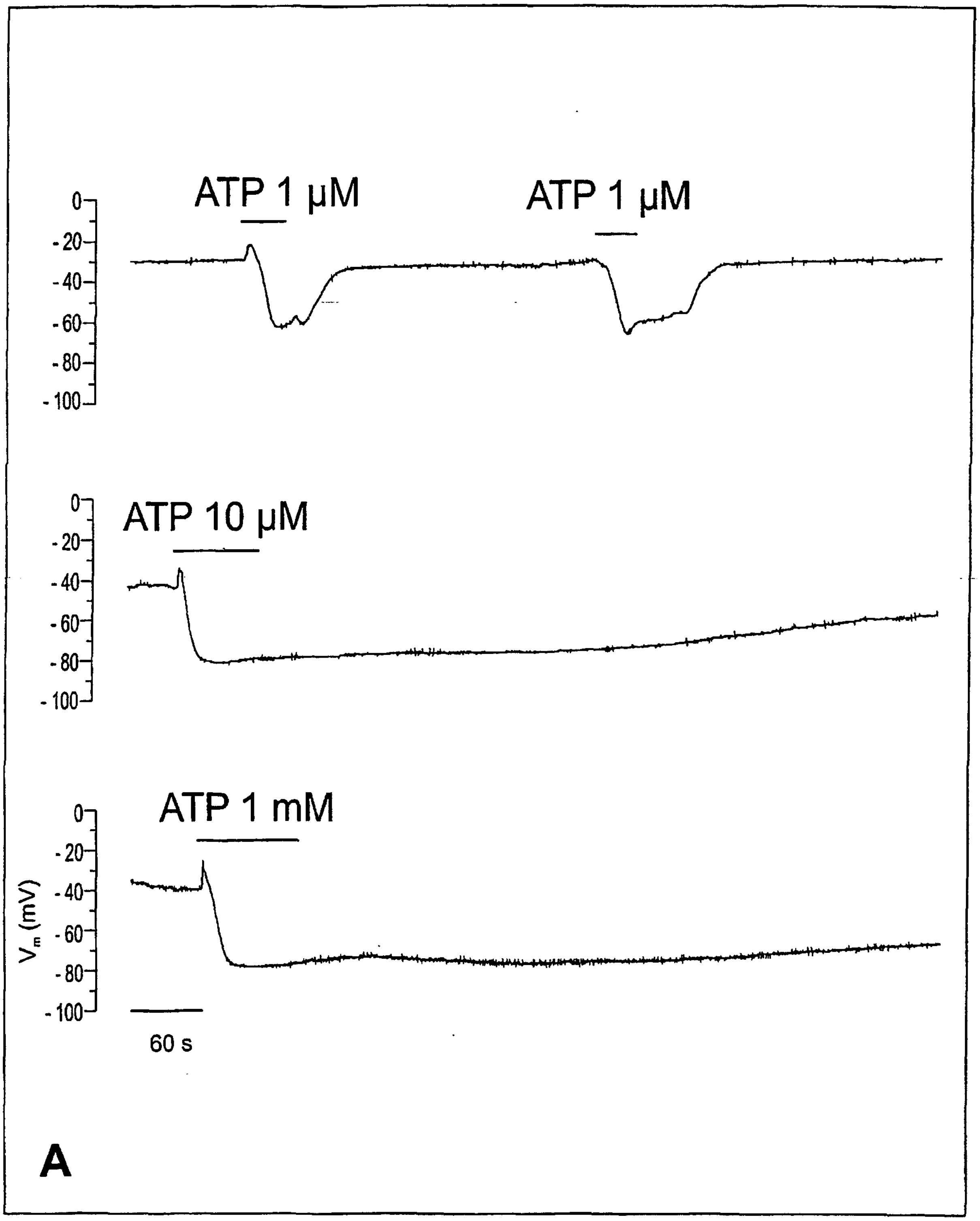
ATP 1 µM
ATP 1 µM
0
-20
-40
-60
-80
-100
ATP 10 µM
0
-20
-40
-60
-80
-100
ATP 1 mM
0
-20
-40
-60
-80
-100
$V_m$ (mV)
60 s
A

Figur 1

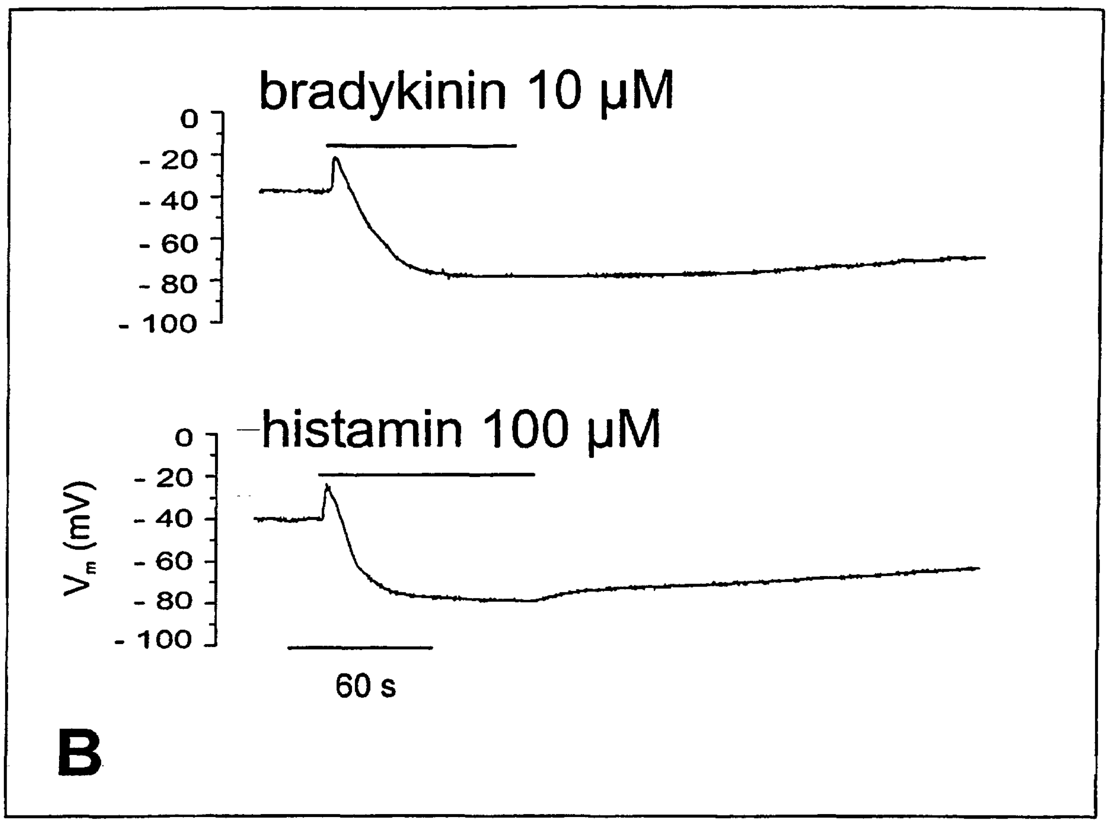
bradykinin 10 µM
0
- 20
- 40
- 60
- 80
- 100
histamin 100 µM
$V_m$ (mV)
0
- 20
- 40
- 60
- 80
- 100
60 s
B

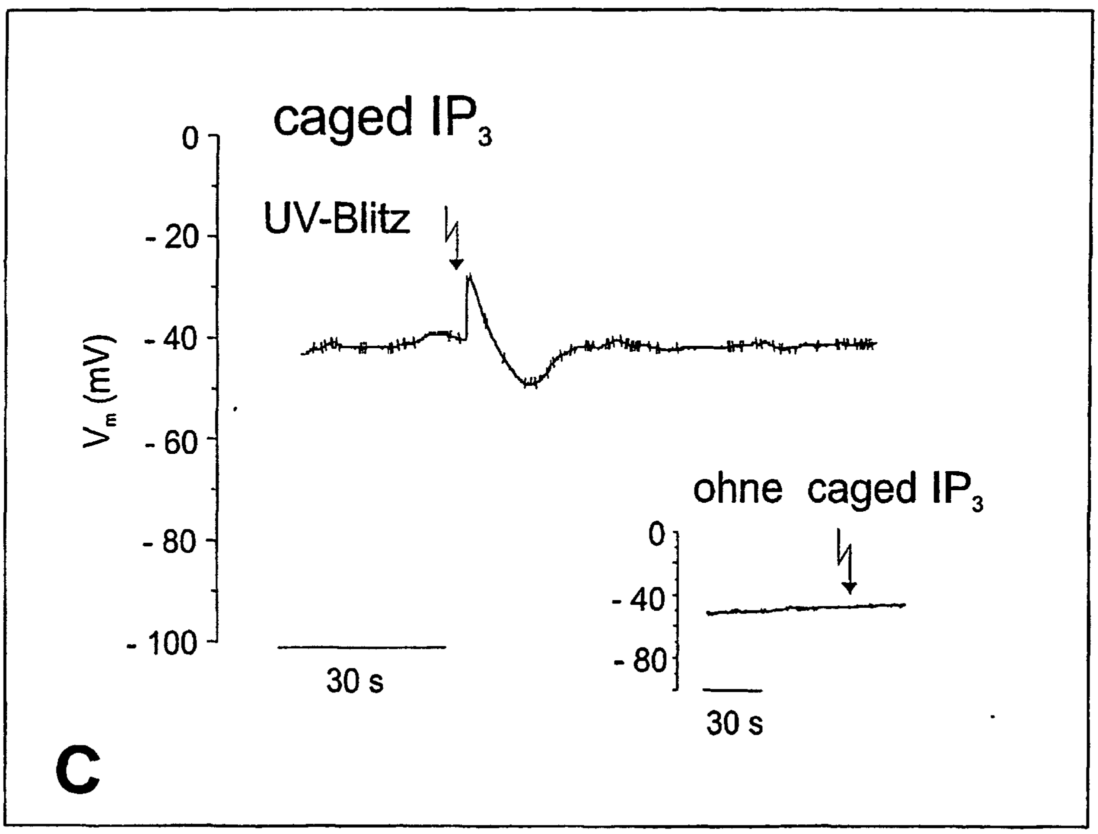
caged $IP_3$
UV-Blitz
$V_m$ (mV)
0
- 20
- 40
- 60
- 80
- 100
30 s
ohne caged $IP_3$
0
- 40
- 80
30 s
C

Figur 2

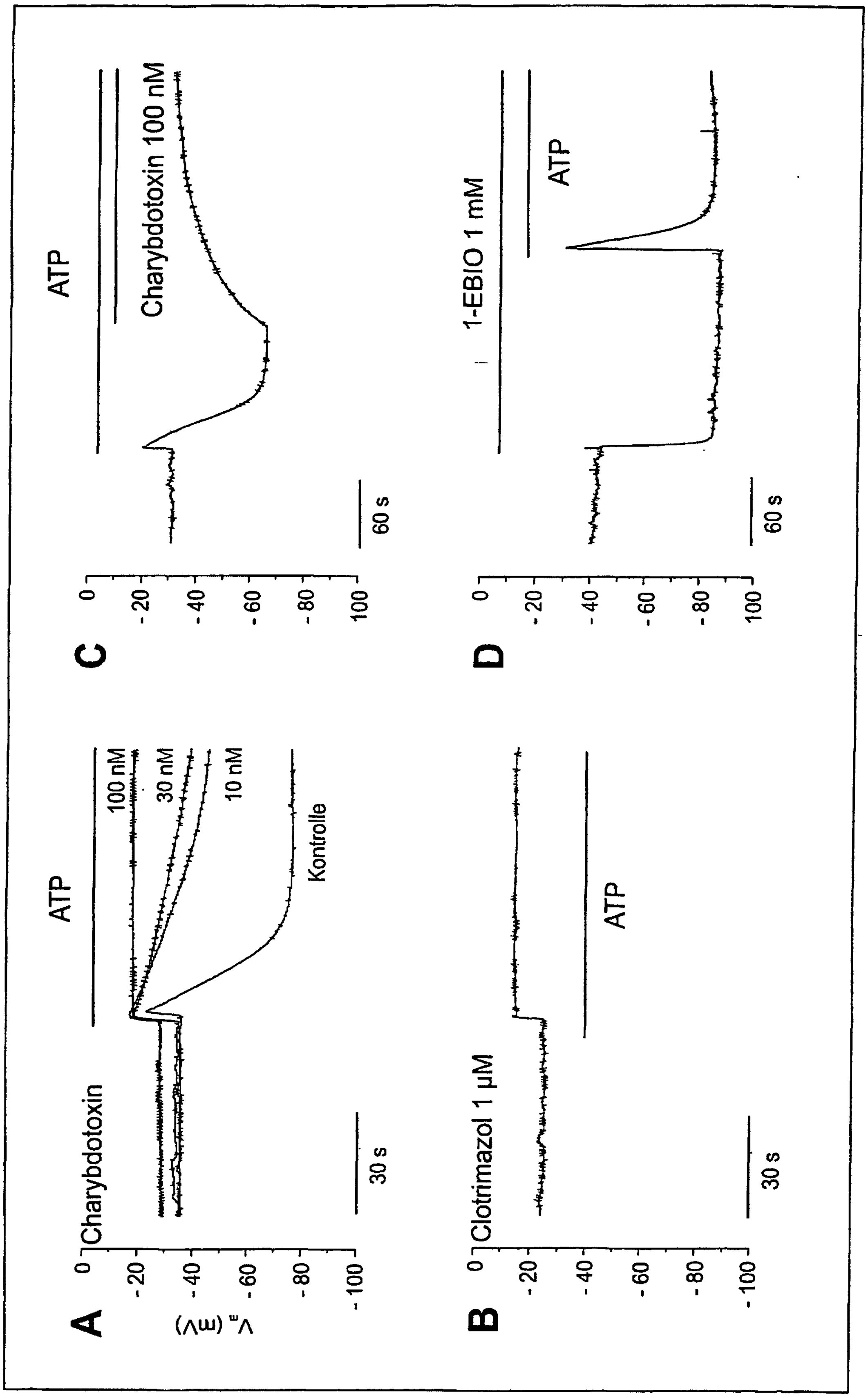
A
B
C
D
$V_m$ (mV)
Charybdotoxin
ATP
100 nM
30 nM
10 nM
Kontrolle
30 s
Clotrimazol 1 µM
ATP
30 s
ATP
Charybdotoxin 100 nM
60 s
1-EBIO 1 mM
ATP
60 s
0
-20
-40
-60
-80
-100

# Figur 3

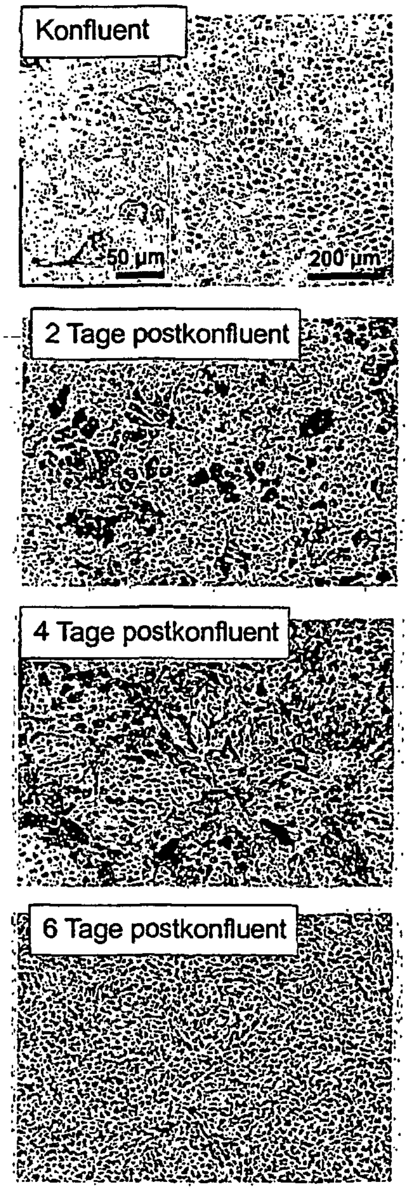
Konfluent
50 µm
200 µm
2 Tage postkonfluent
4 Tage postkonfluent
6 Tage postkonfluent

**Figur 4**

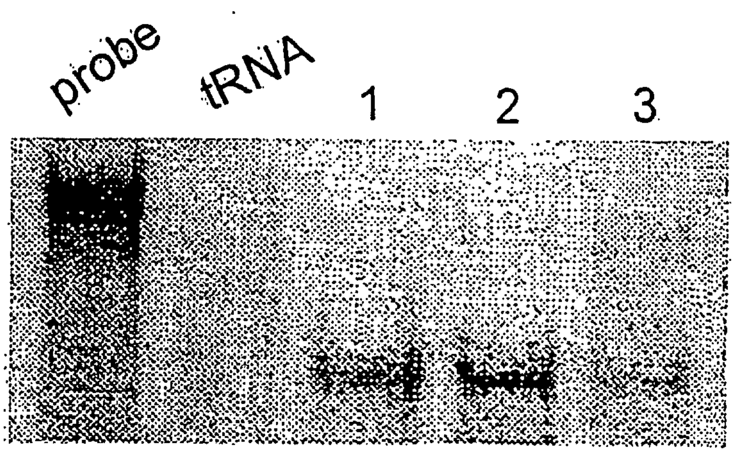

Figur 5

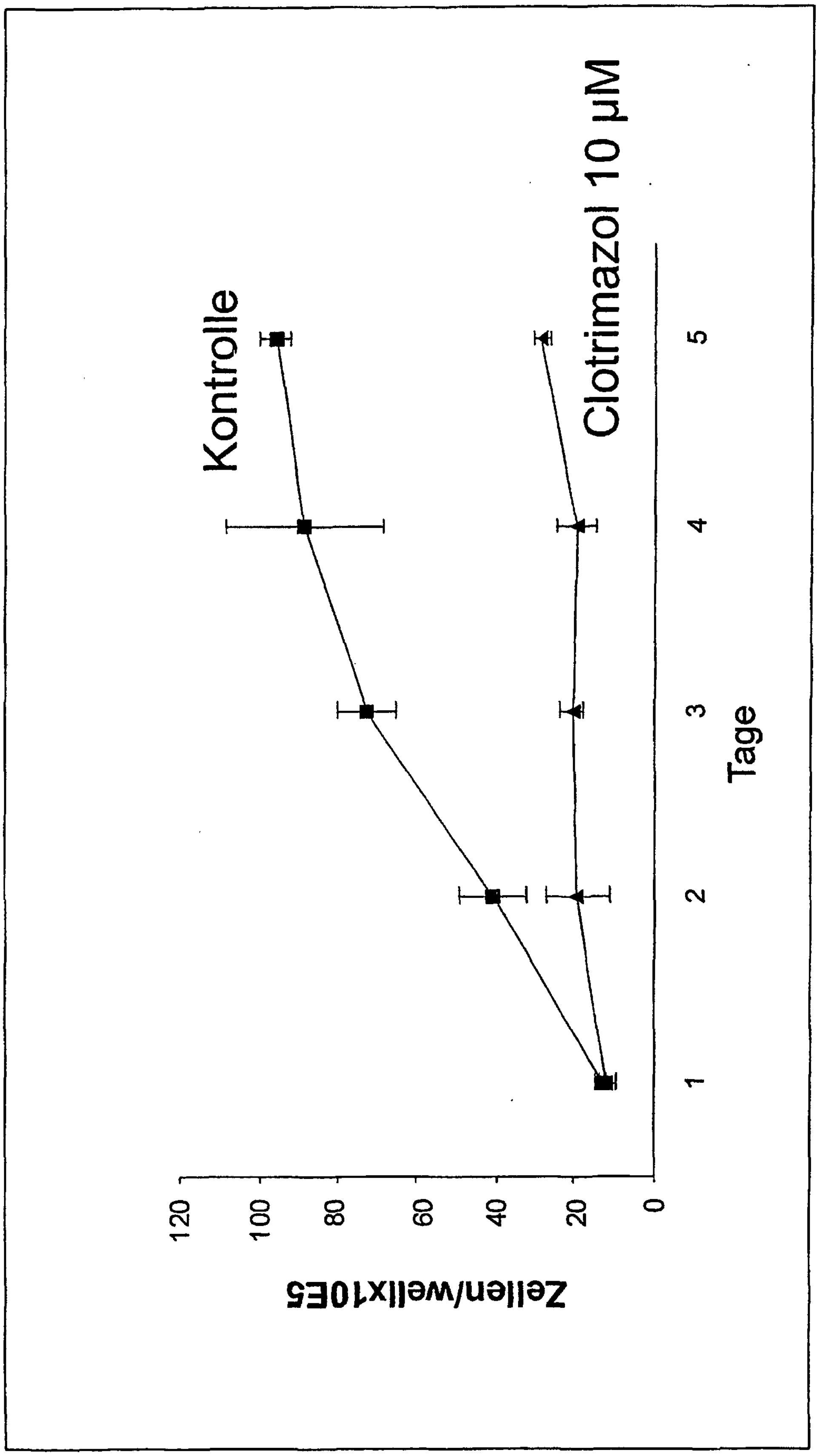
Kontrolle
Clotrimazol 10 µM
Zellen/wellx10E5
0
20
40
60
80
100
120
1
2
3
4
5
Tage

Figur 5

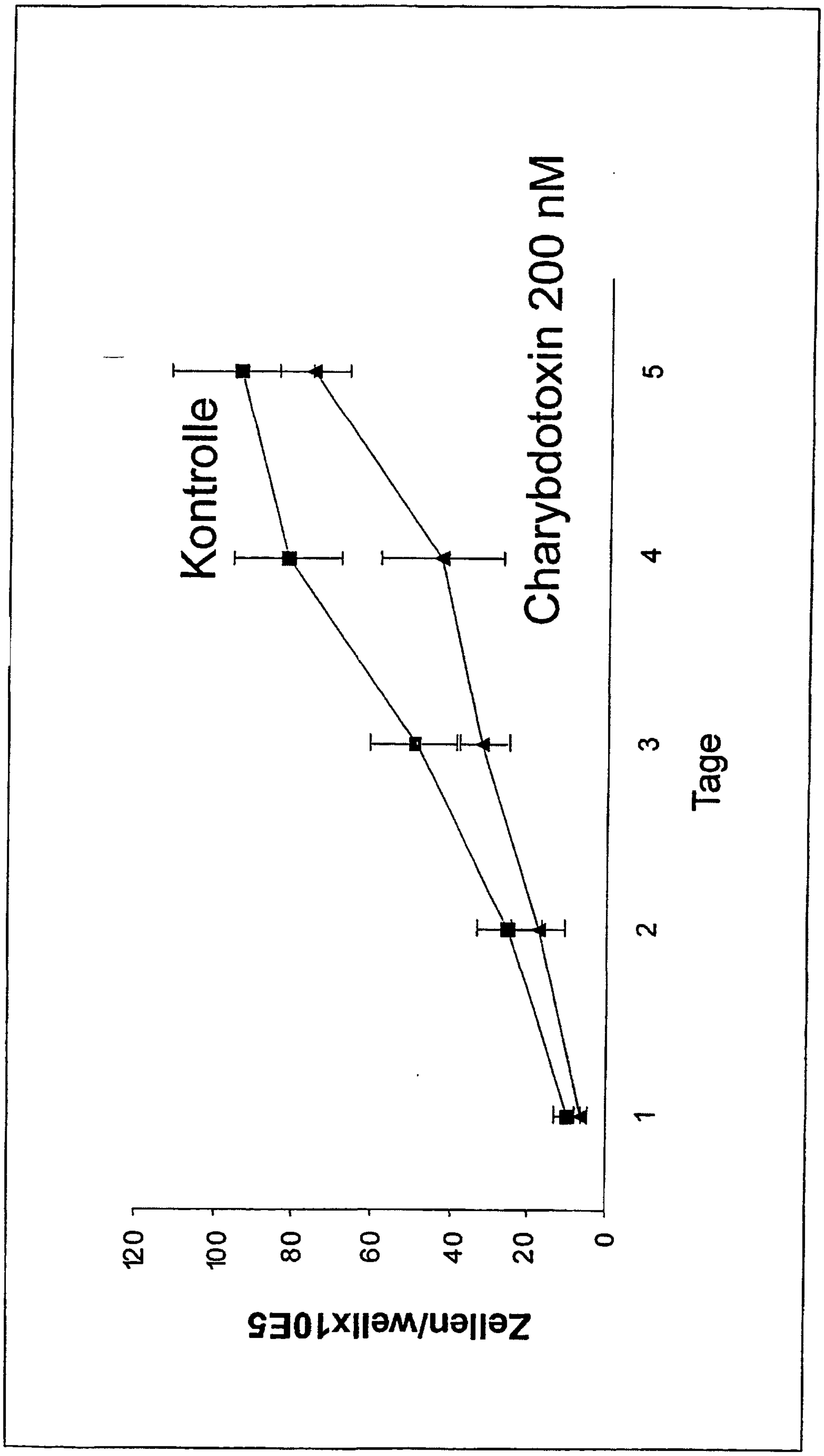

Kontrolle
Charybdotoxin 200 nM
Zellen/wellx10E5
0
20
40
60
80
100
120
Tage
1
2
3
4
5

Figur 5

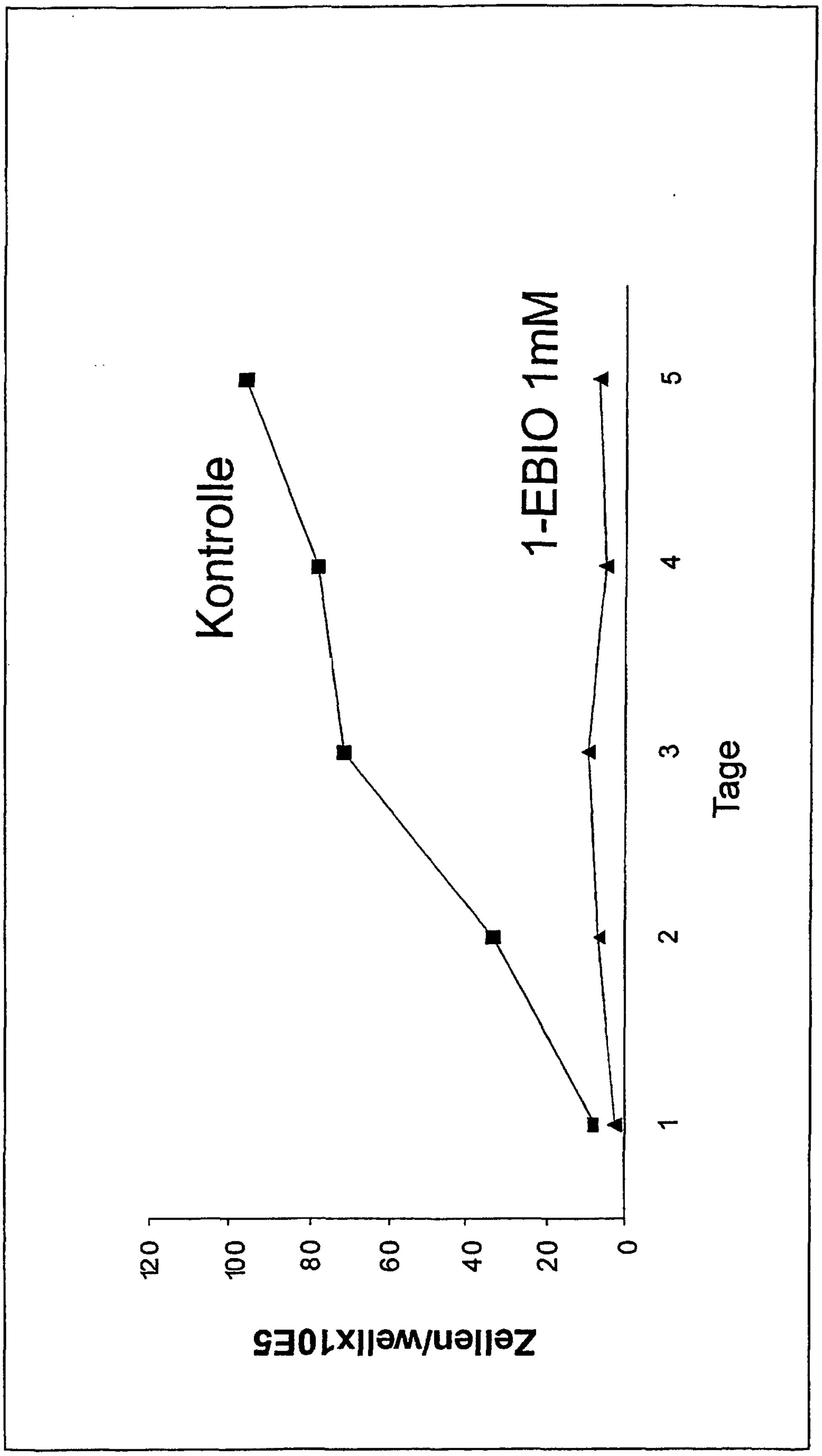

Zellen/wellx10E5
120
100
80
60
40
20
0
1
2
3
4
5
Tage
Kontrolle
1-EBIO 1mM

Figur 6

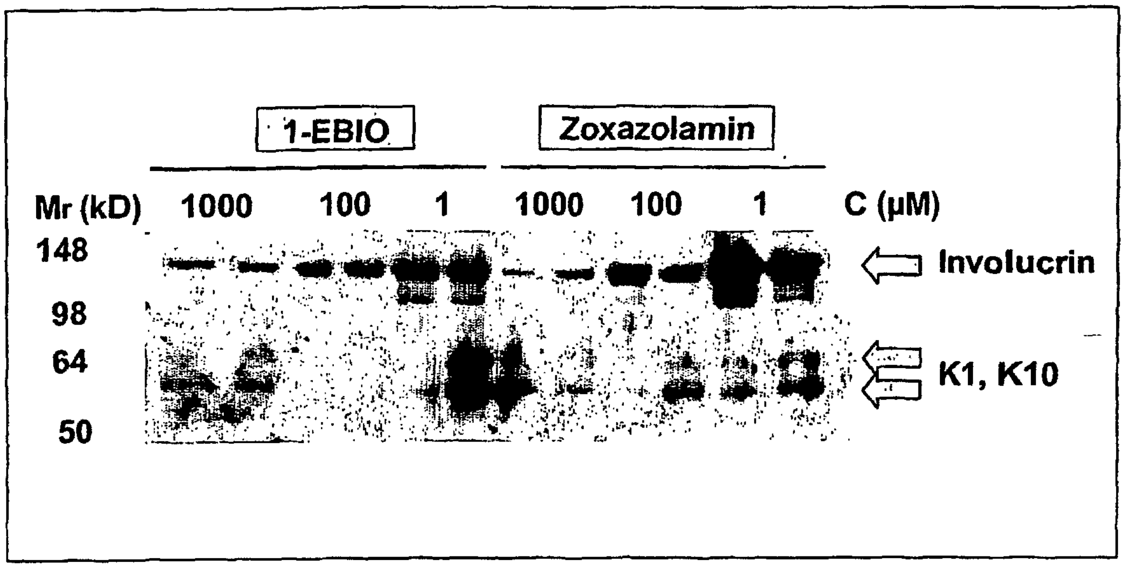
1-EBIO
Zoxazolamin
Mr (kD)
1000
100
1
1000
100
1
C (µM)
148
98
64
50
Involucrin
K1, K10

Figur 7

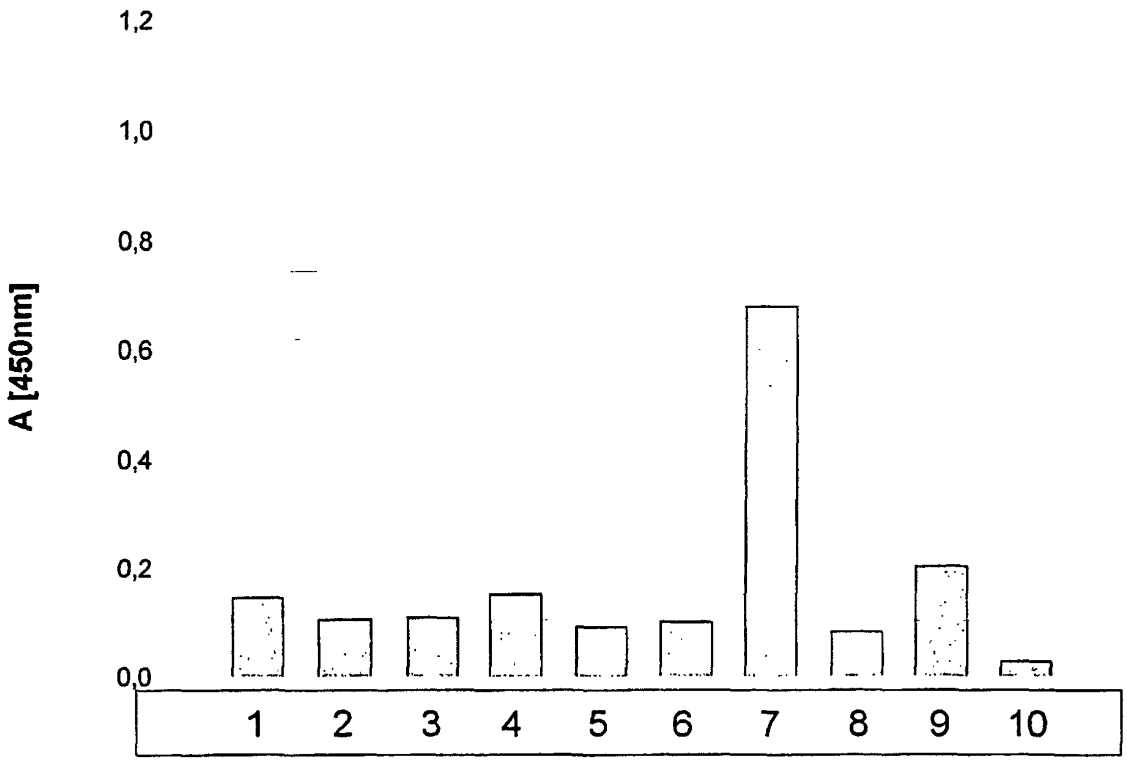


Spur 1: 1000 µM 1-EBIO

Spur 2: 100 µM 1-EBIO

Spur 3: 10 µM 1-EBIO

Spur 4: 1 µM 1-EBIO

Spur 5: 0,1 µM 1-EBIO

Spur 6: 0,01 µM 1-EBIO

Spur 7: KGM-10% FCS

Spur 8: KGM DMSO

Spur 9: KGM

Spur 10: 1000 µM Zoxazolamin

Figur 8

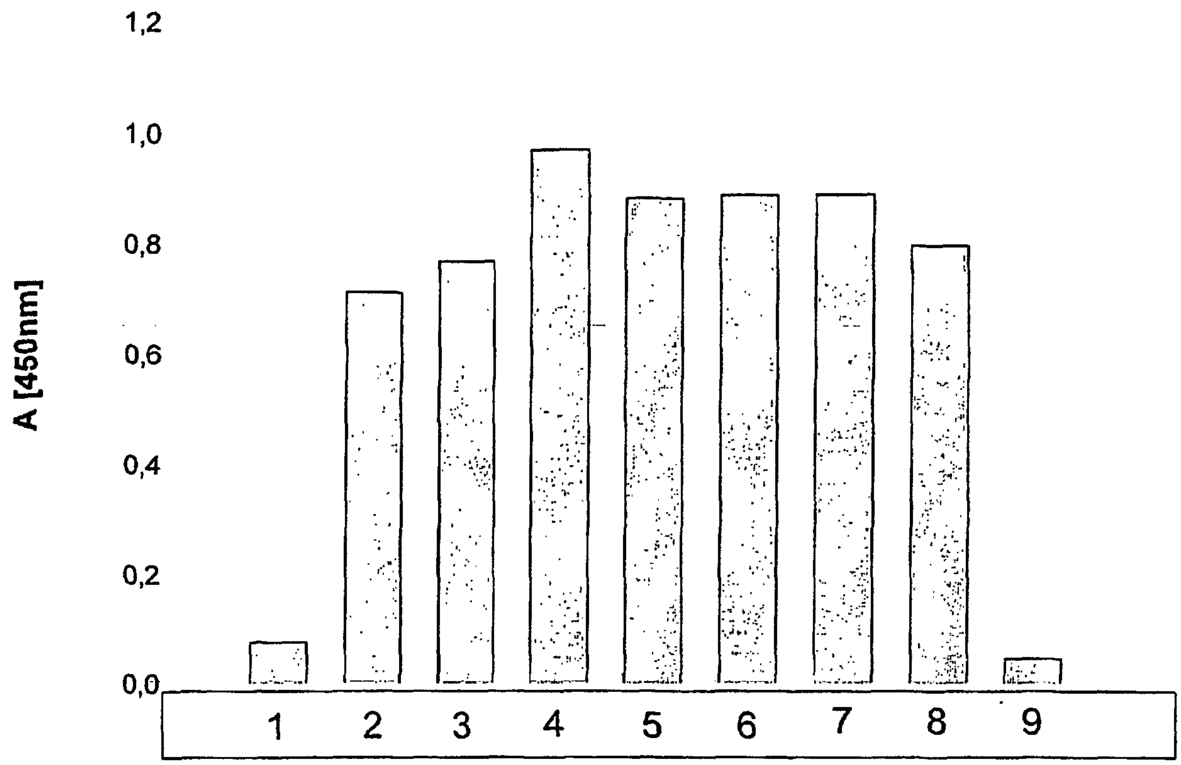


Spur 1: 1000 µM 1-EBIO

Spur 2: 100 µM 1-EBIO

Spur 3: 10 µM 1-EBIO

Spur 4: 1 µM 1-EBIO

Spur 5: 0,1 µM 1-EBIO

Spur 6: 0,01 µM 1-EBIO

Spur 7: DMEM-10% FCS

Spur 8: DMSO

Spur 9: 1000 µM Zoxazolamin

**SEQUENZPROTOKOLL**

<110> Switch Biotech AG

<120> Verwendung von "intermediate-conductance" Kaliumkanälen und Modulatoren zur Diagnose und Behandlung von Krankheiten mit gestörter Keratinozytenaktivität

<130> S35154PC

<150> DE 10065475.4
<151> 2000-12-28

<150> US 60/277,453
<151> 2000-03-20

<160> 13

<170> Word 97, PC-DOS/MS-DOS

<210> 1
<211> 1284
<212> DNA
<213> Homo sapiens

<220>
<223> hIK1

<300>
<308> EMBL-Datenbank: AF000972

<400> 1

```
atgggcgggg atctggtgct tggcctgggg gccttgagac gccgaaagcg cttgctggag   60
caggagaagt ctctggccgg ctgggcactg gtgctggcag gaactggcat tggactcatg  120
gtgctgcatg cagagatgct gtggttcggg gggtgctcgt gggcgctcta cctgttcctg  180
gttaaatgca cgatcagcat ttccaccttc ttactcctct gcctcatcgt ggcctttcat  240
gccaaagagg tccagctgtt catgaccgac aacgggctgc gggactggcg cgtggcgctg  300
accgggcggc aggcggcgca gatcgtgctg gagctggtgg tgtgtgggct gcacccggcg  360
cccgtgcggg gcccgccgtg cgtgcaggat ttaggggcgc cgctgacctc cccgcagccc  420
tggccgggat tcctgggcca aggggaagcg ctgctgtccc tggccatgct gctgcgtctc  480
tacctggtgc cccgcgccgt gctcctgcgc agcggcgtcc tgctcaacgc ttcctaccgc  540
agcatcggcg ctctcaatca agtccgcttc cgccactggt tcgtggccaa gctttacatg  600
aacacgcacc ctggccgcct gctgctcggc ctcacgcttg gcctctggct gaccaccgcc  660
tgggtgctgt ccgtggccga gaggcaggct gttaatgcca ctgggcacct ttcagacaca  720
ctttggctga tccccatcac attcctgacc atcggctatg gtgacgtggt gccgggcacc  780
atgtggggca agatcgtctg cctgtgcact ggagtcatgg gtgtctgctg cacagccctg  840
ctggtggccg tggtggcccg gaagctggag tttaacaagg cagagaagca cgtgcacaac  900
```

```
ttcatgatgg atatccagta taccaaagag atgaaggagt ccgctgcccg agtgctacaa   960
gaagcctgga tgttctacaa acatactcgc aggaaggagt ctcatgctgc ccgcaggcat  1020
cagcgcaagc tgctggccgc catcaacgcg ttccgccagg tgcggctgaa acaccggaag  1080
ctccgggaac aagtgaactc catggtggac atctccaaga tgcacatgat cctgtatgac  1140
ctgcagcaga atctgagcag ctcacaccgg gccctggaga aacagattga cacgctggcg  1200
gggaagctgg atgccctgac tgagctgctt agcactgccc tggggccgag gcagcttcca  1260
gaacccagcc agcagtccaa gtag  1284
```

<210> 2
<211> 1469
<212> DNA
<213> Mus musculus

<220>
<223> mIK1

<300>
<308> EMBL-Datenbank: AF042487

<400> 2

```
ctgggcagga agctggctga gccccaagac ctcaggggcc atgggcgggg agctggtgac   60
tggcctgggg gccctgagac ggagaaagcg cctgctggag caggagaaga gggtggccgg  120
ctgggcgttg gtgctggcgg gaactggcat cggactcatg gttctgcacg ctgagatgtt  180
gtggttcctg ggctgcaagt gggtgctgta cctgctcctg gttaagtgtt tgatcaccct  240
gtccactgcc ttcctccttt gtcttattgt ggtcttccat gccaaggagg tccagctgtt  300
catgactgac aacgggctcc gggactggcg cgtggcgctg acccggcggc aggtggcgca  360
gatcctgctg gagctgttgg tgtgcggggt gcacccggtg cccctacgga gcccgcactg  420
cgccctggcg ggggaggcca ccgacgcgca gccctggccg ggtttcctgg gcgaaggcga  480
ggcgttgctg tccctggcca tgctcctgcg tctctacctg gtgccccgcg cggtgctgct  540
gcgcagcggg gtcctgctca acgcgtccta ccgcagcatc ggggcgctca accaagtccg  600
cttccgccac tggttcgtgg ccaagctgta catgaacacg cacccgggtc gcctgctgct  660
gggcctcacg ctgggtctct ggctcaccac agcttgggtg ctgtctgtgg ctgagaggca  720
ggctgtcaat gccacggggc acctcacaga cacactgtgg ctgattccga tcacattcct  780
gaccattggc tatggggacg tggtacctgg caccatgtgg ggcaagattg tctgcctgtg  840
caccggagtc atgggggtct gctgcacagc tctcctggtg gctgtggtgg ctcggaagct  900
ggagttcaac aaggcggaga aacacgtgca caacttcatg atggacatcc attatgccaa  960
agagatgaag gagtcagcgg cgcggctgct gcaggaagcc tggatgtact acaagcacac 1020
tcgaaggaag gactcccggg ctgcccgcag acatcagcgc aagatgctgg ccgccatcca 1080
cacgttccgc caggtacggc tgaaacaccg gaagctccgg gaacaagtga attccatggt 1140
ggacatctcc aagatgcaca tgatcctgtg cgacctgcag ctgggtctca gctcctcgca 1200
ccgtgccctg gagaagagaa tcgacggtct ggcaggaaag ctggatgccc tgacagagct 1260
gctcggcact gctctgcagc aacagcagct accagaaccc agtcaggagg ccacatagct 1320
ccacatgaac tcacagaaga accaggctaa gtacccaagg accgagctca aggacatgct 1380
ccctgccaat tccgaccaag ccccacgata aatcacctca agatgccagg acccaagtgg 1440
atccacgttg aggtgcatgg actctggtg  1469
```

<210> 3
<211> 427
<212> PRT
<213> Homo sapiens

<220>
<223> hIK1

<300>
<308> trEMBL-Datenbank: 015554

<400> 3

| | | | | | | | | | | |
|---|---|---|---|---|---|---|---|---|---|---|
| Met | Gly | Gly | Asp | Leu | Val | Leu | Gly | Leu | Gly | 10 |
| Ala | Leu | Arg | Arg | Arg | Lys | Arg | Leu | Leu | Glu | 20 |
| Gln | Glu | Lys | Ser | Leu | Ala | Gly | Trp | Ala | Leu | 30 |
| Val | Leu | Ala | Gly | Thr | Gly | Ile | Gly | Leu | Met | 40 |
| Val | Leu | His | Ala | Glu | Met | Leu | Trp | Phe | Gly | 50 |
| Gly | Cys | Ser | Trp | Ala | Leu | Tyr | Leu | Phe | Leu | 60 |
| Val | Lys | Cys | Thr | Ile | Ser | Ile | Ser | Thr | Phe | 70 |
| Leu | Leu | Leu | Cys | Leu | Ile | Val | Ala | Phe | His | 80 |
| Ala | Lys | Glu | Val | Gln | Leu | Phe | Met | Thr | Asp | 90 |
| Asn | Gly | Leu | Arg | Asp | Trp | Arg | Val | Ala | Leu | 100 |
| Thr | Gly | Arg | Gln | Ala | Ala | Gln | Ile | Val | Leu | 110 |
| Glu | Leu | Val | Val | Cys | Gly | Leu | His | Pro | Ala | 120 |
| Pro | Val | Arg | Gly | Pro | Pro | Cys | Val | Gln | Asp | 130 |
| Leu | Gly | Ala | Pro | Leu | Thr | Ser | Pro | Gln | Pro | 140 |
| Trp | Pro | Gly | Phe | Leu | Gly | Gln | Gly | Glu | Ala | 150 |
| Leu | Leu | Ser | Leu | Ala | Met | Leu | Leu | Arg | Leu | 160 |
| Tyr | Leu | Val | Pro | Arg | Ala | Val | Leu | Leu | Arg | 170 |
| Ser | Gly | Val | Leu | Leu | Asn | Ala | Ser | Tyr | Arg | 180 |
| Ser | Ile | Gly | Ala | Leu | Asn | Gln | Val | Arg | Phe | 190 |
| Arg | His | Trp | Phe | Val | Ala | Lys | Leu | Tyr | Met | 200 |
| Asn | Thr | His | Pro | Gly | Arg | Leu | Leu | Leu | Gly | 210 |
| Leu | Thr | Leu | Gly | Leu | Trp | Leu | Thr | Thr | Ala | 220 |
| Trp | Val | Leu | Ser | Val | Ala | Glu | Arg | Gln | Ala | 230 |
| Val | Asn | Ala | Thr | Gly | His | Leu | Ser | Asp | Thr | 240 |
| Leu | Trp | Leu | Ile | Pro | Ile | Thr | Phe | Leu | Thr | 250 |
| Ile | Gly | Tyr | Gly | Asp | Val | Val | Pro | Gly | Thr | 260 |
| Met | Trp | Gly | Lys | Ile | Val | Cys | Leu | Cys | Thr | 270 |
| Gly | Val | Met | Gly | Val | Cys | Cys | Thr | Ala | Leu | 280 |
| Leu | Val | Ala | Val | Val | Ala | Arg | Lys | Leu | Glu | 290 |
| Phe | Asn | Lys | Ala | Glu | Lys | His | Val | His | Asn | 300 |
| Phe | Met | Met | Asp | Ile | Gln | Tyr | Thr | Lys | Glu | 310 |
| Met | Lys | Glu | Ser | Ala | Ala | Arg | Val | Leu | Gln | 320 |
| Glu | Ala | Trp | Met | Phe | Tyr | Lys | His | Thr | Arg | 330 |
| Arg | Lys | Glu | Ser | His | Ala | Ala | Arg | Arg | His | 340 |
| Gln | Arg | Lys | Leu | Leu | Ala | Ala | Ile | Asn | Ala | 350 |
| Phe | Arg | Gln | Val | Arg | Leu | Lys | His | Arg | Lys | 360 |

| | | | | | | | | | | |
|---|---|---|---|---|---|---|---|---|---|---|
| Leu | Arg | Glu | Gln | Val | Asn | Ser | Met | Val | Asp | 370 |
| Ile | Ser | Lys | Met | His | Met | Ile | Leu | Tyr | Asp | 380 |
| Leu | Gln | Gln | Asn | Leu | Ser | Ser | Ser | His | Arg | 390 |
| Ala | Leu | Glu | Lys | Gln | Ile | Asp | Thr | Leu | Ala | 400 |
| Gly | Lys | Leu | Asp | Ala | Leu | Thr | Glu | Leu | Leu | 410 |
| Ser | Thr | Ala | Leu | Gly | Pro | Arg | Gln | Leu | Pro | 420 |
| Glu | Pro | Ser | Gln | Gln | Ser | Lys | 427 | | | |

<210> 4
<211> 425

<212> PRT
<213> Mus musculus

<220>
<223> mIK1

<300>
<308> trEMBL-Datenbank: O89109

<400> 4

| | | | | | | | | | | |
|---|---|---|---|---|---|---|---|---|---|---|
| Met | Gly | Gly | Glu | Leu | Val | Thr | Gly | Leu | Gly | 10 |
| Ala | Leu | Arg | Arg | Arg | Lys | Arg | Leu | Leu | Glu | 20 |
| Gln | Glu | Lys | Arg | Val | Ala | Gly | Trp | Ala | Leu | 30 |
| Val | Leu | Ala | Gly | Thr | Gly | Ile | Gly | Leu | Met | 40 |
| Val | Leu | His | Ala | Glu | Met | Leu | Trp | Phe | Leu | 50 |
| Gly | Cys | Lys | Trp | Val | Leu | Tyr | Leu | Leu | Leu | 60 |
| Val | Lys | Cys | Leu | Ile | Thr | Leu | Ser | Thr | Ala | 70 |
| Phe | Leu | Leu | Cys | Leu | Ile | Val | Val | Phe | His | 80 |
| Ala | Lys | Glu | Val | Gln | Leu | Phe | Met | Thr | Asp | 90 |
| Asn | Gly | Leu | Arg | Asp | Trp | Arg | Val | Ala | Leu | 100 |
| Thr | Arg | Arg | Gln | Val | Ala | Gln | Ile | Leu | Leu | 110 |
| Glu | Leu | Leu | Val | Cys | Gly | Val | His | Pro | Val | 120 |
| Pro | Leu | Arg | Ser | Pro | His | Cys | Ala | Leu | Ala | 130 |
| Gly | Glu | Ala | Thr | Asp | Ala | Gln | Pro | Trp | Pro | 140 |
| Gly | Phe | Leu | Gly | Glu | Gly | Glu | Ala | Leu | Leu | 150 |
| Ser | Leu | Ala | Met | Leu | Leu | Arg | Leu | Tyr | Leu | 160 |
| Val | Pro | Arg | Ala | Val | Leu | Leu | Arg | Ser | Gly | 170 |
| Val | Leu | Leu | Asn | Ala | Ser | Tyr | Arg | Ser | Ile | 180 |
| Gly | Ala | Leu | Asn | Gln | Val | Arg | Phe | Arg | His | 190 |
| Trp | Phe | Val | Ala | Lys | Leu | Tyr | Met | Asn | Thr | 200 |
| His | Pro | Gly | Arg | Leu | Leu | Leu | Gly | Leu | Thr | 210 |
| Leu | Gly | Leu | Trp | Leu | Thr | Thr | Ala | Trp | Val | 220 |
| Leu | Ser | Val | Ala | Glu | Arg | Gln | Ala | Val | Asn | 230 |
| Ala | Thr | Gly | His | Leu | Thr | Asp | Thr | Leu | Trp | 240 |
| Leu | Ile | Pro | Ile | Thr | Phe | Leu | Thr | Ile | Gly | 250 |
| Tyr | Gly | Asp | Val | Val | Pro | Gly | Thr | Met | Trp | 260 |
| Gly | Lys | Ile | Val | Cys | Leu | Cys | Thr | Gly | Val | 270 |

| | | | | | | | | | | |
|---|---|---|---|---|---|---|---|---|---|---|
| Met | Gly | Val | Cys | Cys | Thr | Ala | Leu | Leu | Val | 280 |
| Ala | Val | Val | Ala | Arg | Lys | Leu | Glu | Phe | Asn | 290 |
| Lys | Ala | Glu | Lys | His | Val | His | Asn | Phe | Met | 300 |
| Met | Asp | Ile | His | Tyr | Ala | Lys | Glu | Met | Lys | 310 |
| Glu | Ser | Ala | Ala | Arg | Leu | Leu | Gln | Glu | Ala | 320 |
| Trp | Met | Tyr | Tyr | Lys | His | Thr | Arg | Arg | Lys | 330 |
| Asp | Ser | Arg | Ala | Ala | Arg | Arg | His | Gln | Arg | 340 |
| Lys | Met | Leu | Ala | Ala | Ile | His | Thr | Phe | Arg | 350 |
| Gln | Val | Arg | Leu | Lys | His | Arg | Lys | Leu | Arg | 360 |
| Glu | Gln | Val | Asn | Ser | Met | Val | Asp | Ile | Ser | 370 |
| Lys | Met | His | Met | Ile | Leu | Cys | Asp | Leu | Gln | 380 |
| Leu | Gly | Leu | Ser | Ser | Ser | His | Arg | Ala | Leu | 390 |
| Glu | Lys | Arg | Ile | Asp | Gly | Leu | Ala | Gly | Lys | 400 |
| Leu | Asp | Ala | Leu | Thr | Glu | Leu | Leu | Gly | Thr | 410 |
| Ala | Leu | Gln | Gln | Gln | Gln | Leu | Pro | Glu | Pro | 420 |
| Ser | Gln | Glu | Ala | Thr | 425 | | | | | |

<210> 5
<211> 265
<212> PRT
<213> Homo sapiens

<220>
<223> hIK1 Riboprobe

<400> 5

cattcctgac catcggctat ggtgacgtgg tgccgggcac catgtggggc aagatcgtct 60
gcctgtgcac tggagtcatg ggtgtctgct gcacagccct gctggtggcc gtggtggccc 120
ggaagctgga gtttaacaag gcagagaagc acgtgcacaa cttcatgatg gatatccagt 180
ataccaaaga gatgaaggag tccgctgccc gagtgctaca agaagcctgg atgttctaca 240
aacatactcg caggaaggag tctca 265

<210> 6
<211> 16
<212> DNA
<213> Mus musculus

<220>
<223> mIK1 Primer 1

<400> 6

CTGGCGGGAA CTGGCA 16

<210> 7

<211> 17
<212> DNA
<213> Mus musculus

<220>
<223> mIK1 Primer 2

<400> 7

CACCCACTTG CAGCCCA 17

<210> 8
<211> 19
<212> DNA
<213> Homo sapiens

<220>
<223> hIK1 Primer 1

<400> 8

GCGCTCTCAA TCAAGTCCG 19

<210> 9
<211> 22
<212> DNA
<213> Homo sapiens

<220>
<223> hIK1 Primer 2

<400> 9

GTGTTCATGT AAAGCTTGGC CA 22

<210> 10
<211> 22
<212> DNA
<213> Homo sapiens

<220>
<223> hGAPDH-Primer-1

<400> 10

CATGGGTGTG AACCATGAGA AG 22

<210> 11
<211> 21
<212> DNA

<213> Homo sapiens

<220>
<223> hGAPDH-Primer-2

<400> 11

CTAAGCAGTT GGTGGTGCAG G 21

<210> 12
<211> 19
<212> DNA
<213> Mus musculus

<220>
<223> murine GAPDH Primer 1

<400> 12

atcaacggga agcccatca 19

<210> 13
<211> 20
<212> DNA
<213> Mus musculus

<220>
<223> murine GAPDH Primer 2

<400> 13

GACATACTCA GCACCGGCCT 20